# EUROPEAN PATENT SPECIFICATION

(11) **EP 0 540 859 B1**
(45) Date of publication and mention of the grant of the patent: **14.01.1998**
(21) Application number: 92115600.6
(22) Date of filing: 03.08.1988
(51) Int. Cl.: C07H 15/252, A61K 47/48, A61K 31/71

(54) **Anthracycline prodrugs**
Anthracyclin Prodrugs
Précurseurs médicamenteux à base d'anthracycline

(30) Priority: 04.08.1987 US 81382; 26.02.1988 US 161068; 29.06.1988 US 211301
(43) Date of publication of application: 12.05.1993
(62) Divisional of application: 88112646.0
(73) Proprietor: Bristol-Myers Squibb Company, New York, N.Y. 10154 (US)
(72) Inventor: Senter, Peter D., Seattle, Washington 98102 (US); Saulnier, Mark G., Middletown, Connecticut 06457 (US); Brown, Joseph P., Seattle, Washington 98109 (US); Kerr, David E., Seattle, Washington 98199 (US)
(74) Representative: Kinzebach, Werner, Dr.

(56) References cited:
- WO-A-86/03201
- DE-A- 3 500 023
- FR-A- 1 578 734
- FR-A- 2 122 695

## Description

The present invention relates to novel anthracycline prodrugs, to a method for preparing these prodrugs and to the use of these prodrugs for preparing a pharmaceutical composition for the treatment of cancer. More particularly, this invention allows the delivery of cyctotoxic drugs to the site of a tumor by the administration of a tumor-specific antibody-enzyme conjugate that binds to the tumor cells, and the additional administration of a novel prodrug that is converted at the tumor site, in the presence of the antibody-bound enzyme, to an active cytotoxic drug.

### BACKGROUND OF THE INVENTION

The use of immunoconjugates for the selective delivery of cytotoxic agents to tumor cells in the treatment of cancer is known in the art. The delivery of cytotoxic agents to the site of tumor cells is much desired because systemic administration of these agents often results in the killing of normal cells within the body as well as the tumor cells sought to be eliminated. Thus, according to the antitumor drug delivery systems currently in use, a cytotoxic agent is conjugated to a tumor-specific antibody to form an immunoconjugate that binds to the tumor cells and thereby "delivers" the cytotoxic agent to the site of the tumor. The immunoconjugates utilized in these targeting systems include antibody-drug conjugates [see, e.g., R. W. Baldwin et al., "Monoclonal Antibodies For Cancer Treatment," Lancet, pp. 603-05 (March 15, 1986)] and antibody-toxin conjugates [see, e.g., P. E. Thorpe, "Antibody Carriers Of Cytotoxic Agents In Cancer Therapy: A Review," in Monoclonal Antibodies '84: Biological And Clinical Applications, A. Pinchera et al. (ed.s), pp. 475-506 (1985)].

Both polyclonal antibodies and monoclonal antibodies have been utilized in these immunoconjugates [see, e.g., K. Ohkawa et al., "Selective In Vitro And In Vivo Growth Inhibition Against Human Yolk Sac Tumor Cell Lines By Purified Antibody Against Human α-Fetoprotein Conjugated With Mitomycin C Via Human Serum Albumin," Cancer Immunol. Immunother., 23, pp. 81-86 (1986) and G. F. Rowland et al., "Drug Localisation And Growth Inhibition Studies Of Vindesine-Monoclonal Anti-CEA Conjugates In A Human Tumour Xenograft," Cancer Immunol. Immunother., 21, pp. 183-87 (1986)]. Drugs used in these immunoconjugates include daunomycin [see, e.g., J. Gallego et al., "Preparation Of Four Daunomycin-Monoclonal Antibody 791T/36 Conjugates With Anti-Tumour Activity," Int. J. Cancer, 33, pp. 737-44 (1984) and R. Arnon et al., "In Vitro And In Vivo Efficacy Of Conjugates Of Daunomycin With Anti-Tumor Antibodies," Immunological Rev., 62, pp. 5-27 (1982)], methotrexate [N. Endo et al., "In Vitro Cytotoxicity Of A Human Serum Albumin-Mediated Conjugate Of Methotrexate With Anti-MM46 Monoclonal Antibody," Cancer Research, 47, pp. 1076-80 (1987)], mitomycin C [K. Ohkawa et al., supra], and vindesine [G. F. Rowland et al., supra]. Toxins used in the antibody-toxin conjugates include bacterial toxins such as diptheria toxin and plant toxins such as ricin [see, e.g., F. L. Moolten et al., "Antibodies Conjugated To Potent Cytotoxins As Specific Antitumor Agents," Immunol. Rev., 62, pp. 47-73 (1982)].

Despite the amount of research directed towards the use of immunoconjugates for therapeutic purposes, several limitations involved with these delivery approaches have become apparent [see, e.g., M. J. Embleton, "Targeting Of Anti-Cancer Therapeutic Agents By Monoclonal Antibodies," Biochemical Society Transactions, 14, pp. 393-395 (615th Meeting, Belfast 1986)]. Firstly, the large amount of drug required to be delivered to the target tumor cell to effect killing of the cell is often unobtainable because of limitations imposed by the number of tumor-associated antigens on the surface of the cells and the number of drug molecules that can be attached to any given antibody molecule. This limitation has led to the use of more potent cytotoxic agents such as plant toxins in these conjugates and to the development of polymer-bound antibody-drug conjugates having very high drug multiplicity ratios [see, e.g., P. E. Thorpe, supra, pp. 475-506 and R. W. Baldwin et al., "Design And Therapeutic Evaluation Of Monoclonal Antibody 791T/36 - Methotrexate Conjugates," in Monoclonal Antibodies And Cancer Therapy, pp. 215-31 (Alan R. Liss, Inc. 1985)]. However, even with large drug loading ratios or with the use of potent toxins, many immunoconjugates still display sub-optimal cytotoxic activity and are unable to effect complete killing at doses where all available antigenic sites are saturated.

Secondly, it has been recognized that the cytotoxic activity of an immunoconjugate is often dependent on its uptake, mediated by the antibody component of the conjugate, into the tumor cell [see, e.g., J. M. Lambert et al., "Purified Immunotoxins That Are Reactive With Human Lymphoid Cells," J. Biol. Chem., 260 (No. 22), pp. 12035-12041 (1985)1. This internalization is crucial when using an antibody-drug conjugate in which the drug has an intracellular site of action or when using antibody-toxin conjugates. However, the vast majority of tumor-associated antigens and thus the antibody-drug or antibody-toxin conjugates bound to those antigens, are not internalized. Those conjugates that are internalized are often transported to the lysosome of the cell where the drug or toxin is degraded [see, E.S. Vitetta et al., Science, 238, pp. 1098-1104 (1987)]. Accordingly, although an antibody-drug or antibody-toxin conjugate may have excellent tumor-binding characteristics, the conjugate may nonetheless have a limited cytotoxic utility due to an inability to reach its site of action within the cell.

In addition, it is well established that tumor cell populations are often heterogeneous with respect to antigen expression [see, e.g., A. P. Albino et al., "Heterogeneity In Surface Antigen And Glycoprotein Expression Of Cell Lines Derived From Different Melanoma Metastases Of The Same Patient," J. Exp. Med., 154, pp. 1764-78 (1981)]. Furthermore, it has been demonstrated that antigen-positive tumor cells may give rise to antigen-negative progeny [see, e.g., M. Yeh et al., "Clonal Variation For Expression Of A Human Melanoma Antigen Defined By A Monoclonal Antibody," J. Immunol., 126 (No. 4), pp. 1312-17 (1981)]. Thus, in any population of tumor cells, there will be a certain number of cells that do not possess the antigen for which a particular immunoconjugate is specific. The immunoconjugate will therefore not be able to bind to these cells and mediate their killing.

Due to these drawbacks, the currently utilized antitumor drug or toxin delivery systems have had a limited amount of success, especially when used for in vivo treatment.

In addition to the immunoconjugates discussed above, antibody-enzyme conjugates have been studied in vitro in combination with a second untargeted enzyme for the conversion of iodide or arsphenamine to their toxic forms in order to amplify antibody-mediated cytotoxicity [see, e.g., C. W. Parker et al., "Enzymatic Activation And Trapping Of Luminol-Substituted Peptides And Proteins. A Possible Means Of Amplifying The Cytotoxicity Of Anti-Tumor Antibodies," Proc. Natl. Acad. Sci. USA, 72 (No. 1), pp. 338-42 (1975) and G. W. Philpott et al., "Affinity Cytotoxicity Of Tumor Cells With Antibody-Glucose Oxidase Conjugates, Peroxidase, And Arsphenamine," Cancer Research, 34, pp. 2159-64 (1974)].

According to these in vitro studies, the enzyme, glucose oxidase, is attached to an antibody and used in combination with an untargeted peroxidase enzyme to convert iodide or arsphenamine to cytotoxic iodine or arsenical, respectively. This approach, therefore, requires not only the targeting of glucose oxidase to tumor cells with antibody, but also the presence at the tumor site of two other untargeted agents. The likelihood that all three of these agents will be present in vivo at the tumor site at the same time is small and therefore this approach is unlikely to be of therapeutic importance.

Canadian patent 1,216,791, issued to F. Jansen et al., on January 20, 1987, discloses the conjugation to an antibody of an enzyme capable of liberating ammonium ions from substrates. The ammonium ions are then said to potentiate the cytotoxic action of certain immunotoxins targeted to the tumor site.

Finally, European patent application 84302218.7 discloses a method for treating a diseased cell population such as a tumor wherein an antibody is used to target a non-metabolizable antigen to the tumor cells. The antigen accumulates within at least a percentage of the tumor cells, which are then lysed to release the antigen into a ubiquitous fibronectin capturing matrix formed at the tumor site. At this point in the method of the invention, an iodine-containing ligand which is specific for and will bind to the antigen affixed to the matrix is administered. The cytotoxic iodine then acts to kill the tumor cells at that site. Many alternative embodiments are disclosed in this application, one of which suggests the use of an antibody-enzyme conjugate to target enzyme to a tumor site and the addition of a non-lethal substrate which the enzyme can convert to a cytotoxic material [see European application, pp. 34-35]. However, nowhere in the application is there any disclosure of how one is to perform this embodiment. Similarly, Hellstrom et al., "Antibodies For Drug Delivery," in Controlled Drug Delivery (2nd ed.), Robinson and Lee (ed.s), p. 639 (1987) suggest that "[d]rugs which would be non-toxic until 'activated' by an agent (e.g., an enzyme) localized to tumor may be considered as another approach

To date, however, no one has disclosed or suggested how the approach provided herein might be carried out nor has anyone actually attempted this approach to drug targeting.

### SUMMARY OF THE INVENTION

The present invention addresses the problems referred to above by providing novel prodrugs for delivering cytotoxic agents to tumor cells by the combined use with antibody-enzyme conjugates. Therein, an enzyme that is capable of converting a poorly or non-cytotoxic prodrug into an active cytotoxic drug is conjugated to a tumor-specific antibody. This antibody-enzyme conjugate is administered to a tumor-bearing mammalian host and binds, due to the antibody specificity, to the surface of those tumor cells which possess the tumor antigen for which the antibody is specific. The prodrug is then administered to the host and is converted at the tumor site by the action of the antibody-bound enzyme into a more active cytotoxic drug.

According to the present invention prodrugs having the formula: 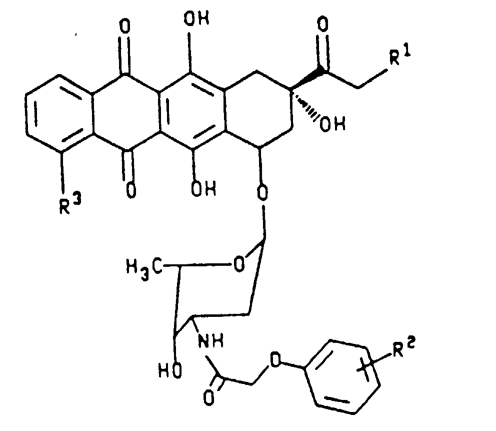 wherein:
R¹ is H, and R³ is OH or OCH₃; or
R¹ is OH and R³ is OCH₃; and
R² is H or OH, or
having the formula 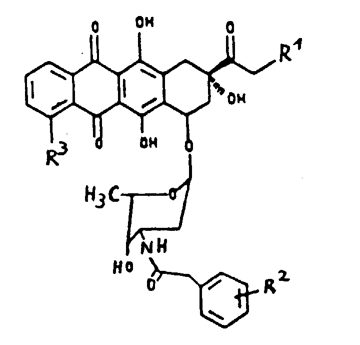 wherein:
R¹ is H, and R³ is OH or OCH₃; or
R¹ is OH and R³ is OCH₃; and
R² is H or OH, are provided.

The present invention also encompasses delivering cytotoxic drugs to tumor cells wherein a series of prodrugs is activated by a single antibody-bound enzyme. In addition, a series of different immunoconjugates, i.e., tumor-specific antibodies bearing different enzymes, can be utilized according to this invention to convert a number of different prodrugs into their more cytotoxic forms for the treatment of tumors. Alternatively, a series of different immunoconjugates wherein the specificity of the antibody component of the conjugate varies, i.e., each immunoconjugate contains an antibody against a different antigenic site on the tumor cell, can be utilized according to this invention to convert a prodrug or a number of prodrugs into a more active cytotoxic form.

According to another embodiment of the invention, an antibody-enzyme conjugate containing the enzyme, penicillin V amidase ("PVA"), has been used in conjunction with a novel prodrug, N-(p-hydroxy-phenoxyacetyl)adriamycin/to effect killing of tumor cells.

The immunoconjugates and prodrugs of this invention may be used in antitumor compositions, such as those comprising a pharmaceutically effective amount of at least one immunoconjugate or prodrug of the invention and a pharmaceutically acceptable carrier. In addition, the immunoconjugates and prodrugs may be used in combinations and methods for treating tumors in mammals comprising the step of treating a mammal with a pharmaceutically effective amount of the compositions of this invention.

Advantageously, the use, immunoconjugates, prodrugs, pharmaceutical compositions and combinations of this invention provide a relatively simple and direct procedure for delivering cytotoxic drugs to tumor cells, allowing enhanced selective cytotoxicity while avoiding the problems of heterogeneous antigen expression, antigen/antibody internalization and insufficient drug potency inherent in conventional antibody-directed immunotherapy techniques.

### BRIEF DESCRIPTION OF THE DRAWINGS

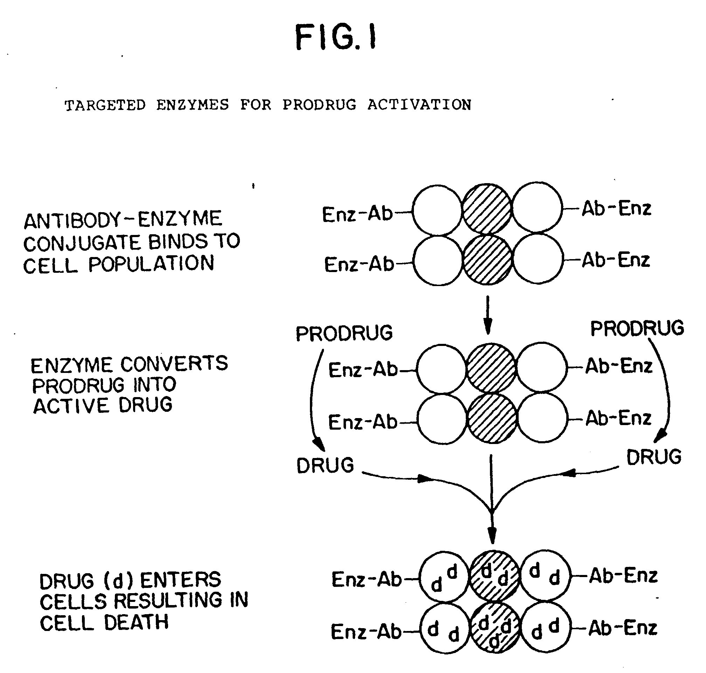

Figure 1 depicts the strategy used for the activation of prodrugs at tumor cells that bind antibody-enzyme conjugates.

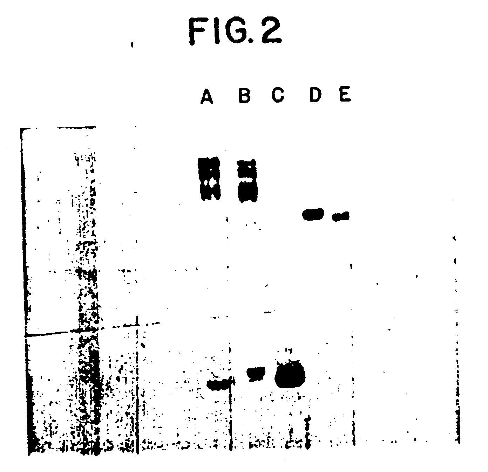

Figure 2 depicts an SDS-polyacrylamide gel analysis (5-12.5% gradient gel, non-reducing) of: (A) the 96.5-AP immunoconjugate; (B) the L6-AP immunoconjugate; (C) AP; (D) monoclonal antibody 96.5; and (E) monoclonal antibody L6.

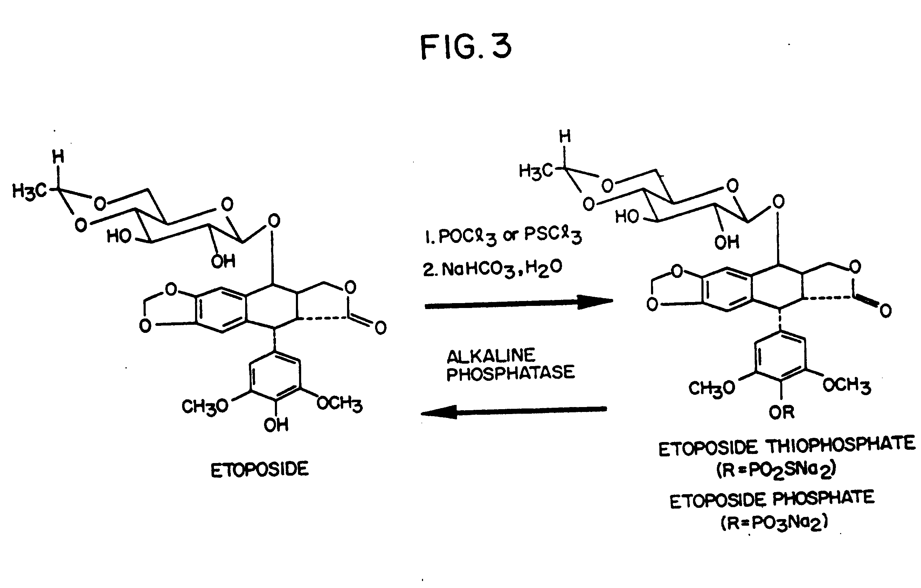

Figure 3 depicts the preparation and hydrolysis of etoposide phosphate and etoposide thiophosphate prodrugs of this invention.

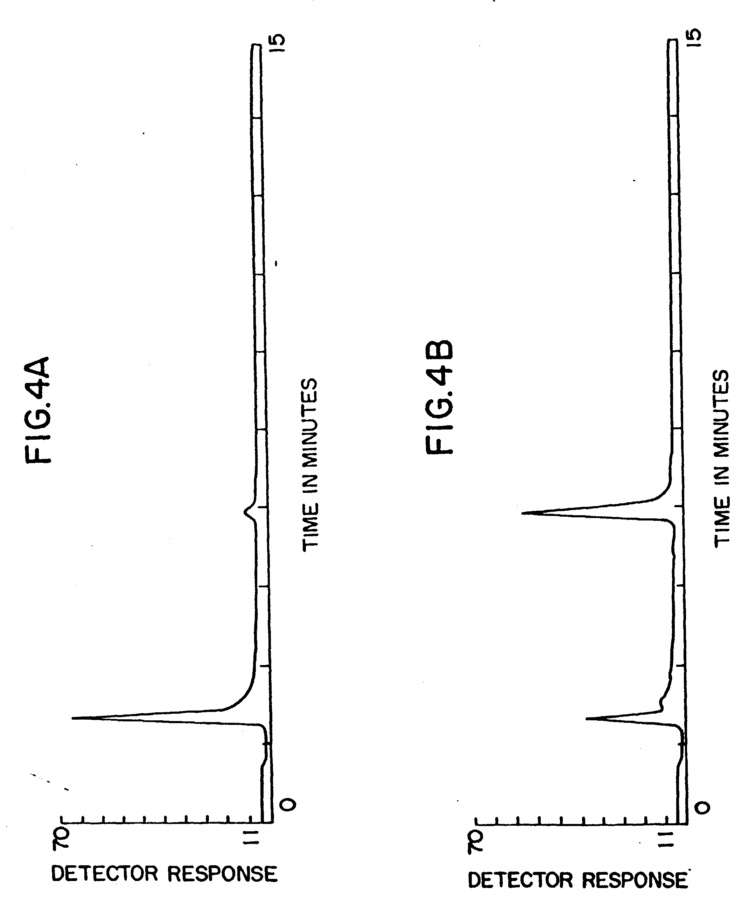

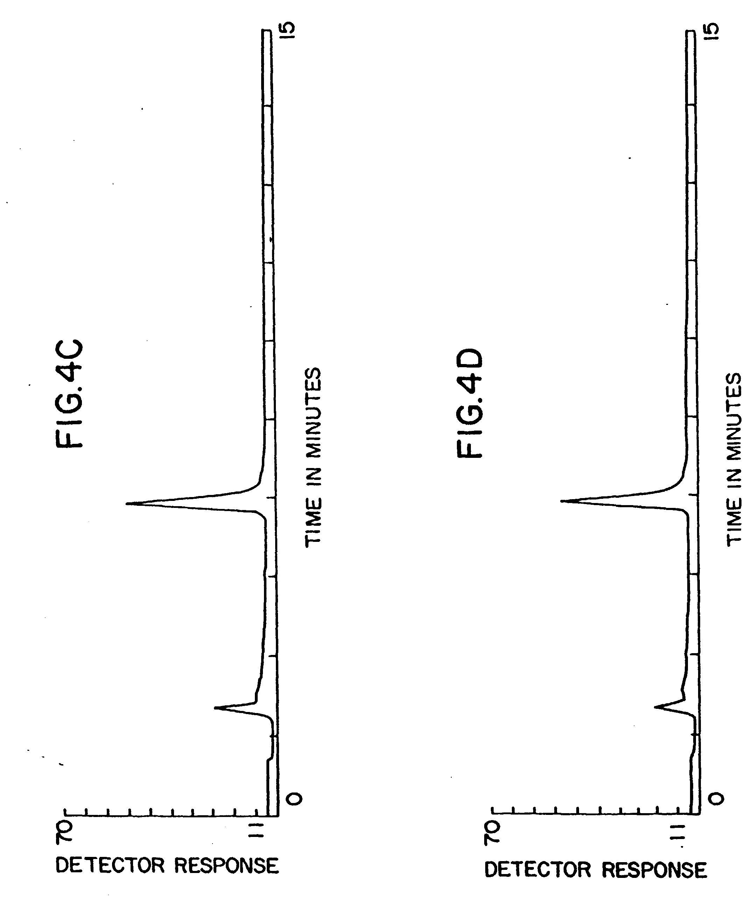

Figure 4 depicts the high pressure liquid chromatography (HPLC) (as monitored at 254 nm) of: (A) etoposide-4'-phosphate alone, i.e., in the absence of AP or the AP-L6 conjugate; (B) etoposide alone; (C) the product produced 5 minutes after the reaction of the etoposide-4'-phosphate prodrug with AP; and (D) the product produced 5 minutes after the reaction of the etoposide-4'-phosphate prodrug with the L6-AP conjugate of the invention.

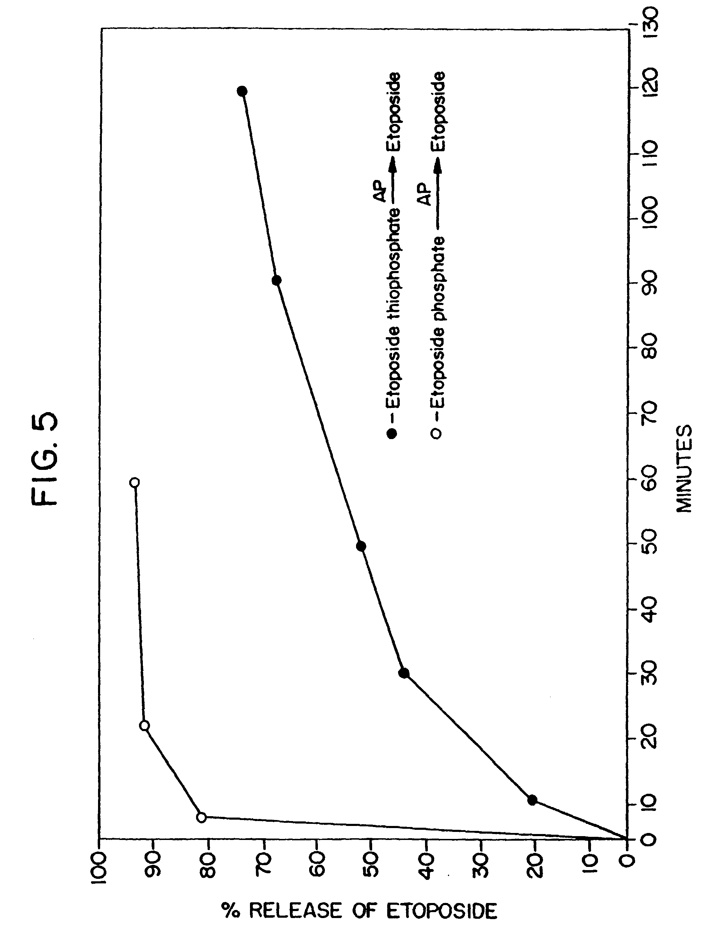

Figure 5 is a comparative graphical presentation of the percentage of etoposide release over time upon exposure of etoposide-4'-phosphate or etoposide-4'-thiophosphate to alkaline phosphatase.

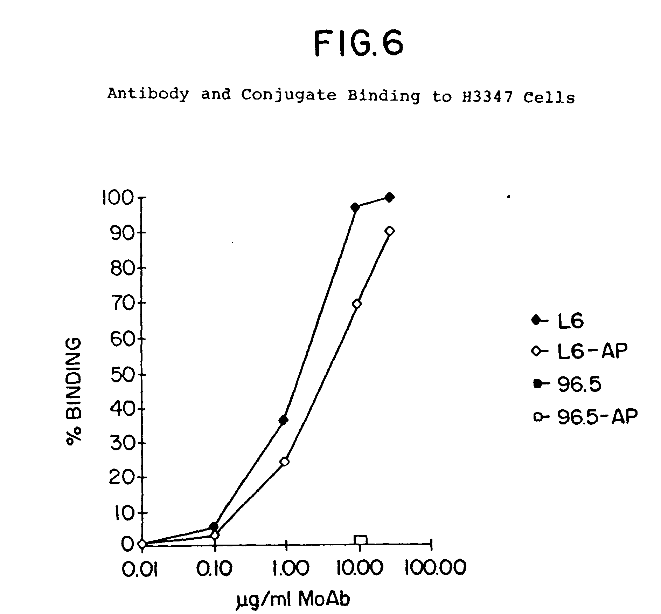

Figure 6 depicts the comparative binding to H3347 tumor cells of the L6 and 96.5 monoclonal antibodies and the L6-AP and 96.5-AP conjugates,

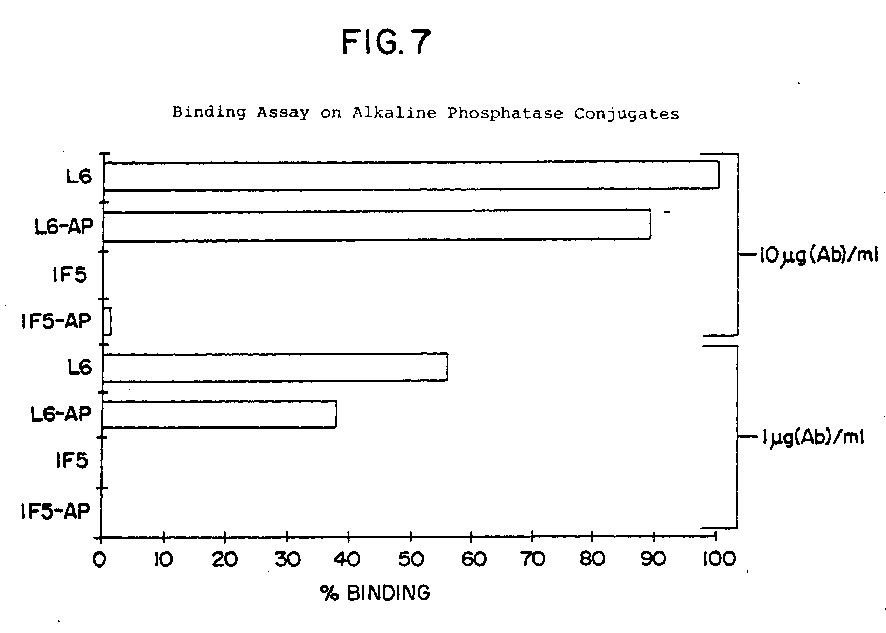

Figure 7 depicts the comparative binding to H3347 tumor cells of the L6 and 1F5 monoclonal antibodies and the L6-AP and lFS-AP conjugates.

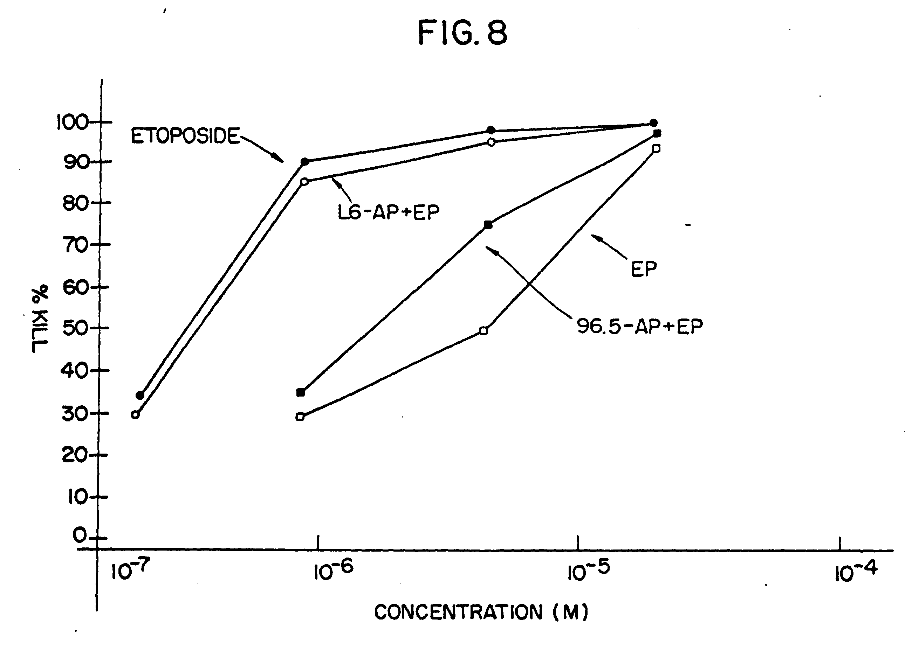

Figure 8 is a comparative graphical presentation of the percentage of tumor cells killed vs. molar concentration of etoposide or the etoposide phosphate prodrug. The graph depicts the increased percentage killing that results from the reaction of the relatively non-cytotoxic prodrug with either the L6-AP conjugate or the 96.5-AP conjugate,

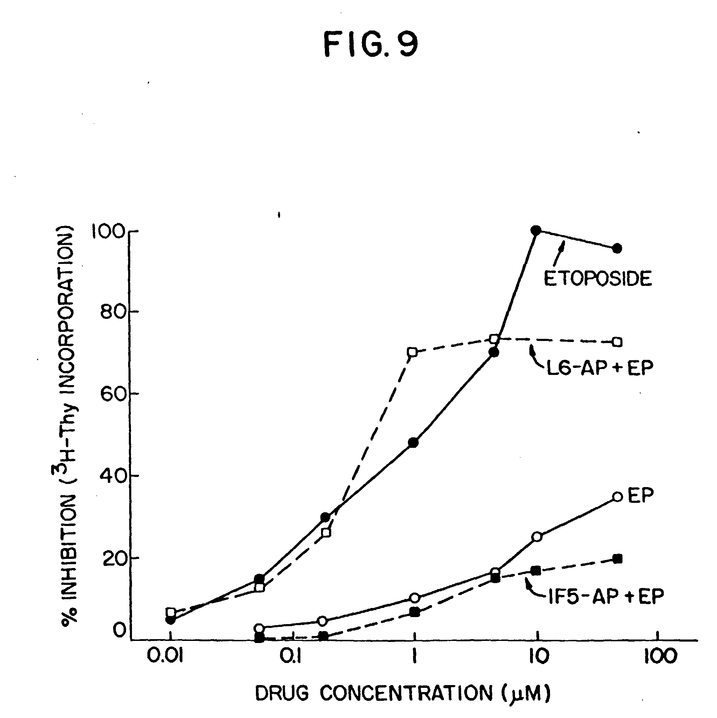

Figure 9 is a comparative graphical presentation of the percent inhibition of ³H-thymidine incorporation into the DNA of H3347 tumor cells treated with •: etoposide, ○: EP, □: L6-AP+EP or ■: 1F5-AP+EP. The graph depicts the increase in cytotoxic activity observed when the tumor cells were treated with L6-AP and EP as compared to the activity seen upon treatment with EP alone.

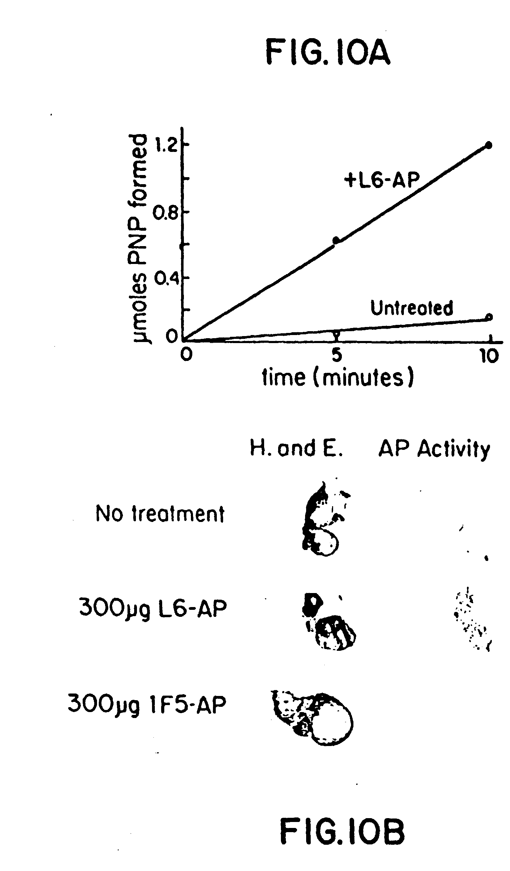

Figure 10 depicts analyses of the phosphatase activity in tumors that were untreated or treated with conjugates. Figure 10A depicts the total phosphatase activity of H3347 tumors over time in untreated mice vs. mice treated 24 hours earlier with the L6-AP conjugate. Figure 10B shows tumor cross-sections from untreated or L6-AP or 1F5-AP-pretreated mice stained either with hematoxylin and eosin or with an AP substrate. Dark areas indicate high phosphatase activity.

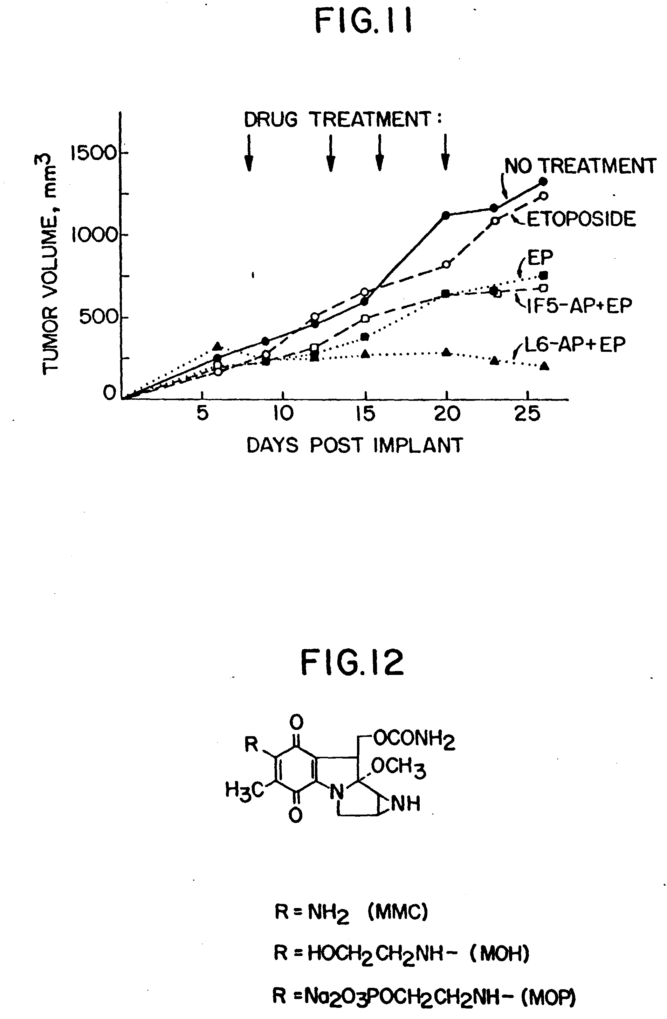

Figure 11 is a comparative graphical presentation of tumor volume over time in mice •: untreated or treated with ○: etoposide, ■: EP, □: 1F5-AP+EP or ▲: L6-AP+EP. Arrows indicate the start of drug treatment and where applicable, the conjugates were administered 18-24 hours earlier. The graph depicts the pronounced antitumor effect observed upon treatment with L6-AP and EP.

Figure 12 depicts the chemical structures of the mitomycin derivatives including the prodrug, 7-(2'-aminoethyl phosphate)mitomycin ("MOP").

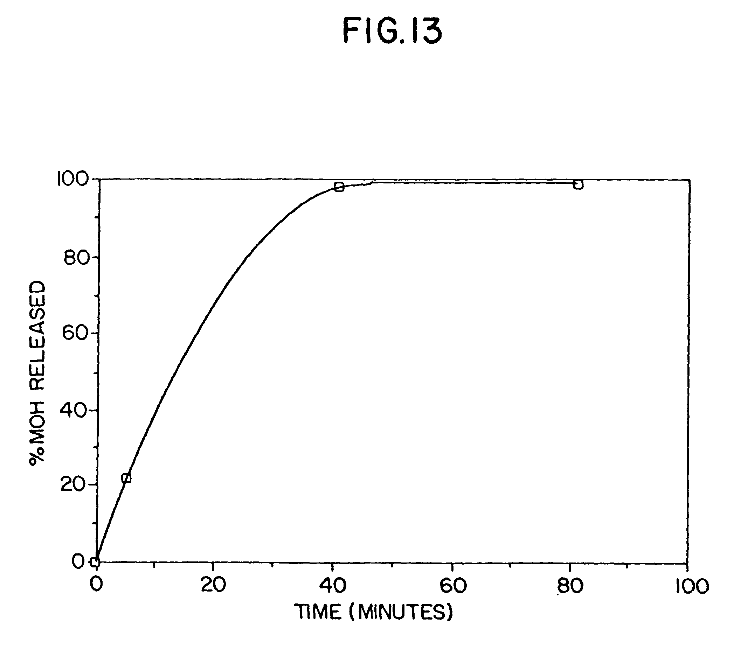

Figure 13 depicts the reaction of MOP with the alkaline phosphatase enzyme over time. The course of the reaction was monitored by HPLC for the release of MOH, the mitomycin alcohol derivative of MOP.

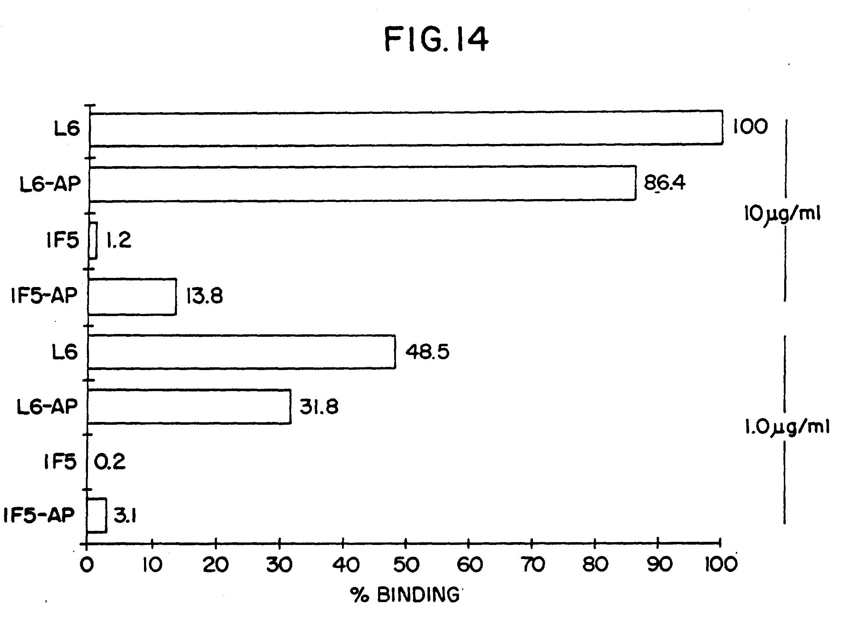

Figure 14 depicts the comparative binding to H2981 tumor cells of the L6 and 1F5 monoclonal antibodies and the L6-AP and 1F5-AP conjugates.

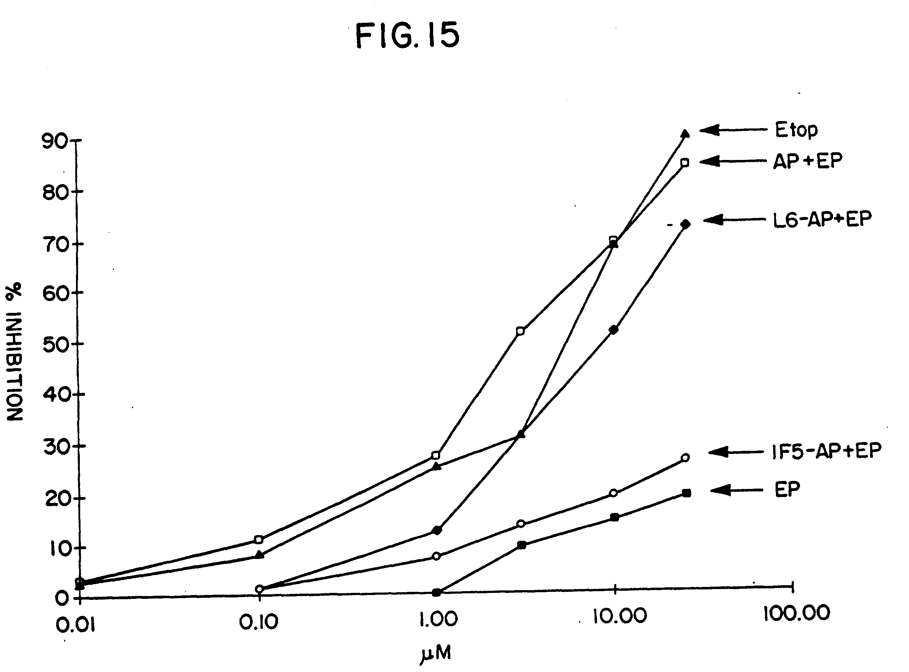

Figure 15 is a comparative graphical presentation of the percent inhibition of ³H-thymidine incorporation into the DNA of H2981 tumor cells treated with ▲: etoposide, ■: EP, □: AP+EP, •: L6-AP+EP or ○: 1F5-AP+EP. The graph depicts the increase in cytotoxic activity over EP alone observed when the tumor cells were pretreated with the L6-AP conjugate,

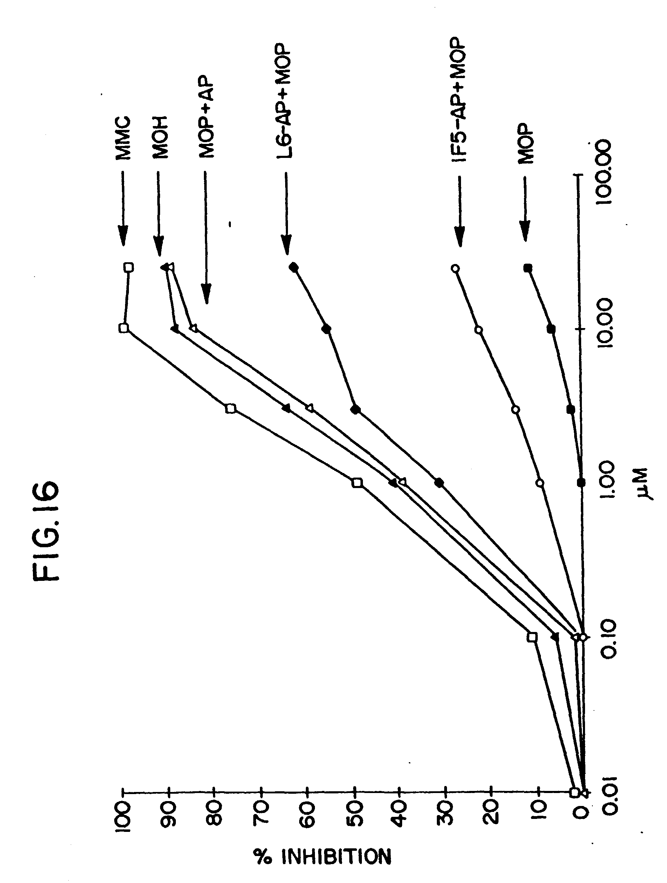

Figure 16 is a comparative graphical presentation of the percent inhibition of ³H-thymidine incorporation into the DNA of H2981 tumor cells treated with □: mitomycin C (MMC), ▲: MOH, ■: MOP, Δ: MOP+AP, •: L6-AP+MOP or ○: 1F5-AP+MOP. The graph depicts the increase in cytotoxic activity observed when the tumor cells were pretreated with the L6-AP conjugate followed by MOP treatment as compared to the activity seen upon treatment with MOP alone.

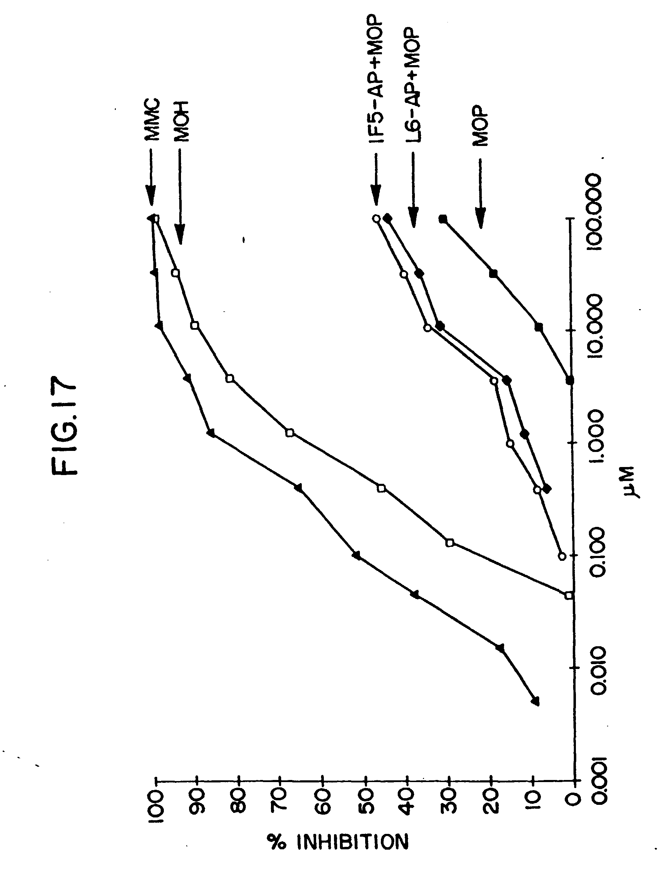

Figure 17 is a comparative graphical presentation of the percent inhibition of ³H-thymidine incorporation into the DNA of CEM cells treated with ▲: MMC, □: MOH, ■: MOP, •: L6-AP+MOP or ○: 1F5-AP+MOP. This graph demonstrates the specificity of the enhanced cytotoxicity seen in Figure 16 above because a significant enhancement is not seen on CEM cells that lack the L6 antigen.

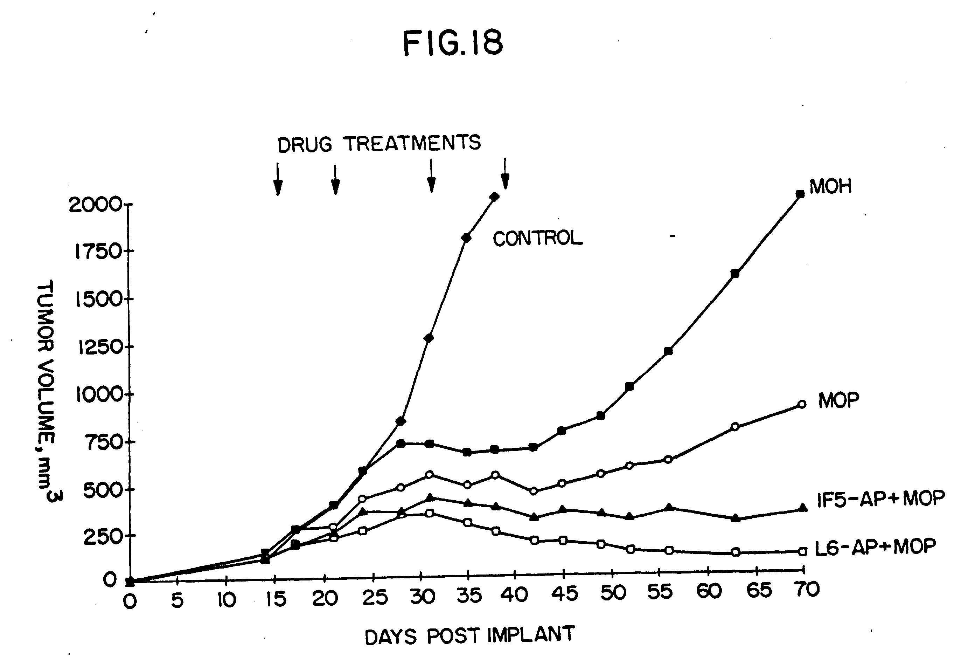

Figure 18 is a comparative graphical presentation of tumor volume over time in mice •: untreated (control) or treated with ■: MOH, ○: MOP, ▲: 1F5-AP+MOP or □: L6-AP+MOP. Arrows indicate the spaced drug treatments and where applicable, the conjugates were administered 18-24 hours earlier than each drug treatment. The graph depicts the pronounced antitumor effect observed upon treatment of the tumors with L6-AP+MOP.

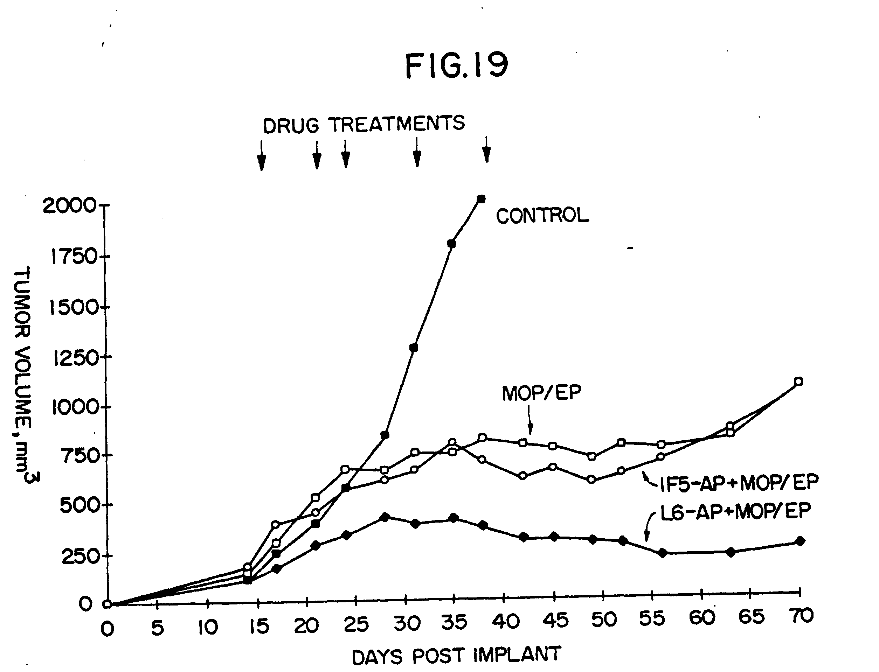

Figure 19 is a comparative graphical presentation of tumor volume over time in mice ■: untreated (control) or treated with □: MOP/EP, ○: 1F5-AP+MOP/EP or •: L6-AP+MOP/EP. Arrows indicate the spaced drug treatments and where applicable, the conjugates were administered 18-24 hours earlier than each drug treatment. The graph demonstrates the pronounced antitumor effect observed upon treatment of the tumors with the L6-AP conjugate and a combination of the prodrugs, MOP and EP.

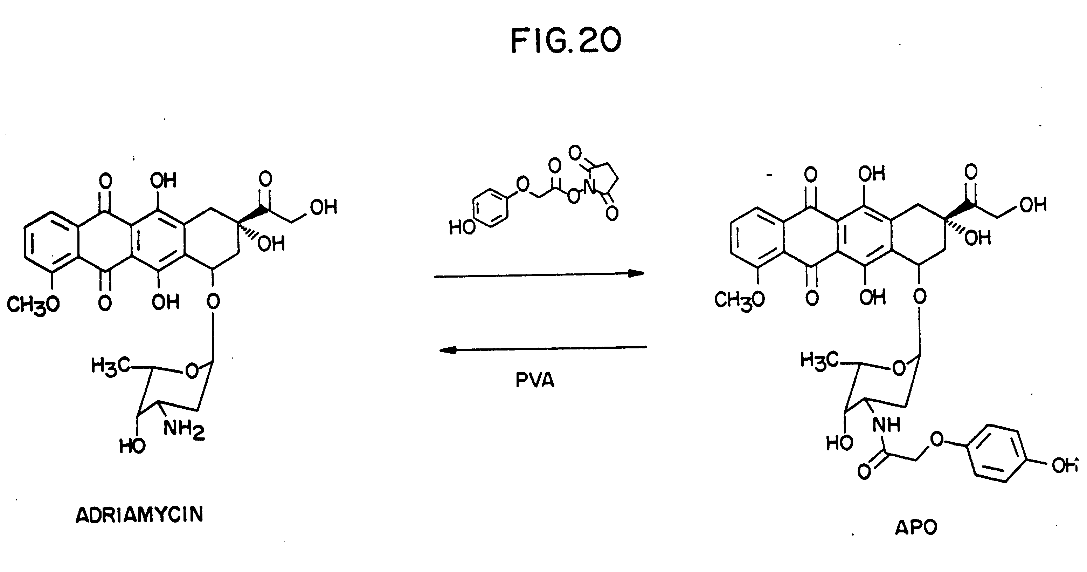

Figure 20 depicts the chemical structure of an adriamycin prodrug of the invention ("APO") and its preparation from adriamycin.

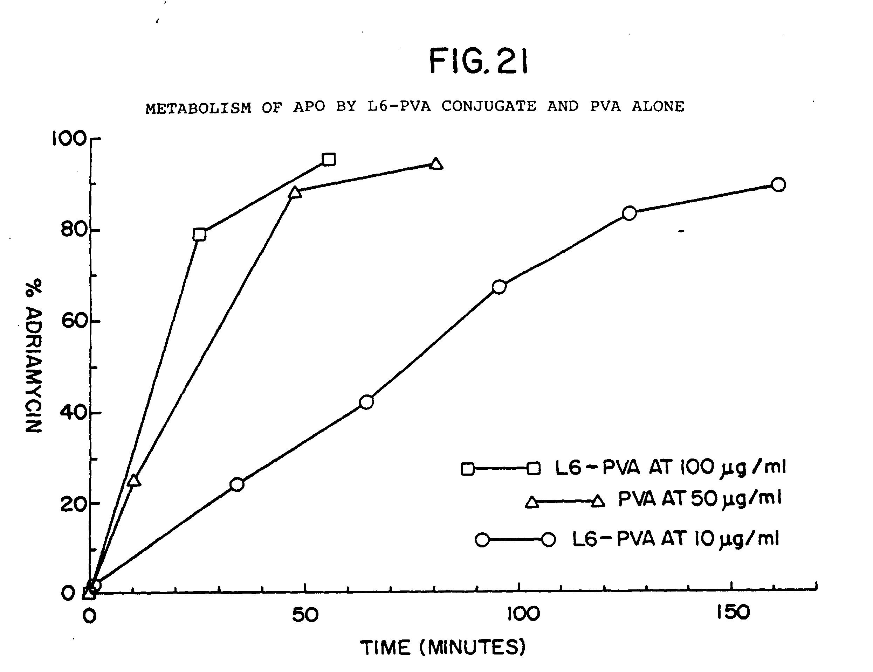

Figure 21 is a comparative graphical presentation of the percentage of adriamycin released over time upon reaction of APO with Δ: free penicillin V amidase enzyme or ○ and □: the L6-PVA conjugate of the invention at 10 and 100 µg total protein/ml, respectively. The course of the reaction was monitored by HPLC.

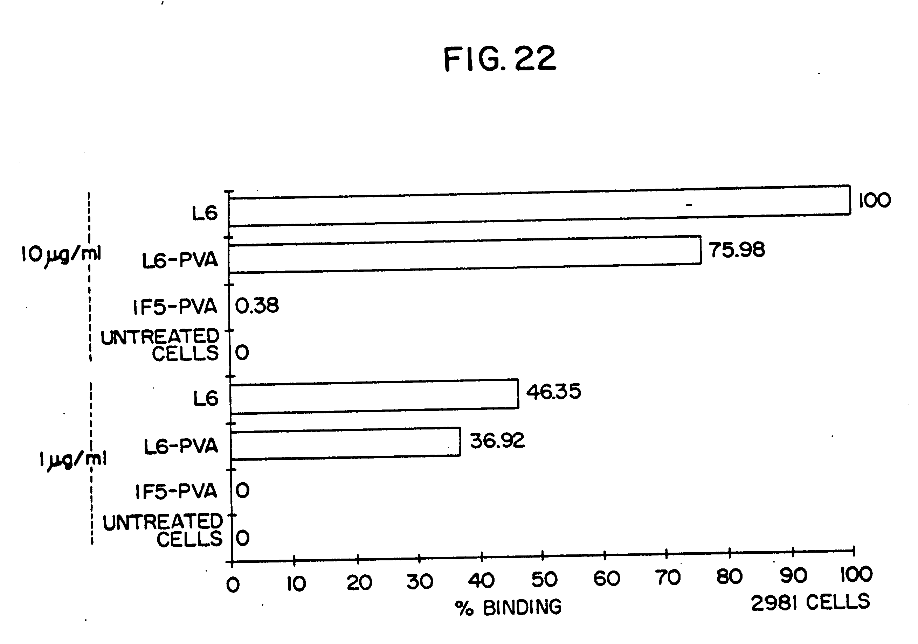

Figure 22 depicts the comparative binding to H2981 tumor cells of the L6 monoclonal antibody and the L6-PVA and 1F5-PVA conjugates of the invention.

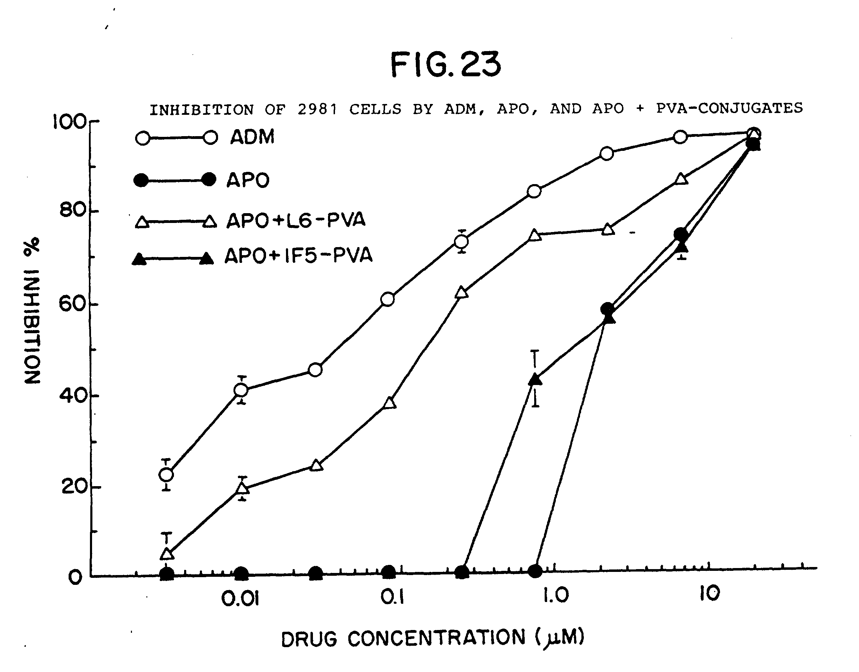

Figure 23 is a comparative graphical presentation of the percent inhibition of ³H-thymidine incorporation into the DNA of H2981 tumor cells treated with ○: adriamycin (ADM), •: APO, Δ: L6-PVA+APO or ▲: 1F5-PVA+APO. The graph depicts the increase in cytotoxic activity observed when the tumor cells were pretreated with the L6-PVA conjugate followed by APO treatment as compared to the activity seen upon treatment with APO alone.

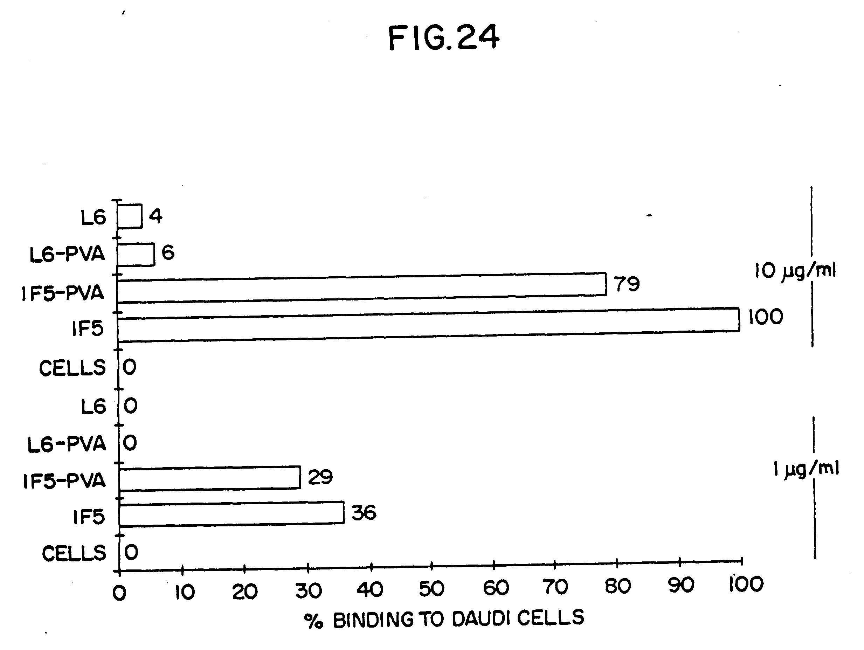

Figure 24 depicts the comparative binding to Daudi lymphoma cells of the L6 and 1F5 monoclonal antibodies and the L6-PVA and 1F5-PVA conjugates of the invention.

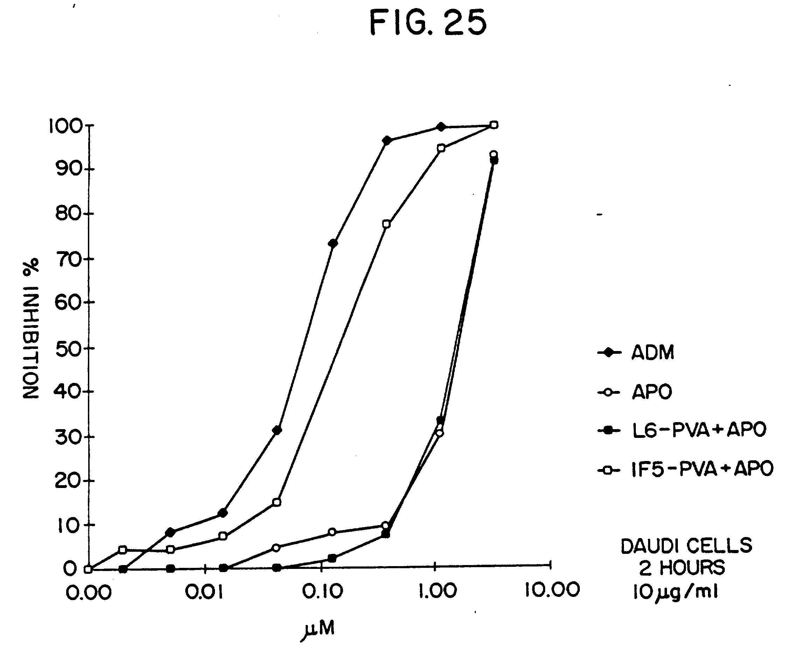

Figure 25 is a comparative graphical presentation of the percent inhibition of ³H-thymidine incorporation into the DNA of Daudi lymphoma cells treated with •: ADM, ○: APO, ■: L6-PVA+APO or □: 1F5-PVA+APO. The graph depicts the increase in cytotoxic activity observed when the tumor cells were pretreated with the 1F5-PVA conjugate followed by APO treatment as compared to the cytotoxic effect seen upon treatment of the cells with APO alone.

### DETAILED DESCRIPTION OF THE INVENTION

The present invention relates to novel prodrugs applicable in a method for the delivery of cytotoxic agents to tumor cells and provides for enhanced selective killing of tumor cells in the treatment of cancers, such as carcinomas and melanomas, as well as other tumors.

According to the invention, an antibody-enzyme conjugate is administered to a tumor-bearing mammalian host. This antibody-enzyme conjugate consists of a tumor-specific antibody linked to an enzyme that is capable of converting & prodrug, that is less cytotoxic to tumor cells than the parent drug, into the more active parent drug. When introduced into the host, the antibody component of the conjugate, which is reactive with an antigen found on the tumor cells, directs the conjugate to the site of the tumor and binds to the tumor cells. The antibody can therefore be viewed as delivering the enzyme to the site of the tumor. A prodrug that is a substrate for the enzyme is then introduced into the host and is converted, at the tumor site, by the enzyme into an active cytotoxic drug. The drug is thus activated extracellularly and can diffuse into all of the tumor cells at that site, i.e., those cells bearing the particular tumor antigen to which the antibody of the conjugate is specific and to which the antibody has bound as well as those cells that are negative for that antigen but are nonetheless present at the site of the tumor (see Figure 1). The method of this invention therefore overcomes the current problems of tumor antigen heterogeneity and the requirement of antigen/conjugate internalization associated with conventional immunoconjugate drug delivery techniques.

Furthermore, because the present method does not require the drug to be bound directly to the antibody and thereby limit the amount of drug that can be delivered, the commonplace problem of drug potency at the tumor site does not arise. In fact, the present method amplifies the number of active drug molecules present at the tumor site because the antibody-bound enzyme of the conjugate can undergo numerous substrate turnovers, repeatedly converting prodrug into active drug. Moreover, the present method is capable of releasing the active drug specifically at the tumor site as opposed to release at other tissues. This is so because the concentration of the enzyme at the tumor site is higher than its concentration at other tissues due to the coating of the tumor cells with the antibody-enzyme conjugate.

The antibody component of the immunoconjugate of the invention includes any antibody which binds specifically to a tumor-associated antigen. Examples of such antibodies include, but are not limited to, those which bind specifically to antigens found on carcinomas, melanomas, lymphomas and bone and soft tissue sarcomas as well as other tumors. Antibodies that remain bound to the cell surface for extended periods or that are internalized very slowly are preferred. These antibodies may be polyclonal or preferably, monoclonal, may be intact antibody molecules or fragments containing the active binding region of the antibody, e.g., Fab or F(ab')₂, and can be produced using techniques well established in the art [see, e.g., R. A. DeWeger et al., "Eradication Of Murine Lymphoma And Melanoma Cells By Chlorambucil-Antibody Complexes, Immunological Rev., 62, pp. 29-45 (1982) (tumor-specific polyclonal antibodies produced and used in conjugates); M. Yeh et al., "Cell Surface Antigens Of Human Melanoma Identified By Monoclonal Antibodies," Proc. Natl. Acad. Sci., 76, p. 2927 (1979); J. P. Brown et al. "Structural Characterization Of Human Melanoma-Associated Antigen p97 With Monoclonal Antibodies," J. Immunol., 127 (No.2), pp. 539-546 (1981) (tumor-specific monoclonal antibodies produced); and J.P. Mach et al., "Improvement Of Colon Carcinoma Imaging: From Polyclonal Anti-CEA Antibodies And Static Photoscanning To Monoclonal Fab Fragments And ECT", in Monoclonal Antibodies For Cancer Detection And Therapy, R.W. Baldwin et al. (ed.s), pp. 53-64 (Academic Press 1985) (antibody fragments produced and used to localize to tumor cells)]. In addition, if monoclonal antibodies are used, the antibodies may be of mouse or human origin cr chimeric antibodies [see, e.g., V.T. Oi, "Chimeric Antibodies," BioTechniques, 4 (No. 3), pp. 214-221 (1986)].

The enzyme component of the immunoconjugate of the invention includes an enzyme capable of acting on a prodrug in such a way so as to convert it into its more active, cytotoxic form. The term "prodrug" as used in this application refers to a precursor or derivative form of a pharmaceutically active substance that is less cytotoxic to tumor cells compared to the parent drug and is capable of being enzymatically activated or converted into the more active parent form [see, e.g., D.E.V. Wilman, "Prodrugs In Cancer Chemotherapy," Biochemical Society Transactions, 14, pp. 375-382 (615th Meeting, Belfast 1986) and V. J. Stella et al.,"Prodrugs: A Chemical Approach To Targeted Drug Delivery," Directed Drug Delivery, R. Borchardt et al. (ed.), pp.247-267 (Humana Press 1985)].

Enzymes that are useful in the method of this invention include pencillin amidases, such as pencillin V amidase or penicillin G amidase, useful for converting drugs derivatized at their amine nitrogens with phenoxyacetyl or phenylacetyl groups, respectively, into free drugs.

Similarly, the prodrugs of this invention include optionally substituted phenoxyacetamide-containing prodrugs or optionally substituted phenylacetamide-containing prodrugs, which can be converted by the enzyme of the conjugate into the more active, cytotoxic free drug. Examples of cytotoxic drugs that can be derivatized into a prodrug form for use in this invention include, but are not limited to, adriamycin, daunomycin and carminomycin.

The enzymes of this invention can be covalently bound to the antibodies of this invention by techniques well known in the art such as the use of the heterobifunctional crosslinking reagents SPDP (N-succinimidyl-3-(2-pyridyldithio)propionate) or SMCC (succinimidyl 4-(N-maleimidomethyl) cyclohexane-1-carboxylate [see, e.g., P. E. Thorpe et al., "The Preparation And Cytotoxic Properties Of Antibody-Toxin Conjugates," Immunological Rev., 62, pp. 119-58 (1982); J.M. Lambert et al., supra, at p. 12038; G. F. Rowland et al., supra, at pp. 183-84 and J. Gallego et al., supra, at pp. 737-38]. Alternatively, fusion proteins comprising at least the antigen binding region of an antibody of the invention linked to at least a functionally active portion of an enzyme of the invention can be constructed using recombinant DNA techniques well known in the art [see, e.g., M.S. Neuberger et al., Nature, 312, pp. 604-608 (1984)]. These fusion proteins act in essentially the same manner as the antibody-enzyme conjugates described herein.

According to the present invention the enzyme was covalently linked to the monoclonal antibody, L6, an IgG2a antibody that binds to a glycoprotein antigen on human lung carcinoma cells [I. Hellstrom et al., "Antitumor Effects Of L6, An IgG2a Antibody That Reacts With Most Human Carcinomas," Proc. Natl. Acad. Sci. USA, 83, pp. 7059-63 (1986)]. The immunoconjugate that resulted showed no loss of enzymatic activity when compared to that of the unconjugated enzyme. In addition, most of the binding activity of the L6 antibody was preserved in the immunoconjugate.

In a preferred embodiment of the invention, a penicillin amidase enzyme was covalently linked to the L6 monoclonal antibody and the resulting immunoconjugate was used to convert a novel adriamycin prodrug into the active antitumor drug, adriamycin. The particular amidase utilized was a penicillin V amidase ("PVA") isolated from Fusarium oxysporum that hydrolyzes phenoxyacetyl amide bonds. Thus, the particular prodrug utilized was N-(p-hydroxyphenoxyacetyl)adriamycin ("APO"), which was hydrolyzed by the amidase to release the potent antitumor agent, adriamycin. The L6-PVA immunoconjugate showed no loss of enzymatic activity when compared to that of the unconjugated enzyme and most of the binding activity of the L6 antibody was preserved in the conjugate.

According to our in vitro studies, treatment of human lung tumor cells with the L6-PVA conjugate followed by exposure of the cells to the APO prodrug resulted in a cytotoxicity comparable to that seen upon treatment of the cells with adriamycin alone. Importantly, the APO prodrug alone demonstrated much less cytotoxicity toward the tumor cells.

Similar in vitro studies were also performed using an 1F5-PVA conjugate in which the PVA enzyme was conjugated to 1F5, a monoclonal antibody reactive with an antigen found on lymphoma cells. Treatment of Daudi lymphoma cells with the 1F5-PVA conjugate followed by exposure of the cells to APO resulted in a cytotoxicity comparable to that seen upon treatment with adriamycin alone, while treatment of the cells with APO alone resulted in very little cytotoxicity.

Although the synthesis and use of the novel adriamycin prodrug, N-(p-hydroxyphenoxyacetyl)adriamycin, is described herein, it should be understood that the present invention includes the synthesis and use of other related adriamycin prodrugs that can be derivatized in substantially the same manner. For example, the prodrug, N-(phenoxyacetyl) adriamycin is also within the scope of the invention in that the prodrug can be synthesized using the protocol described herein but substituting phenoxyacetic acid for the reactant, p-hydroxyphenoxyacetic acid (see Example 4, infra). In addition, it is to be understood that the adriamycin prodrugs of this invention include other N-hydroxyphenoxyacetyl derivatives of adriamycin, e.g., substituted at different positions of the phenyl ring, as well as N-phenoxyacetyl derivatives containing substituents on the phenyl ring other than the hydroxyl group described herein.

Furthermore, the present embodiment encompasses the use of other amidases, such as penicillin G amidase, 'as the enzyme component of the immunoconjugate as well as other prodrugs correspondingly derivatized such that the particular amidase can hydrolyze that prodrug to an active antitumor form. For example, when a penicillin G amidase is used as the enzyme, the prodrug should contain a phenylacetylamide group (as opposed to the phenoxyacetylamide group of APO) because penicillin G amidases hydrolyze this type of amide bond [see, e.g., A.L. Margolin et al., Biochim. Biophys. Acta, 616, pp. 283-89 (1980)]. Thus, other prodrugs of the invention include N-(p-hydroxyphenylacetyl)adriamycin, N-(phenylacetyl) adriamycin and other optionally substituted N-phenylacetyl derivatives of adriamycin.

It should also be understood that the present invention includes any prodrug derived by reacting the amine group of the parent drug with the carboxyl group of phenoxyacetic acid, phenylacetic acid or other related acids. Thus, prodrugs of anthracyclines other than adriamycin that are capable of being derivatized and acting in substantially the same manner as the adriamycin prodrugs described herein falls within the scope of this invention. For example, other prodrugs that can be produced and used in accordance with this invention include hydroxyphenoxyacetylamide derivatives, hydroxyphenylacetylamide derivatives, phenoxyacetylamide derivatives and phenylacetylamide derivatives of anthracyclines such as daunomycin and carminomycin.

It is apparent therefore that the present invention encompasses compounds having formulae I and II: 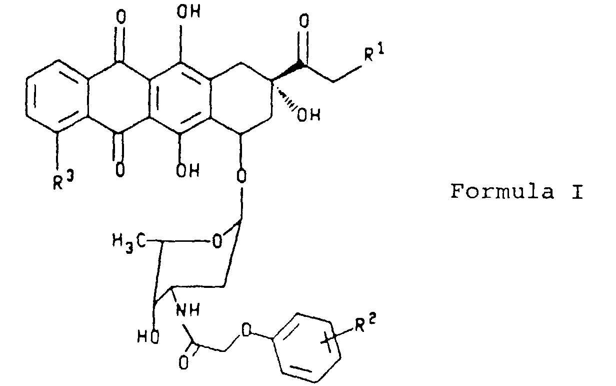 wherein:
R¹ is H, and R³ is OH or OCH₃; or
R¹ is OH and R³ is OCH₃; and
R² is H or OH; and 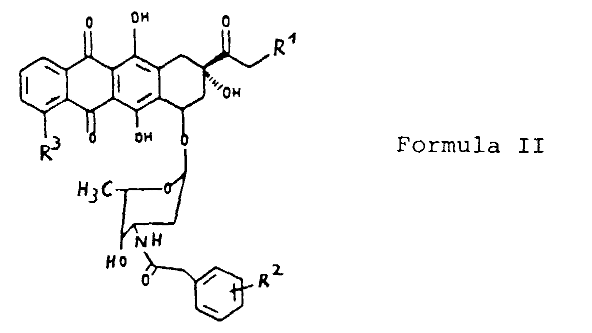
wherein:
R¹ is H, and R³ is OH or OCH₃; or
R¹ is OH and R³ is OCH₃; and
R² is H or OH.

It is apparent from the extensive data described herein that the immunoconjugate/prodrug combination of this invention provides a selective mechanism for killing tumor cells wherein a prodrug is administered that has diminished cytotoxic activity, the prodrug being converted to a highly cytotoxic state at the site of tumor cells, due to the presence there of the antibody-targeted enzyme. Furthermore, the cytotoxicity achieved by this method is enhanced over conventional antibody-targeting techniques because the active drug released at the tumor site is not encumbered by the physical limitations that accompany antibody-drug conjugate delivery systems, as discussed above. It is clear, therefore, that the present invention provides a way to enhance selective cytotoxicity with respect to tumor cells in the treatment of cancers and other tumors.

Another embodiment of the method of this invention provides a combination chemotherapy using several prodrugs and only a single antibody-enzyme conjugate. According to this embodiment, a number of prodrugs are used that are all substrates for the same enzyme in an immunoconjugate. Thus, a particular antibody-enzyme conjugate converts a number of prodrugs into cytotoxic form, resulting in increased antitumor activity at the tumor site.

According to another embodiment, a number of different immunoconjugates are used, wherein the enzyme component of the conjugate varies. Each immunoconjugate can be used to convert its respective prodrug or prodrugs into cytotoxic form at the tumor site. For example, an antitumor antibody can be linked to a first enzyme to form one conjugate and can be linked to a second enzyme to form another conjugate. Both immunoconjugates are then administered to a tumor-bearing host and will bind to the tumor antigen at the tumor site via the antibody specificity. Administration of the corresponding two prodrugs will result in the formation of the potent antitumor agents, at the tumor site.

Still another embodiment of this invention involves the use of a number of immunoconjugates wherein the specificity of the antibody component of the conjugate varies, i.e., a number of immunoconjugates are used, each one having an antibody that binds specifically to a different antigen on the tumor of interest. The enzyme component of these immunoconjugates may be the same or may vary. This embodiment may be especially useful in situations where the amounts of the various antigens on the surface of a tumor is unknown and one wants to be certain that sufficient enzyme is targeted to the tumor site. The use of a number of conjugates bearing different antigenic specificities for the tumor increases the likelihood of obtaining sufficient enzyme at the tumor site for conversion of a prodrug or series of prodrugs. Additionally, this embodiment is important for achieving a high degree of specificity for the tumor because the likelihood that normal tissue will possess all of the same tumor-associated antigens is small [cf., I. Hellstrom et al., "Monoclonal Antibodies To Two Determinants Of Melanoma-Antigen p97 Act Synergistically In Complement-Dependent Cytotoxicity", J. Immunol., 127 (No. 1), pp. 157-160 (1981)].

The present invention also encompasses pharmaceutical compositions, combinations and methods for treating cancers and other tumors. More particularly, the invention includes combinations comprising the antibody-enzyme conjugates of the invention and the corresponding prodrug or prodrugs for use in a method for treating tumors wherein a mammalian host is treated in a pharmaceutically acceptable manner with a pharmaceutically effective amount of an antibody-enzyme conjugate or conjugates and a pharmaceutically effective amount of a prodrug or prodrugs. The combination and methods of this invention are useful in treating any mammal, including humans, dogs, cats, and horses.

According to a preferred embodiment, the antibody-enzyme conjugate is administered prior to the introduction of the prodrug into the host. Sufficient time should be allowed between administration of the conjugate and the prodrug to allow the antibody of the conjugate to target and localize the enzyme to the tumor site. Such sufficient time may range from 12 hours to one week depending upon the conjugate used.

The conjugates and prodrugs of the invention can be administered using conventional modes of administration including, but not limited to, intravenous, intraperitoneal, oral, intralymphatic, or administration directly into the tumor. Intravenous administration is preferred.

The compositions of the invention -- comprising the immunoconjugates or prodrugs -- may be in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application. For example, oral administration of the antibody-enzyme conjugate may be disfavored because the conjugate proteins tend to be degraded in the stomach if taken orally, e.g., in tablet form.

The conjugate or prodrug compositions also preferably include conventional pharmaceutically acceptable carriers and adjuvants known in the art such as human serum albumin, ion exchangers, alumina, lecithin, buffer substances such as phosphates, glycine, sorbic acid, potassium sorbate, and salts or electrolytes such as protamine sulfate.

The most effective mode of administration and dosage regimen for the compositions of this invention depends upon the severity and course of the disease, the patient's health and response to treatment and the judgment of the treating physician. Accordingly, the dosages of the immunoconjugates and prodrugs should be titrated to the individual patient.

Nevertheless, an effective dose of the antibody-enzyme conjugate of this invention may be in the range of from about 1.0 to about 100 mg/m². An effective dose of the prodrug of the invention will depend upon the particular prodrug used and the parent drug from which it is derived. Since the prodrug is less cytotoxic than the parent drug, dosages in excess of those recognized in the art for the parent drug may be used. For example, an effective dose of the etoposide prodrugs may be in the range of from about 75-500 mg/m². An effective dose of the mitomycin phosphate prodrugs may be in the range of from about 50-1000 mg/m². An effective dose of the adriamycin prodrugs may be in the range of from about 15-150 mg/m². And, an effective dose of 5-fluorocytosine and other 5-fluorouridine prodrugs may be in the range of from about 600-2000 mg/m².

In order that the invention described herein may be more fully understood, the following examples are set forth. It should be understood that these examples are for illustrative purposes only and are not to be construed as limiting the scope of this invention in any manner.

### EXAMPLE 1

The following example demonstrates the use of the immunoconjugates and methods for the conversion of an etoposide phosphate prodrug into etoposide by antibody-bound alkaline phosphatase and the resulting in vitro cytotoxicity towards tumor cells and in vivo antitumor effects demonstrated by the use of the methods of this invention.

### Preparation Of Antibody-Alkaline Phosphatase Conjugates

In this example, three immunoconjugates were prepared and studied, comprising either the monoclonal antibody L6, 96.5 or 1F5 conjugated to the enzyme, alkaline phosphatase (AP). L6 is a monoclonal antibody of the IgG2a subclass that is specific for and binds to a glycoprotein antigen on human lung carcinoma cells [see I. Hellstrom et al., (1986), supra]. 96.5 is a monoclonal IgG2a antibody that is specific for p97, a melanoma-associated antigen [see J. P. Brown et al., "Structural Characterization Of Human Melanoma-Associated Antigen p97 With Monoclonal Antibodies," J. Immunol., 127 (No. 2), pp. 539-46 (1981)]. 1F5 is a monoclonal IgG2a antibody that is specific for the CD-20 antigen on normal and neoplastic B cells [see, E.A. Clark et al., "Role Of The Bp35 Cell Surface Polypeptide In Human B-Cell Activation," Proc. Natl. Acad. Sci. USA, 82, pp. 1766-70 (1985)]. The L6 hybridoma that produces the L6 monoclonal antibody was deposited with the American Type Culture Collection (ATCC) under accession number HB8677 in connection with the filing of European patent application 207963, published on January 14, 1987. The 1F5 hybridoma that produces the 1F5 monoclonal antibody was deposited with the ATCC on February 12, 1988 under ATCC No. HB9645. The 96.5 monoclonal antibody is commercially available.

The antibody-enzyme conjugates were prepared by covalently linking AP to the monoclonal antibodies L6, 96.5, or 1F5 through a thioether linkage using a method similar to that described in J. M. Lambert et al., "Purified Immunotoxins That Are Reactive With Human Lymphoid Cells," J. Biol. Chem., 260 (No. 22), pp. 12035-12041 (1985). According to one experimental protocol, the conjugates, L6-AP and 96.5-AP, were prepared as follows: We added 2-iminothiolane (SO mM in 0.5M triethanolamine hydrochloride with 10 mM EDTA at pH 8.0) to a 8.0 mg/ml solution of L6 or 96.5 antibody (in 50 mM triethanolamine hydrochloride and 1 mM EDTA at pH 8.0) so that the final concentration of the 2-iminothiolane was 1.3 mM. After 90 min at 0°C, the reaction was stopped by gel filtration on Sephadex G-25 using phosphate buffered saline (PBS) at pH 7.2 as eluant. Reaction of the antibodies with 2-iminothiolane introduced sulfhydryl groups, the number of which was determined to be 1.9-3.5 using Ellman's reagent [see P. W. Riddles et al., "Ellman's Reagent: 5,5'-Dithiobis(2-nitrobenzoic Acid)-A Reexamination," Analytical Biochemistry, 94, pp. 75-81 (1979)].

Alkaline phosphatase (calf intestine, Boehringer Mannheim, 10 mg/ml) in 100 mM phosphate buffer at pH 7.0 was treated with sulfosuccinimidyl 4-(N-maleimidomethyl)cyclohexane-1-carboxylate (sulfo-SMCC)(Pierce Chemical Co., 20 mM in dimethyl formamide (DMF)) so that the final sulfo-SMCC concentration was 2.4 mM. After 30 min at 30°C, the modifed enzyme was purifed by gel filtration on G-25 Sephadex and eluted with PBS.

The modified AP was then added to the thiolated antibody in a 2:1 molar ratio. Reaction of AP with sulfo-SMCC introduced maleimido groups into the enzyme that when reacted with the sulfhydryl groups on each modified antibody resulted in the formation of a thioether linkage between the antibody and AP. Iodoacetamide (final concentration 1 mM) was added to the protein solution after 1 hour of reaction time in order to block any remaining unreacted thiols, and the conjugates were purified on a Sephacryl 5-300 column using PBS as eluant. Fractions were monitored at 280 nm and the AP activity of each fraction (diluted 64,000 fold) was assayed for at pH 9.5 using p-nitrophenylphosphate as substrate [P. Tijssen, Laboratory Techniques In Biochemistry And Molecular Biology, pp. 366-67, (Elsevier Press 1985)]. Those fractions containing conjugates with appropriate levels of AP-antibody ratios were determined by SDS-PAGE on a 5-12.5% gradient gel (see Figure 2) and were then pooled. The protein concentration was determined at 280 nm where 0.1% solution of the antibodies and AP absorb 1.4 and 0.76 OD's, respectively. Analysis of the conjugates on the gel indicated that they consisted primarily of 1:1 ratios of antibody to enzyme. Under the denaturing conditions used for the gel, AP, which exists in nature as a homo-dimer of molecular weight 140 kd, migrates as a single band of 70kd. This 70kd protein band was observed on the gel in those columns which also contained the higher molecular weight conjugate bands because one of the subunits of the enzyme dissociated from the covalently linked antibody-enzyme conjugate (see Figure 2, lanes A and B). Gel filtration on an S-300 Sephacryl column indicated that there was no free enzyme present in the conjugate preparation.

We also used a second, similar experimental protocol to prepare the L6-AP and 1F5-AP conjugates of the invention, wherein the antibody was modified with iminothiolane (0.5 mM) to introduce a single free thiol group and AP was modified with succinimidyl 4-(N-maleimidomethyl)-cyclohexane-1-carboxylate (SMCC)(Pierce Chemical Co., Rockford, IL) so that the final concentration of SMCC was 1.0 mM. The modified proteins were then combined and the resulting conjugates purified by gel filtration on S-300 Sephacryl. Subsequent SDS-PAGE analysis indicated that these conjugate preparations were free of unconjugated proteins and aggregates. As described above, the protein concentrations of the preparations were determined by absorbance at 280 nm where 1 mg/ml solutions of the antibody (molecular weight: 160 kd) and AP (molecular weight: 140 kd) absorb 1.4 and 0.76 OD units, respectively.

### Preparation Of The Prodrugs, Etoposide Phosphate And Etoposide Thiophosphate

According to the next step each antibody-enzyme conjugate was reacted with a etoposide phosphate or etoposide thiophosphate prodrug. More particularly, the prodrugs utilized were the 4'-disodium phosphate ester of etoposide and the 4'-disodium thiophosphate ester of etoposide, respectively, having the formulae depicted in Figure 3.

Etoposide phosphate and etoposide thiophosphate were synthesized by reacting etoposide with phosphorous oxychloride or thiophosphoryl chloride, respectively, to produce either a dichlorophosphate or dichlorothiophosphate intermediate. The phosphorylation reaction is performed in a suitable anhydrous organic solvent, e.g., acetonitrile, and preferably in the presence of a tertiary amine base, e.g., N,N-diisopropylethylamine. The course of the reaction is monitored by thin layer chromatography (TLC), by which the optimum reaction time may be judged by the appearance of product of the disappearance of the starting material, or both. According to our experience, the reaction may take from about 4 hours to about 72 hours, depending on the quality of the phosphorous reagents used. Hydrolysis of the dichlorophosphate or dichlorothiophosphate intermediate to the disodium phosphate or thiophosphate prodrug, respectively, was carried out by adding a solution of sodium bicarbonate (20-50 fold excess) in water directly to the reaction mixture and allowing the mixture to stir at room temperature for 1.5 or 3 hours, respectively. Partitioning with ethyl acetate and water followed by reverse phase chromatography of the aqueous layer using water-methanol yields the desired prodrugs after lyophilization or evaporation of the aqueous media in vacuo.

A more detailed description of the preparation of the 4'-disodium phosphate derivative of etoposide which was used as one prodrug is as follows:

A magnetically stirred suspension of etoposide (Bristol-Myers Co., 2.30 g, 3.91 mmol) in dry acetonitrile (210 ml) was warmed to give a nearly complete solution, cooled to room temperature, and treated with N,N-diisopropylethylamine (2.36 ml, 13.5 mmol). The mixture was then cooled to 0°C and treated via syringe over 30 sec with phosphoryl chloride, POCl₃ (666 mg, 4.34 mmol). The mixture was allowed to slowly come to room temperature over 2-3 hours and stirred at room temperature for 63 hours. At the end of this period, the reaction mixture was treated with a solution of sodium bicarbonate (6.0 g, 71.4 mmol) in deionized H₂O (110 ml), the mixture was stirred at room temperature for 80 min, and then partitioned with saturated aqueous sodium bicarbonate (20 ml), deionized H₂O (125 ml), and ethyl acetate (350 ml). The organic layer was further extracted with deionized H₂O (1 x SO ml) and the combined aqueous layers were washed with ethyl acetate (250 ml) and then subjected to a vacuum of 0.5 mm at room temperature for 1 hour to remove dissolved solvents. The aqueous portion was then applied to a 4 cm diameter column containing 15 cm of octadecylsilane (C-18) bonded to silica gel that had been packed in methanol and then equilibrated with H₂O. After all of the aqueous portion was applied, the column was eluted with H₂O (175 ml) to remove inorganic salts and then the product was eluted with 20% methanol in water. Concentration of the solvent at 0.5 torr provided 744 mg (36%) of the pure etoposide phosphate compound as a colorless solid. Alternatively, lyophilization provides the pure compound as a very fluffy low density solid.

According to another embodiment, the etoposide phosphate prodrug was prepared as follows:

A magnetically stirred suspension of etoposide (10.50 g, 17.84 mmol, dried over P₂O₅ at 80°C/0.5 torr) in dry acetonitrile (450 ml) was treated with diisopropylethylamine (4.20 ml, 24.1 mmol). Diphenyl chlorophosphate (2.00 ml, 9.65 mmol) was then added via syringe. The mixture was stirred under N₂ for 2 h at 50°C at which point all of the etoposide had dissolved. Additional diphenyl chlorophosphate (1.80 ml, 8.68 mmol) was added and the reaction mixture was held at 45°C for 72 h. After more of the amine base (0.75 ml) and diphenyl chlorophosphate (0.80 ml, 3.86 mmol) were added, the mixture was stirred at 40-45°C for 27 h, treated with more diphenyl chlorophosphate (0.40 ml), and maintained at 40-45°C for 22 h. Isopropanol (20 ml) was then added, the solvent was evaporated in vacuo, and the solid residue was dissolved in CH₂Cl₂ (500 ml), and partitioned with H₂O (400 ml). The aqueous layer was further extracted with CH₂Cl₂ (100 ml) and the combined organic extracts were washed with brine (250 ml) and dried (Na₂SO₄/MgSO₄). Rotary evaporation followed by flash chromatography on silica gel using 2-3% CH₃OH in CH₂Cl₂ provided 12.50 g (85%) of etoposide-4'-diphenyl phosphate as a colorless solid.

Next, platinum oxide (0.198 g, 0.87 mmol) from a freshly opened bottle (Aldrich Chemical Co.) was added to a solution of the etoposide 4'-diphenyl phosphate (0.79 g, 0.962 mmol) in 95 ml of absolute ethanol. The solution was hydrogenated on a Parr apparatus under 45-50 PSI for 4 h at room temperature. The reaction mixture was filtered through a pad of celite using ethanol as eluant. Concentration in vacuo and drying over P₂O₅ for 14 h in vacuo provided etoposide-4'-phosphate as a white solid (0.627,94%):
FAB MS m/e 669 (M+H)⁺
IR (KBr) 3440, 2930, 1778, 1604, 1498 cm⁻¹.
¹H NMR (DMSO-d₆) δ 6.93 (s,1H), 6.46 (s,1H), 6.12 (s,2H), 5.94 (m,2H), 5.17 (bs,1H), 4.86 (d,J=3.93Hz,1H), 4.64 (q,J=7.5,5.8Hz,1H), 4.51-4.42 (m,2H), 4.20 (d,J=10.7Hz,1H), 4.01 (dd,J=12.1,5.3Hz,1H), 3.51 (s,6H), 3.51-2.75 (m,7H), 2.83 (m,1H), 1.16 (d,J=5.1Hz,3H).
¹³CNMR (DMSO-d₆) δ 174.5, 151.2, 151.1, 147.7, 146.2, 126.1, 132.3, 128.8, 109.8, 109.7, 107.9, 101.5, 101.2, 98.5, 80.0, 74.3, 72.7, 71.7, 67.6, 67.2, 65.7, 55.8, 43.0, 37.1, 20.2, 18.5.
Anal. Calcd. for C₂₉H₃₃O₁₆P. 0.85% H₂O: C,50.95; H, 5.11. Found: C,51.42; H,4.97.

The etoposide-4'-phosphate was then converted to its disodium salt by adding deionized H₂O (SO ml) and solid sodium bicarbonate (3.00 g, 35.7 mmol) to 2.90 g (4.34 mmol) of the etoposide-4'-phosphate product. The mixture was stirred at room temperature for 0.5 h during which time the evolution of CO₂ ceased. This mixture was then applied directly to a C-18 column as described in the previous embodiment. The column was first eluted with 300 ml of deionized H₂O to remove the excess salts and was then eluted with 4:1 H₂O/CH₃OH to yield 1.90 g (61%) of pure etoposide 4'-phosphate disodium salt as a fluffy white solid following lyophilization.

The 4'-disodium phosphate or thiophosphate derivatives of etoposide represent highly water soluble prodrugs of etoposide with reduced cytotoxic activity. However, reaction of these compounds with alkaline phosphatase removes the phosphate or thiophosphate moiety, respectively, releasing the potent anti-cancer drug, etoposide (see Figure 3).

An experiment wherein etoposide-4'-phosphate and etoposide-4'-thiophosphate were each reacted with alkaline phosphatase indicated that both prodrugs are substrates for the enzyme. As Figure 5 demonstrates, the etoposide phosphate is hydrolyzed by the enzyme more rapidly than the etoposide thiophosphate prodrug. However, the etoposide thiophosphate may, under certain conditions, have a particular utility due to its increased stability towards hydrolysis.

### Reaction Of The Antibody-Alkaline Phosphatase Conjugates With An Etoposide Phosphate Prodrug

The conjugates of this example did not exhibit any apparent loss in enzymatic activity due to the attachment of the enzyme to the antibody as evidenced by the fact that the conjugates and free enzyme displayed equal activities on the substrates, p-nitrophenyl phosphate [see P. Tijssen, supra] or etoposide phosphate.

For example, either AP alone or the antibody-enzyme conjugate, L6-AP, produced as described above (final AP concentration 5 µg/ml) were added to a solution of etoposide-4'-phosphate (0.1 mM) in Tris buffer (100 mM) containing MgCl₂ (1 mM) and ZnCl₂ (0.1 mM) at pH 7.0. The reaction was monitored by HPLC using an IBM C-18 column (3 µ, 4.5 x 100 mm) and 50% aqueous methanol as eluant (0.5 ml/min, monitored at 254 nm). It was found that within 5 min of the start of the reaction, AP, whether in its free enzyme form or as part of the L6 antibody-enzyme conjugate, had effected the hydrolysis of at least 85X of the etoposide-4'-phosphate to etoposide (see Figures 4C and 4D). As the figures indicate, there was no loss in AP enzyme activity due to its attachment to the antibody in the conjugate. In the absence of enzyme, no phosphate hydrolysis occurred (see Figure 4A). Aqueous solutions of etoposide phosphate or etoposide thiophosphate were stable for at least 8 hours at room temperature and for several days at 4°C.

### Binding Of The Antibody-Alkaline Phosphatase Conjugates To H3347 Tumor Cells

Figure 6 depicts the results of a conjugate binding assay performed to test the ability of the L6-AP and 96.5-AP conjugates as well as the free L6 and 96.5 antibodies to bind to tumor cells from the metastatic human colon carcinoma cell line, H3347 (provided by Judy Anderson, Oncogen).

The binding assay was performed as follows: the immunoconjugates or free antibodies were serially diluted in incomplete modified Delbecco's medium (IMDM, Gibco) and 100 µl aliquots were incubated at 4°C with 10⁶ cells for 30 min. The cells were washed and incubated with 50 µl of FITC-goat anti-mouse antibody (Tago, diluted 1:12.5) for an additional 30 min at 4°C. Cells were washed and analyzed on a Coulter Epics-C fluorescence cell analyzer. Dead cells were gated out and the mean log green fluorescent intensity of each sample was obtained. This number was converted to a linear scale and ratios between the negative control (cells + FITC-goat anti-mouse antibody) and all test samples were calculated.

Figure 6 demonstrates that most of the binding ability of the antibodies was preserved in the conjugates, i.e., conjugation did not affect the antibodies' binding ability. Furthermore, the figure shows the specificity of binding of the antibodies, i.e., that both the 96.5 free antibody and the 96.5-AP conjugate bound much more weakly to the tumor cells than the L6 antibody and L6-AP conjugate. This result may be expected when it is considered that the H3347 tumor cells are from a human carcinoma and the L6 antibody is specific for a carcinoma antigen while the 96.5 antibody is specific for a melanoma antigen.

Similar binding experiments using L6, L6-AP, 1F5 and 1F5-AP also showed that L6 and L6-AP bound to the H3347 carcinoma cell line (saturation at 10 µg/ml antibody) while very little or no detectable binding by 1F5 or 1F5-AP was observed (see Figure 7). This result demonstrated again the specificity of binding of the conjugates, with the L6-AP conjugate binding to the L6-positive tumor cell line and the 1F5-AP conjugate, with a specificity for B lymphoma cells, showing no binding.

### In Vitro Cytotoxicity Of A Conjugate/Prodrug Combination Of The Invention

Next, the cytotoxic effect of the conjugate/prodrug combinations was demonstrated in vitro using either a clonogenic cytotoxicity assay or a ³H-thymidine uptake assay.

The clonogenic cytotoxicity assay we used was the colony inhibition assay described by I. Hellstrom et al., "Colony Inhibition And Cytotoxicity Assays," in In Vitro Methods In Cell-Mediated Immunity, Bloom and Glade (ed.s), pp.- 409-14 (1971). The cells used to detect cytotoxicity were the H3347 tumor cells described above. Both the L6-AP and 96.5-AP conjugates were tested for their ability to convert prodrug into the free drug.

Briefly, the H3347 cells (10⁶/ml) were suspended in IMDM growth media (containing 10 µg/ml of each immunoconjugate based on antibody concentration) and incubated for 30 min at 37°C. The cells were washed twice, resuspended in IMDM, and the drug or prodrug in medium was added. Incubation at 37°C was continued for 15 hours. After washing twice, the cells were plated out and the number of colonies (>8 cells/colony) were counted 7-10 days later.

The results of the assay are shown in Figure 8 (the percent inhibition is the average of six samples). As shown in the figure, etoposide (IC₅₀ = 0.20 µM) was much more cytotoxic than the etoposide-4'-phosphate (EP) (IC₅₀ = 5.8 µM). The prodrug alone showed very little cytotoxic activity. Treatment of the H3347 cells with the L6-AP conjugate, followed by exposure to etoposide phosphate, resulted in a very large increase in cytotoxic activity over that seen with the prodrug alone, the increased cytotoxic activity being comparable to the cytotoxic activity seen with etoposide alone. Treatment of the cells with the 96.5-AP conjugate and etoposide phosphate showed a much smaller increase in cytotoxic activity over that seen with the prodrug alone. This result may be attributable to the small amount of 96.5-AP conjugate that binds to the H3347 cells as discussed above (see Figure 6). The conjugates are not themselves cytotoxic since treatment of the cells with the conjugates alone did not cause any cell death.

The cytotoxic effect of the conjugates was also studied using a ³H-thymidine uptake assay. According to this assay, a suspension of 10⁶ H3347 tumor cells in 0.1 ml of IMDM with 10% fetal calf serum was incubated for 1 h at 4°C in the presence of 5 µg/ml of conjugate. The cells were washed twice with the medium containing 10% fetal calf serum, resuspended (in 1 ml) and plated into 96-well microtiter plates (10,000 cells/well-). The drug or prodrug in IMDM was then added and incubation at 37°C was commenced for 6 h. The cells were washed twice and the incubation was continued an additional 12 h, followed by a 6 h pulse with ³H-thymidine (1.0 µCi/well). The plates were frozen at -20°C to detach the cells and the cells were harvested onto glass fiber discs. The filters were counted on a Beckman 3801 scintillation counter.

Using this assay, we measured the inhibition of ³H-thymidine incorporation into the DNA of the tumor cells and thus, the cytotoxic effect of etoposide or the prodrug, EP, on the cells in the absence or presence of the L6-AP or 1F5-AP conjugates. As shown in Figure 9, etoposide (IC₅₀ = 1 µM) was more than 100-fold more toxic than EP (35X inhibition at 100 µM). Pretreatment of the cells with 1F5-AP prior to EP exposure resulted in no enhancement of cytotoxicity. However, a dramatic increase in cytotoxic activity was observed when the cells were first exposed to L6-AP and then to EP. Thus, in both assays used to determine in vitro cytotoxicity, the cytotoxic effect of the conjugate/prodrug combination was comparable to that of etoposide alone, and this effect was antigen-specific, as indicated by the fact that EP cytotoxicity was not similarly enhanced by treatment of the H3347 cells with the control conjugates, 96.5-AP and 1F5-AP, respectively.

### Localization Of The Conjugates In Tumor Xenographs In Mice

In vivo localization studies were undertaken next to find out how rapidly and to what extent the conjugates accumulated in a tumor. This information would prove useful in determining an appropriate interval of time between the administration of the antibody-enzyme conjugate and the prodrug in our tumor therapy studies.

First, Balb/C nu/nu female mice (4-6 wk old) (obtained from Life Sciences, St. Petersburg, Florida) were injected with 10⁷ H3347 tumor cells subcutaneously (s.c.) in the left and right hind flanks. The tumor cells were obtained from in vitro cultures that had been suspended by treatment for 2 min with trypsin (0.5 g/l) and EDTA (0.2 g/l). The cells were washed twice with IMDM and incubated for 1 h at 37°C in IMDM with 10% fetal calf serum. The cells were washed, suspended in PBS, and kept at 4°C prior to injection. Both the localization and therapy studies described herein were initiated when the tumors reached an average size of 225 mm³.

For our localization studies, L6 and L6-AP were labeled with ¹²⁵I and 1F5 and 1F5-AP were labeled with ¹³¹I, using the iodogen method [see P.J. Fraker et al., Biochem. Biophys. Res. Commun., 80, pp. 849-857 (1978)]. Two days prior to the localization experiments, the animals were put on 0.5% (v/v) Lugol's iodine solution. Each mouse was injected i.p. with 100 µg (based on each monoclonal antibody) of either of the following solutions: L6-AP (5 µCi) and 1F5-AP (2.5 µCi) in 0.2 ml of PBS at pH 7.2 or a combination of L6 (5 µCi) and 1F5 (2.5 µCi) in 0.2 ml of PBS. At periodic intervals, the mice were anesthetized, bled through the orbital plexis and sacrificed. Tissues were weighed and then counted on a gamma counter. Localization was determined by comparing ¹²⁵I-L6 localization with that of ¹³¹I-1F5, i.e., by determining the ratios of specific (¹²⁵I) to non-specific (¹³¹I) uptake of counts in various tissues. The results for tumor and liver uptake are summarized in Table 1 below.

**Table 1**

| PERCENT INJECTED DOSE PER GRAM TISSUE WEIGHT OF ADMINISTERED PROTEINS | | | | |
|---|---|---|---|---|
| L6 | | | L6-AP | |
| | tumor | liver | tumor | liver |
| 2 hours | 1.6 (8.0) | 4.9 (2.0) | 1.5 (7.5) | 5.2 (0.7) |
| 24 hours | 3.6 (12.0) | 2.3 (1.4) | 1.0 (10.0) | 1.3 (1.3) |
| 48 hours | 4.0 (8.0) | 2.5 (1.3) | 0.5 (5.0) | 0.8 (1.0) |
| Numbers in parentheses represent ratios of L6/1F5 or L6-AP/1F5-AP. | | | | |

As the table indicates, unconjugated L6 localized efficiently to the tumor within 24 h and remained there for at least 48 h. During this period, the ratio of L6 to 1F5 in the tumor ranged from 8-12, while the ratio in the liver was quite low (1.3-1.4). The maximum level of specific uptake in the tumor for L6-AP occurred at approximately 24 h, at which point the ratio of L6-AP to 1F5-AP was 10.0. These results indicate that the L6-AP conjugate localized within the tumor far better than did 1F5-AP, but not as well as unmodified L6.

Next, we determined the amount of natural phosphatase activity in the tumor and the degree to which this activity could be raised by targeting AP to the tumor using the above conjugates. Tumors were excised from mice that had been treated for 24 h with 100 µg (based on L6) of L6-AP and the total phosphatase activity was measured, using p-nitrophenyl phosphate as a substrate as follows: The excised tumor was washed and then gently rotated at 23°C with p-nitrophenyl phosphate (1 mg/ml) in pH 9.5 Tris (100 mM) containing NaCl (100 mM) and MgCl₂ (5 mM). The course of the reaction was monitored by measurement of the p-nitrophenol released at 410 nm and the results were corrected for tumor weight. It was found that tumors from mice that had received the L6-AP conjugate displayed as much as 10 times the level of phosphatase activity observed in tumors from untreated mice (see Figure 10A).

A more detailed histological analysis of the phosphatase activity of the tumor was undertaken on cross-sections of tumors obtained from mice that had been untreated or previously treated 24 h earlier with 300 µg (based on antibody) of either L6-AP or 1F5-AP. Phosphatase activity was estimated by immunohistology, using a phosphatase substrate that deposited a dark precipitate at the site of enzyme activity as follows: Excised tumors were quickly frozen to -28°C and 8 µm sequential cross-sections were made using a Reichert-Jung microtome. The phosphatase activity was measured with an AP substrate kit from Vector Laboratories (Burlingame, CA) and the results were compared to sections that were stained with hematoxylin and eosin (H. and E., see Figure 10B).

As Figure 10B demonstrates, little enzyme activity was detected in tumors from mice that were untreated or treated with 1F5-AP. However, in mice that received L6-AP, phosphatase activity was highly elevated and could be seen distributed throughout the tumor. Microscopic evaluation revealed that most of the tumor cells in the L6-AP treated mice stained highly positive for phosphatase activity.

### In Vivo Antitumor Effect Of A Conjugate/Etoposide Phosphate Prodrug Combination

Therapy experiments were performed on nude mice that had s.c. tumors approximately 225 mm³ in volume. The conjugates, L6-AP and 1F5-AP, were administered (i.p.) 18-24 h prior to treatment with EP. Tumor growth was compared to that in untreated mice and in mice treated with maximum tolerated doses of etoposide or EP alone.

More particularly, a group of 8 nude mice with bilateral H3347 tumors was treated with either etoposide (0.2 ml containing 1.2 mg etoposide in 2:3 DMSO:H₂O) or EP (0.2 ml containing 2 mg EP in H₂O) alone or with L6-AP (0.1 ml containing 300 µg antibody in PBS) or 1F5-AP (0.1 ml containing 300 µg antibody in PBS) followed by EP treatment Each experiment contained a control group of mice that was untreated. Tumor volumes were estimated at various days post tumor implant, using the formula:${\text{[(perpendicular width}}^{\text{2}} \text{/2)] X longest length}$

The results of these experiments are shown in Figure 11. Etoposide had very little effect on tumor growth at the dose used and higher doses were not well tolerated. The prodrug, EP, was less toxic to the animals, and the higher dose that could therefore be administered resulted in a greater antitumor effect than seen with etoposide itself. A similar degree of antitumor activity was observed in mice receiving the control conjugate, 1F5-AP, prior to treatment with EP. However, when the mice were treated with L6-AP followed by. EP, a much more pronounced antitumor effect was observed. L6-AP alone had no effect on tumor growth (data not shown).

A summary of the responses of each individual tumor to the therapy is shown in Table 2 below. Out of 16 tumors in the 8 mice treated with L6-AP and EP, 6 tumors underwent complete regression and 2 others became smaller in size than at the start of treatment. No complete or partial responses were observed in any of the other treatment protocols.

**Table 2**

| EFFECTS OF VARIOUS TREATMENTS ON TUMOR GROWTH | | | | |
|---|---|---|---|---|
| Agent | RESPONSE* | | | |
| | progression | stable | partial | complete |
| None | 16 | 0 | 0 | 0 |
| Etoposide | 12 | 4 | 0 | 0 |
| EP | 6 | 10 | 0 | 0 |
| 1F5-AP + EP | 9 | 7 | 0 | 0 |
| L6-AP + EP | 3 | 5 | 2 | 6 |
| Data represents responses of 16 tumors in each group 23 days after tumor implant. | | | | |

| | | | | |
|---|---|---|---|---|
| *Response: progression - continued tumor growth; stable - no additional tumor growth; partial - decrease in size; complete - regression leading to no apparent tumor. | | | | |

The present example clearly demonstrates the applicability of the conjugates for the delivery of a cytotoxic antitumor drug to tumor cells using a tumor-specific antibody-enzyme conjugate and a prodrug capable of being converted by the enzyme from a relatively non-cytotoxic to a potent, cytotoxic form.

### EXAMPLE 2

This example demonstrates the use of the immunoconjugates and methods to convert a relatively non-cytotoxic mitomycin^{.}phosphate prodrug into an active mitomycin drug, leading to in vitro cytotoxicity toward tumor cells. Furthermore, as was demonstrated in Example 1 above, the following example demonstrates the applicability of the immunoconjugates, prodrugs and methods for the delivery of a cytotoxic antitumor drug to tumor cells in vivo.

This example utilizes the L6-AP and 1F5-AP immunoconjugates prepared as described in Example 1 above. Each of these antibody-enzyme conjugates was reacted with a novel mitomycin phosphate prodrug. More particularly, the prodrug utilized was a disodium salt of an N⁷-C₁₋₈ alkyl phosphate of mitomycin C. The antitumor. agent released as a result of this reaction was a mitomycin alcohol derivative. The L6-AP/mitomycin phosphate prodrug combination of this invention resulted in cytotoxicity toward tumor cells in vitro and a pronounced in vivo antitumor effect in mice.

### Preparation Of A Novel Mitomycin Phosphate Prodrug

The mitomycin phosphate prodrug, 7-(2'-aminoethyl phosphate)mitomycin (referred to hereinafter as "MOP") is the 2-aminoethyl phosphate derivative of mitomycin C ("MMC") and was prepared as follows:

A solution of 2-aminoethyl dihydrogen phosphate (56 mg, 0.4 mmol) in water (0.35 ml) and triethylamine (0.3 ml, 2 mmol) was added to mitomycin A (referred to hereinafter as "MMA") (140 mg, 0.4 mmol) in methanol (6 ml) and the reaction was allowed to proceed at room temperature overnight. 1.4 ml of saturated aqueous sodium bicarbonate was then added and the solution was partitioned between water and methylene chloride. The aqueous phase was concentrated to dryness and several portions of methanol were added and evaporated. The residue was taken up into methanol, filtered, and applied to a 2 x 10 cm C-18 (reverse phase) silica column. The product was eluted with water and all volatile material was evaporated. Methanol was added and evaporated as before and the residue was dried for 24 h under high vacuum in a desiccator with phosphorus pentoxide. The mitomycin phosphate derivative, MOP, was obtained as a fine blue powder (190 mg, 97%).

360 MHz ¹H-NMR (D₂O) δ 1.94 (s, 3H, CH₃), 2.9-3.1 (m, 4H), 3.20 (s, OCH₃), 3.28 (s, 1H), 3.36 (s, 1H), 3.5-3.65 (m, 4H), 4.1-4.25 (m, 2H), 4.50-4.57 (dd, 1H, 10-H).

Thus, MOP was prepared by displacement of the 7-methoxy group of MMA with 2-aminoethyl phosphoric acid (see Figure 12). The product was converted to the water soluble disodium salt upon treatment with sodium bicarbonate.

The corresponding known mitomycin alcohol derivative, 7-[(2-hydroxyethyl)amino]-9a-methoxymitosane (referred to hereinafter as "MOH") was prepared by reacting MMA (100 mg, 0.286 mmol) with ethanolamine (26 mg, 0.429 mmol) according to the method of B.S. Iyengar et al., "Mitomycin C and Forfiromycin Analogues With Substituted Ethylamines At Position 7", J. Med. Chem., 26, pp. 16-20 (1983). The product was obtained as a fine blue powder (58 mg, 54%).

### Reactivity And Stability Of The Mitomycin Phosphate Prodrug

The MOP prodrug was then tested for its reactivity with AP. To a solution of MOP (1 mM) in 100 mM Tris, pH 7.2 buffer at room temperature was added either calf intestinal or human placental AP (final conc. 1 µg/ml). The course of the reaction was monitored by HPLC using a C-18 column (4.6 x 150 mm) and the following conditions: detection at 280 nm; 30-95% methanol in acetate buffer (100 mM, pH 5.2) over 8 min, re-equilibration after 15 min; 0.8 ml/min flow rate. Under these conditions, MMC eluted at 7.0 min, MOH eluted at 8.5 min, and MOP eluted 4.0 min. As demonstrated in Figure 13, the phosphate group on the MOP prodrug was rapidly cleaved with AP. HPLC served to confirm that the corresponding alcohol, MOH, was formed. Under the reaction conditions used, the half life for hydrolysis of MOP was about 10 min and the reaction went to completion within 40 min.

The stability of MOP and EP in human serum was determined using HPLC by measuring both the rate of disappearance of the prodrugs and the rate of formation of MOH and etoposide. Thus, for example, a solution of MOP (1 mM in 100 mM Tris, pH 7.2) was added to fresh human serum so that the final drug concentration was 0.1 mM. Aliquots (0.25 ml) were diluted with methanol (0.25 ml) and EDTA (SO µl at 100 mM) to precipitate the serum proteins and stop the reaction. The samples were centrifuged and analyzed by HPLC as described immediately above. It was found that 50% hydrolysis of EP took place after 1 h, but that only 25% of the MOP hydrolyzed after 4 h. Complete hydrolysis could be rapidly achieved by adding AP to the serum.

### Binding Of The Antibody-Alkaline Phosphatase Conjugates To H2981 Tumor Cells

The ability of the L6-AP and 1F5-AP antibody-enzyme conjugates to bind to H2981 tumor cells was then measured. The H2981 cell line was established from a primary human adenocarcinoma of the lung [see, I. Hellstrom et al., "Monoclonal Mouse Antibodies Raised Against Human Lung Carcinomas", Cancer Res., 46 (No. 8), pp. 3917-23 (1986)]. The L6 antibody is known to bind strongly to H2981 cells (saturation at 10 µg/ml) while 1F5 shows very little binding to these cells.

The binding assay was performed as described in Example 1. FACS analysis indicated that L6 and L6-AP bound strongly to the cells, while much weaker binding was displayed for 1F5 and 1F5-AP (see Figure 14).

### In Vitro Cytotoxicity Of The Conjugate/Prodrug Combination Of The Invention On H2981 Tumor Cells

The cytotoxic effect of the conjugate/prodrug combinations was demonstrated in vitro via the ³H-thymidine uptake assay described in Example 1; in this case using H2981 tumor cells to test for in vitro cytotoxicity and using CEM cells as a control. The T cell ALL cell line, CEM, was obtained from the ATCC and does not bind the L6 or 1F5 monoclonal antibodies. The cytotoxic effects of the prodrugs, EP and MOP, on the tumor cells in the absence or presence of the L6-AP or 1F5-AP immunoconjugates were analyzed. The cytotoxic effects of these combinations were also compared to the cytotoxic effect of each parent drug alone.

Briefly, a suspension of 10⁶ H2981 or CEM cells in 0.1 ml of IMDM containing 10% fetal calf serum was incubated for 1 h at 4°C in the presence of 10 µg/ml of conjugate. The cells were washed twice with the medium containing 10% fetal calf serum, resuspended in 1 ml of phosphate buffered saline, pH 7.2 (PBS) and plated into 96-well microtiter plates (10,000 cells/well). The prodrug in PBS was then added and incubation at 37°C was commenced for 1 h (for MOP) or 5 h (for EP). The cells were then washed twice and incubation was continued for a total of 24 h (including a 6 h pulse with ³H-thymidine, 1.0 µCi/well). The plates were frozen at -70°C to detach the cells and after thawing, the cells were harvested onto glass fiber discs. The filters were counted on a Beckman 3801 scintillation counter and the cytotoxic effects of the conjugate/prodrug combinations were compared to the cytotoxicity seen upon treatment of the cells with the prodrug or parent drug alone. The results are shown in Figures 15-17.

As shown in Figure 15, etoposide (IC₅₀ of 2 µM) was significantly more toxic to the H2981 cells than EP (20% kill at 30 µM). Pretreatment of the cells with 1F5-AP prior to prodrug exposure resulted in a very slight enhancement of cytotoxicity. However, a dramatic increase of cytotoxic activity was observed when the cells were first exposed to L6-AP and then to EP. The cytotoxic effect was comparable to that of etoposide alone.

A similar result was observed using mitomycin derivatives. As indicated in Figure 16, MMC and MOH were equally cytotoxic towards H2981 cells and had IC₅₀ values of about 1 µM. The phosphate prodrug, MOP, was much less cytotoxic (5% cell kill at 10 µM), probably owing to its inability to penetrate the cell. However, the activity of MOP was comparable to MOH and MMC when the tumor cells were pre-exposed to the L6-AP conjugate of the invention. This enhancement was antigen specific, since the non-binding conjugate, 1F5-AP, did not significantly affect the cytotoxic activity of the prodrug. Neither L6-AP nor 1F5-AP dramatically enhanced the cytotoxic effect of MOP against CEM cells, consistent with the fact that the conjugates do not bind to this cell line (see Figure 17). Thus, these results indicate that the phosphate group of each of the tested prodrugs inactivates the drug and that upon hydrolysis of that phosphate group by an antibody-enzyme conjugate bound to the tumor cell surface, either of the prodrugs, EP and MOP, yield active cytotoxic drugs.

### In Vivo Antitumor Effect Of The Conjugate/Mitomycin Prodrug Combination

Prior to investigating the in vivo antitumor activity of MOP in combination with the L6-AP conjugate the relative toxicities of the prodrug and its released active derivative, MOH, were determined in Balb C nu/nu mice. When the drugs were administered (i.p.) in two equal doses spaced 4 days apart, LD₅₀ values of 45 and 90 mg drug/kg body weight were obtained for MOH and MOP, respectively. It was also found that considerably more drug could be administered using smaller doses over a longer period of time. Total amounts of up to 40 mg/kg of MOH and 100 mg/kg of MOP were well tolerated if given in 4 equal doses over a 25 day period. These studies indicated that significantly more of the mitomycin prodrug was tolerated because of its reduced toxicity.

Therapy studies were then performed on nude Balb C nu/nu female mice (6 mice per treatment group) (4-6 wk old) obtained from Life Sciences (St. Petersburg, Fla.) that had been implanted (s.c., right hind flank) with a H2981 tumor obtained from in vivo sourcing. The experiments were run when the tumors reached approximately 100 mm³ in volume. The L6-AP and 1F5-AP conjugates (0.1 ml containing 250 µg antibody in PBS) were each administered (i.p.) 18-24 h prior to treatment with MOP (0.2 ml containing 0.6 mg MOP in H₂O). Tumor growth was compared to that observed in untreated mice and in mice treated with maximum tolerated doses of MOP (0.2 ml containing 0.6 mg MOP in H₂O) or MOH (0.2 ml containing 0.2 mg MOH in H₂O) alone.

As shown in Figure 18, both MOH and MOP had significant antitumor activities in vivo. The time required to reach an average tumor volume of 750 mm³ was 45 days in mice treated with MOR, 63 days in mice treated with the MOP prodrug and 27 days in the control group. As discussed above, the MOP prodrug was less toxic to the animals and therefore the higher dosage that could be administered resulted in a greater antitumor effect than that seen with the MOH derivative. Although the non-binding conjugate, 1F5-AP, enhanced the activity of MOP somewhat, a much more pronounced effect was observed in the group that received L6-AP prior to MOP treatment. As the figure indicates, at day 70, tumors that had been pretreated with the L6-AP conjugate (followed by MOP treatment) were approximately one third the size of tumors pretreated with the 1F5-AP conjugate. Furthermore, as Table 3 below indicates, by day 63 post-implant, 3 out of 6 tumors in the L6-AP+ MOP-treated mice underwent complete regression and the remaining 3 tumors had not increased in size from the onset of treatment. In contrast, 3 out of 5 tumors in the 1F5-AP + MOP-treated group actually progressed in size, 2 out of 5 of the tumors were stable, and there were no partial or complete regressions.

**Table 3**

| RESPONSE OF TUMORS (BY DAY 63) TO TREATMENT WITH ANTIBODY-AP CONJUGATES AND MITOMYCIN DERIVATIVES | | | | |
|---|---|---|---|---|
| GROUP | TUMOR RESPONSES | | | |
| | PROGRESSION | STABLE | PARTIAL REGRESSION | COMPLETE REGRESSION |
| Control | 6/6 | | | |
| MOH | 6/6 | | | |
| MOP | 4/5 | 1/5 | | |
| 1F5-AP + MOP | 3/5 | 2/5 | | |
| L6-AP + MOP | | 3/6 | | 3/6 |

These experiments clearly demonstrate the specificity and enhanced antitumor effect of the targeted enzyme/MOP combination in vivo.

### EXAMPLE 3

This example demonstrates the applicability of the immunoconjugates, prodrugs and methods for the delivery of a number of different drugs to tumor cells. Using the antibody-alkaline phosphatase conjugate, L6-AP, in combination with the prodrugs, EP and MOP, enhanced antitumor activity in vivo was demonstrated. Thus, the present invention provides the use of a single antibody-targeted enzyme with a panel of prodrugs for combination chemotherapy against tumors.

The prodrugs, EP and MOP, were prepared as described in Examples 1 and 2, respectively. Preparation of the L6-AP and 1F5-AP immunoconjugates is described in Example 1. The in vivo studies on nude mice were carried out as described in Examples 1 and 2 above. Thus, nude mice that had been implanted with a H2981 tumor were pre-exposed to the L6-AP or 1F5-AP conjugate 18-24 h prior to treatment with a combination of MOP/EP (0.2 ml containing 1 mg EP and 0.3 mg MOP in H₂O). Tumor growth was compared to that observed in untreated mice and in mice treated with the MOP/EP combination alone.

As shown in Figure 19, the antitumor activities of the MOP/EP combination alone and the MOP/EP combination plus 1F5-AP treatment were approximately equal. And, as Table 4 below indicates, all of the tumors in these two groups as well as the tumors of the untreated control mice grew in size. Pre-treatment of the tumor-bearing mice with the L6-AP conjugate followed by the MOP/EP combination, however, resulted in a pronounced antitumor response. As figure 19 indicates, at day 70, tumors that had been pretreated with L6-AP (followed by combined MOP/EP treatment) were approximately one third the size of the tumors pretreated with the 1F5-AP conjugate. Furthermore, Table 4 shows that by day 63 post-implant, one out of six tumors in the L6-AP-pretreated group of mice had completely regressed, 3 out of six tumors had stopped growing and only two out of six tumors progressed in size.

**Table 4**

| RESPONSE OF TUMORS (BY DAY 63) TO TREATMENT WITH ANTIBODY-AP CONJUGATES AND MITOMYCIN/ETOPOSIDE COMBINATIONS | | | | |
|---|---|---|---|---|
| GROUP | TUMOR RESPONSES | | | |
| | PROGRESSION | STABLE | PARTIAL REGRESSION | COMPLETE REGRESSION |
| Control | 6/6 | | | |
| MOP/EP | 5/5 | | | |
| 1F5-AP + MOP/EP | 6/6 | | | |
| L6-AP + MOP/EP | 2/6 | 3/6 | | 1/6 |

Thus, these in vivo studies indicate the applicability of the conjugates, prodrugs and methods for combination therapy against tumors.

Alternatively, the conjugates such as L6-AP can be used with other'combinations of prodrugs, such as EP, adriamycin-14-phosphate and 5-fluorouridine monophosphate to deliver a number of different cytotoxic agents to tumor cells.

Again, preparation of the antibody-enzyme conjugate, L6-AP, and the prodrug, etoposide-4'-phosphate, is as described in Example 1. Adriamycin-14-phosphate is prepared as described in United States Patent 4,185,111, issued to J.B. Ducep on January 22, 1980. 5-Fluorouridine monophosphate is prepared as described in M. J. Robins et al., Can. J. Chem., 53, pp. 1302-1306 (1975).

The reaction of L6-AP with the three above-mentioned prodrugs is carried out as follows: either AP alone or the L6-AP conjugate (final AP concentration 5 µg/ml) is added to solutions of etoposide-4'-phosphate or adriamycin-14-phosphate (0.1 mM) in Tris buffer (100 mM) containing MgCl₂ (1 mM) and ZnCl₂ (0.1 mM) at pH 7.0. For the 5-fluorouridine prodrug, reaction conditions require a solution of 5-fluorouridine (3 µM) in phosphate buffer (100 mM) at pH 8.0. The reaction of L6-AP with either the etoposide phosphate or 5-fluorouridine prodrug is monitored as described in Example 1. The reaction of L6-AP with adriamycin-14-phosphate is monitored by HPLC using an IBM C-18 column (3 µ, 4.5 x 100 mm) and 65% methanol in water containing 3% ammonium acetate as eluant (0.5 ml/min, monitored at 495 nm).

The reaction of the antibody-AP conjugate with each prodrug results in the removal by hydrolysis of the phosphate moieties to release the free drugs [see, e.g., R. B. McComb et al., Alkaline Phosphatase, Plenum Press (New York 1979)].

The cytotoxicity for tumor cells of each of the three prodrugs in the presence of the L6-AP conjugate can be demonstrated using the colony inhibition assay as described in Example 1 above. Upon removal of the phosphate moiety from each of the prodrugs by the conjugate, etoposide, adriamycin and 5-fluorouridine are released. Each of these drugs has been shown to be potent antitumor agents [see, e.g., P.J. O'Dwyer et al., "Etoposide: Current Status Of An Active Anticancer Drug", New England Journal Of Medicine, 312, pp. 692-700 (1985); M.J. Embleton et al., "Antibody Targeting Of Anti-Cancer Agents", in Monoclonal Antibodies For Cancer Detection and Therapy, R. W. Baldwin and V. S. Byers (ed.s), pp. 321-22 (Academic Press 1985); United States Patent 4,185,111, supra; S.T. Crooke and S.D. Reich (ed.s), Anthracyclines: Current Status And New Developments, Academic Press (New York 1980); and C. Heidelberger et al., "Fluorinated Pyrimidines And Their Nucleosides" in Adv. Enzymol. Relat. Areas Mol. Biol., 54, pp. 57-119 (1983)].

The prodrugs of etoposide, adriamycin and 5-fluorouridine can therefore by used together or sequentially for the release of the corresponding known antitumor agents at the site of the tumor by the antibody-alkaline phosphatase conjugates. It has been demonstrated, for example, that antitumor agents administered in combination with each other can act synergistically [see, e.g., S. Monfardini et al., Manual of Cancer Chemotherapy, UICC Technical Report Series, 56 (1981)]. This embodiment of the invention therefore provides a method for combined chemotherapy against tumors.

### EXAMPLE 4

The following example demonstrates the use of immunoconjugates and prodrugs of the invention for the conversion of a relatively non-cytotoxic prodrug into an active antitumor agent displaying in vitro cytotoxicity toward tumor cells. According to this example, an L6-penicillin V amidase (referred to hereinafter as "PVA") immunoconjugate is used to convert a N-phenoxyacetyl derivative of adriamycin into the known antitumor agent, adriamycin.

### Preparation Of Antibody-Penicillin V Amidase Conjugates Of The Invention

In this example, an L6-PVA immunoconjugate and an 1F5-PVA conjugate were prepared. The antibodies L6 and 1F5 and their sources have been described earlier. The amidase enzyme utilized was a penicillin V amidase isolated from a fungal culture of Fusarium oxysporum according to the methods disclosed by D.A. Lowe et al., "Enzymatic Hydrolysis Of Penicillin V to 6-Aminopenicillanic Acid By Fusarium Oxysporum, Biotechnology Letters, 8 (3), pp. 151-56 (1986). Fusarium oxysporum strains from which this enzyme can be isolated are deposited with the ATCC. Thus, PVA is a readily-available enzyme that converts penicillin-V to penicillanic acid. More specifically, PVA hydrolyzes the phenoxyacetyl amide bond of penicillin-V to yield penicillanic acid. The enzyme, which reacts with phenoxyacetamides, may therefore be used to cleave prodrugs of known cytotoxic agents that have been derivatized with phenoxyacetic acid or p-hydroxyphenoxyacetic acid.

The antibody-PVA conjugates of this embodiment of the invention were prepared in essentially the same manner as described for the AP conjugates of Example 1. The antibodies, L6 and 1F-5, were reacted with iminothiolane as described and the number of sulfhydryl groups introduced onto each of the antibodies was determined to be between 1-2.

The PVA enzyme was then dissolved at 9 mg/ml in PBS and treated with SMCC (Pierce Chemical Co., 100 mM in DMF) so that the final concentration was 5 mM. Treatment with SMCC introduced maleimido groups onto the enzyme. After 30 min at 30°C, the modified enzyme was purified by gel filtration on G-25 PD-10 Sephadex (Pharmacia, Upsalla, Sweden) and eluted with PBS. The modified PVA was then added to a solution of each thiolated antibody in a 3:1 molar ratio. Each reaction mixture was saturated with nitrogen and left at room temperature for 3 h and then incubated at 4°C for an additional 18 h. At that point, 2-aminoethanethiol (1 mM final concentration) was added to each solution to block any additional unreacted maleimides.

Each reaction mixture was then passed through a gel filtration column (G-25), using 20 mM Tris, pH 7.2, with 50 mM NaCl as the eluant. The resulting mixtures were purified on DEAE Sephadex columns (2.5 x 10 cm). Fractions were monitored at 280 nm. The unreacted antibody of each mixture did not bind to the column and the conjugate and unreacted PVA were eluted with 20 mM Tris, pH 7.2, with 0.5 M NaCl. The fractions containing PVA and conjugate were then concentrated using an Amicon YM-30 ultrafiltration filter and purified on a Sephacryl S-300 column (2.5 x 95 cm) using PBS as eluant. Fractions were monitored at 280 nm and those that contained pure conjugate, as determined by SDS-PAGE (4-12% gradient gel), were pooled.

### Preparation Of A Novel Adriamycin Prodrug

Each of the antibody-PVA conjugates prepared above was then reacted with a novel adriamycin prodrug. More particularly, the prodrug utilized was N-(p-hydroxyphenoxy acetyl)adriamycin (referred to hereinafter as "APO"), wherein adriamycin is acylated at the amino-sugar position with p-hydroxy-phenoxyacetic acid as depicted in Figure 20.

This adriamycin prodrug was synthesized as follows:

Into 10 ml of tetrahydrofuran were placed 84 mg (0.5 mmole) of p-hydroxy-phenoxyacetic acid, 57 mg (0.5 mmole) of N-hydroxysuccinimide, and 100 mg (0.49 mmole) of dicyclohexylcarbodiimide. This mixture was stirred for 2 h at which time the solution was filtered and the filtrate added to 200 mg (0.35 mmole) of adriamycin hydrochloride. 0.1 ml of triethylamine was added to the reaction mixture and stirring was continued for 4 h. The reaction mixture was then filtered through glass wool and evaporated to a residue under high vacuum. The resulting mixture was purified on a silica gel 60 column (2.5 x 20 cm) eluted with 95:5 dichloromethane:methanol. The pooled fractions were purified again on the same kind of column to yield 70 mg (0.1 mmole, 30% yield) of pure N-(p-hydroxyphenoxyacetyl) adriamycin.

FAB MS m/e 694.2125 (M+H)⁺. Calculated C₃₅H₃₆NO₁₄, 694.2136. 360 MHz ¹H NMR (CDCl₃) δ 1.06 (d, 3H, sugar CH₃), 1.5-2.2 (m, 6H, sugar H), 3.0 (q, 2H) 4.0 (s, 3H, OCH₃), 4.35 (s, 2H, COCH₂O), 4.8-5.0 (m, 3H), 5.2 and 5.4 (s, 1H), 6.6-6.8 (dd, 4H, phenoxy ArH), 7.4-7.9 (m, 3H, 2,3,4-H), 9.0 (s, 1H, Ar'OH), 11.61 and 12.39 (s, 1H, ArOH).

It should be understood that other phenoxyacetyl amide derivatives of adriamycin can be synthesized using substantially the same procedure as described above. For example, N-(phenoxyacetyl)adriamycin can be synthesized as described in this section wherein the p-hydroxyphenoxyacetic acid is replaced by 0.5 mmole (76 mg) of phenoxyacetic acid. Similarly, N-(p-hydroxyphenoxyacetyl)melphalan or daunomycin prodrugs or N-(phenoxyacetyl)melphalan or daunomycin prodrugs can be synthesized by this synthetic protocol, wherein 100 mg of mephalan or 200 mg of daunomycin (0.35 mmole) are used.

### Reaction Of An Antibody-Penicillin V Amidase Conjugate With An Adriamycin Prodrug

The ability of the antibody-PVA conjugate, L6-PVA, to convert the novel prodrug, APO, to adriamycin was measured as follows: either a) PVA alone (final concentration:50 µg/ml), b) 100 µg/ml of the L6-PVA conjugate (final PVA concentration: 25 µg/ml) or c) 10 µg/ml of L6-PVA (final PVA concentration: 2.5 µg/ml) were added to a solution of APO (0.1 mM) in PBS. Each reaction was monitored by HPLC using a Phenominex C-18 column (3 µm, 4.5 x 100 mm) and a gradient elution of 20-60% tetrahydrofuran in water with 0.1% H₃PO₄ (1.0 ml/min, monitored at 495 nm). Under these conditions, the adriamycin eluted at 8.9 minutes and the APO eluted at 12.2 minutes. The results are shown in Figure 21.

As the figure demonstrates, the amide group of APO was in fact hydrolyzed by PVA as indicated by the generation of adriamycin. Under the conditions used, the half life for the hydrolysis of APO by PVA was approximately 20 min. Furthermore, it was found that within 40 minutes of the start of the reaction, either the enzyme alone or the antibody-PVA conjugate was able to effect the hydrolysis of at least 80% of APO to adriamycin. The conjugate at 10 µg/ml (2.5 µg/ml of PVA) was able to effect this level of hydrolysis in 120 minutes. Finally, it is evident from these studies that the antibody-PVA conjugate of this invention did not exhibit any apparent loss in enzymatic activity due to the attachment of the enzyme to the antibody as evidenced by the fact that the conjugate and free enzyme displayed similar abilities in hydrolyzing APO to adriamycin.

### Serum Stability Of The Novel Adriamycin Prodrug Of The Invention

The stability of APO in human serum was determined using HPLC and measuring the rate of disappearance of APO and the rate of formation of adriamycin. Thus, a solution of APO (10 mM in dimethylformamide) was added to fresh human serum such that the final concentration was 0.1 mM. Aliquots (50 µl) were diluted with methanol (50 µl) to precipitate serum proteins. These samples were then centrifuged and analyzed by HPLC as described immediatley above. No hydrolysis of APO to adriamycin occurred in two hours.

### Binding Of The Antibod -PVA Conjugates To H2981 Tumor Cells

The ability of the L6-PVA and 1F5-PVA conjugates of the invention to bind to H2981 tumor cells was measured as described in Examples 1 and 2. The results of the binding assay are depicted in Figure 22.

FACS analysis indicated that both L6 and the L6-PVA conjugate bound strongly to the tumor cells while the 1F5-PVA conjugate did not display any significant amount of binding. This binding study indicates firstly that conjugation to the enzyme did not substantially affect the binding ability of the antibody component of the immunoconjugates of this invention. Secondly, this assay again demonstrates the specificity of binding of the conjugates; the L6-PVA conjugate binding to the L6-positive H2981 tumor cells and the 1F5-PVA conjugate, due to the 1F5 antibody's lack of specificity for the tumor cells, showing essentially no binding.

### In Vitro Cytotoxicity Of The Antibody-PVA Conjugate/ Adriamycin Prodrug Combination Of The Invention On H2981 Tumor Cells

The in vitro cytotoxic effect of the antibody-PVA/ adriamycin prodrug combination of the invention toward H2981 tumor cells was measured using the ³H-thymidine uptake assay described in Examples 1 and 2 above. Briefly, the H2981 tumor cells were plated into 96-well microtiter plates in IMDM (10,000 cells/well) and were allowed to attached for 18 h at 37°C. The antibody-PVA conjugates, L6-PVA or 1F5-PVA, were then added at a concentration of 10 µg/ml of antibody and the plates were incubated for 30 min at 4°C. The wells were then washed four times with IMDM and APO was added at varying concentrations in IMDM. After two h, the wells were again washed, IMDM was added and the cells were left for 18 h at 37°C. At that point, ³H-thymidine was added (1 µCi per well) and after 6 h, the plates were frozen at -70°C to detach the cells. After thawing, the cells were harvested onto glass fiber filters. The incorporation of ³H-thymidine was measured in a Beckman 3801 scintillation counter and compared to cells treated with APO or adriamycin (ADM) alone.

Using this assay, we measured the inhibition of ³H-thymidine incorporation into the DNA of the tumor cells and thus, the cytotoxic effect of the prodrug, APO, on the cells with or without pretreatment of the cells with the L6-PVA or 1F5-PVA conjugates. The cytotoxic effects of these combinations were compared to the cytotoxicity observed upon treatment of the cells with the parent drug, adriamycin, alone. As shown in Figure 23, on tumor cells untreated with any conjugate, adriamycin, with an IC₅₀ of 38 nM, was significantly more toxic than APO with an IC₅₀ of 2 µM. This was expected based upon previous reports showing that adriamycin amides are less toxic than adriamycin [see, e.g., Y. Levin and B. A. Sela, FEBS Letters, 98, p. 119 (1979) and R. Baurain et al., J. Med. Chem., 23, p. 1171 (1980)]. Pretreatment of the cells with L6-PVA enhanced the cytotoxicity of APO 20-fold to a level comparable to that seen with adriamycin alone. Pretreatment of the cells with 1F5-PVA did not affect the toxicity of APO at all. These results indicate that the L6-PVA conjugate is capable of hydrolyzing the relatively non-cytotoxic prodrug, APO, to kill the tumor cells to an extent comparable to the use of adriamycin alone and that this cytotoxicity is antigen specific as indicated by the fact that the 1F5-PVA conjugate, which does not bind significantly to this particular tumor cell line, showed no such cytotoxicity.

### Binding Of The Antibod -PVA Conjugates To Daudi Lymphoma Cells

The ability of the L6-PVA and 1F5-PVA conjugates of the invention to bind to the known Daudi cell line was also measured. This cell line is a Burkitt lymphoma cell line, deposited with the ATCC (ATCC # CCL 213), that expresses the CD-20 antigen to which the 1F5 antibody binds. The binding assay was carried out as described in Example 1, except that the cells used were Daudi cells, and the results are depicted in Figure 24.

In this instance, the 1F5 monoclonal antibody and the 1F5-PVA conjugate both bound strongly to the lymphoma cells. Thus, this study again indicates that the binding ability of the conjugates was not significantly affected by the conjugation procedure.

Furthermore, as the figure indicates, the L6 antibody and the L6-PVA conjugate showed no appreciable binding to the Daudi cells. This was to be expected because Daudi tumor cells do not possess the antigen with which the L6 antibody reacts. Thus, this study taken in combination with the previous binding studies described herein, clearly demonstrates the specificity of binding of the conjugates of this invention, i.e., the L6-containing conjugates bind specifically to L6-positive tumor cells and the 1F5-containing conjugates bind specifically to CD-20-positive tumor cells.

### In Vitro Cytotoxicity Of The Antibody-PVA Conjugate/ Adriamycin Prodrug Combination Of The Invention On Daudi Cells

We then tested the in vitro cytotoxic effect on Daudi cells of the L6-PVA or 1F5-PVA conjugate in combination with the APO prodrug.

The ³H-thymidine assay was performed essentially as described in the examples above with slight modifications due to the fact that the Daudi cells are non-adherent. Thus, approximately 250,000 Daudi cells in IMDM were plated into each well of a 96-well microtiter plate and the antibody-enzyme conjugate added. The reaction mixture was incubated at 4°C for 30 min. Unbound antibody-enzyme conjugate was removed by centrifuging at 500 x g for 5 min and removing the supernatant. The cells were resuspended in IMDM and the washing procedure was repeated three times to remove all unbound conjugate. APO in IMDM was then added for 2 h and washed once as described above. The remainder of the assay was performed as described in the examples above.

Using this assay, we measured the inhibition of ³H-thymidine incorporation into the DNA of the Daudi cells and thus, the cytotoxic effect of the APO prodrug on the cells with or without pretreatment of the cells with the L6-PVA or 1F5-PVA conjugate. The cytotoxic effects of these combinations were compared to the cytotoxicity observed upon treatment of the cells with adriamycin alone. As shown in Figure 25, on Daudi cells untreated with any conjugate, adriamycin was significantly more toxic than APO. Pretreatment of the cells with the 1F5-PVA conjugate significantly enhanced the cytotoxicity of the APO prodrug to a level comparable to that seen with adriamycin alone, whereas pretreatment with the L6-PVA conjugate resulted in no such enhancement.

It should be noted that the results obtained from these binding and cytotoxicity studies are the opposite of the results obtained with these conjugates in the studies described earlier in this example using H2981 tumor cells where the L6-PVA plus APO combination showed enhanced cytotoxic effects and the 1F5-PVA plus APO combination did not. This was to be expected given the different specificities of the L6 and 1F5 antibodies of the conjugates and clearly demonstrates the specificity of the cytotoxic effects obtained with the conjugate/prodrug combinations of the invention.

Furthermore, this study indicates the usefulness of the 1F5-PVA conjugate in combination with APO for the production of cytotoxic effects on tumor cells in vitro. Thus, the in vitro cytotoxicity studies of this example demonstrate the applicability of the present invention to any conjugate containing an antibody reactive with a tumor-associated antigen for the treatment of tumors with which that antibody reacts.

While we have hereinbefore presented a number of embodiments of this invention, it is apparent that our basic construction can be altered to provide other embodiments which utilize the methods, immunoconjugates and prodrugs of this invention. Therefore, it will be appreciated that the scope of this invention is to be defined by the claims appended hereto rather than by the specific embodiments which have been presented hereinbefore by way of example.

## Claims (Claims for the following Contracting State(s): AT, BE, CH, LI, DE, FR, GB, IT, LU, NL, SE)

1. A compound having the formula: 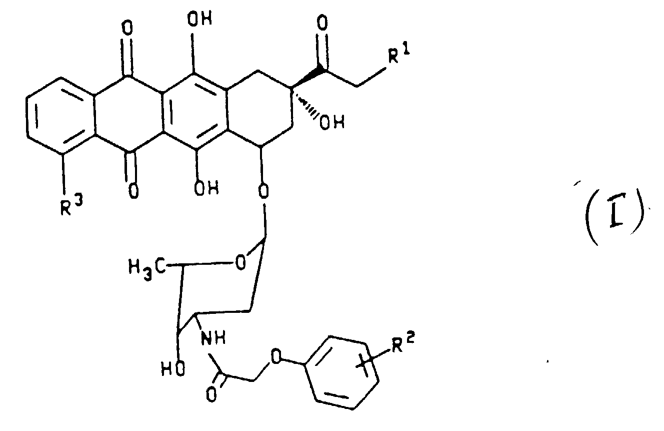 wherein:
R¹ is H, and R³ is OH or OCH₃; or
R¹ is OH and R³ is OCH₃; and
R² is H or OH, or
a compound having the formula 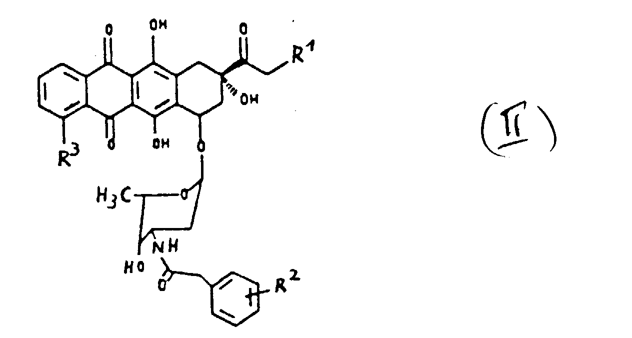 wherein:
R¹ is H, and R³ is OH or OCH₃; or
R¹ is OH and R³ is OCH₃; and
R² is H or OH.

2. N-(p-hydroxyphenoxyacetyl)adriamycin or N-(phenoxyacetyl)adriamycin.

3. The use of at least one prodrug of claim 1 or 2 that is weakly cytotoxic to tumor cells compared to its corresponding parent drug and of at least one antibody-enzyme conjugate comprising an antibody reactive with an antigen on the surface of tumor cells conjugated to an enzyme capable of converting said prodrug into the more cytotoxic parent drug, for preparing a pharmaceutical composition for the treatment of tumors.

4. The use of claim 3, wherein the antibody is selected from the group consisting of polyclonal, monoclonal or chimeric antibodies.

5. The use of claim 3, wherein the antibody is selected from the group consisting of monoclonal antibodies L6, 96,5 and 1F5.

6. The use of claim 3, wherein the enzyme is selected from penicillin amidases.

7. The use of claim 3, wherein the enzyme is penicillin V amidase or penicillin G amidase.

8. The use of claim 3, wherein the prodrug is selected from the group consisting of N-(p-hydroxyphenoxyacetyl)-adriamycin, N-(phenoxyacetyl)adriamycin, N-(p-hydroxyphenylacetyl) adriamycin or N-(phenylacetyl)adriamycin.

9. The use of claim 3, wherein the antibody-enzyme conjugate is L6-PVA or 1F5-PVA.

10. The use of claim 3, wherein the antibody-enzyme conjugate is L6-PVA and the prodrug is selected from the group consisting of N-(p-hydroxyphenoxyacetyl)adriamycin and N-(phenoxyacetyl)adriamycin.

11. The use of claim 3, wherein the tumor cells are of an origin selected from the group consisting of carcinomas, melanomas, lymphomas, bone and soft tissue sarcomas and other tumors.

12. The use of anyone of the claims 3 to 11 wherein more than one prodrug is used, each of which is weakly cytotoxic to tumor cells compared to its corresponding parent drug.

13. The use of claim 12, wherein the prodrugs are selected from the group consisting of N-phenoxyacetyl derivatives of adriamycin , and N-phenylacetyl derivatives of adriamycin.

14. The use of claim 3, wherein more than one antibody-enzyme conjugate is used, wherein the antibody of each conjugate is reactive with the same or a different antigen located on the surface of said tumor cells and the enzyme of each conjugate is the same or different and is capable of converting at least one prodrug, that is weakly cytotoxic to tumor cells compared to its corresponding parent drug, into the more cytotoxic parent drug.

15. A pharmaceutically acceptable composition consisting of two separate components useful in the treatment of tumors which comprises as one component a pharmaceutically effective amount of at least one prodrug according to claim 1 or 2.

16. A combination of two separate components, wherein one component comprises at least one antibody-enzyme conjugate and the other component comprises at least one prodrug that is weakly cytotoxic to tumor cells compared to its corresponding parent drug, as defined in anyone of claims 1 to 14.

17. The combination of claim 16, wherein the antibody-enzyme conjugate is L6-PVA.

18. The combination of claim 16, wherein the prodrug is selected from the group consisting of N-phenoxyacetyl derivatives of adriamycin and N-phenylacetyl derivatives of adriamycin.

19. The use of Claim 3, wherein the antibody-enzyme conjugate is a fusion protein comprising at least the antigen binding region of an antibody reactive with a tumor-associated antigen linked to at least a functionally active portion of an enzyme capable of converting at least one weakly cytotoxic prodrug into its more cytotoxic parent drug.

20. A process for the preparation of the compounds of claims 1 and 2, characterized in that, a compound of the general formula 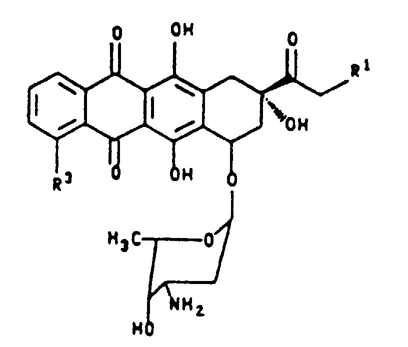
a) is reacted with p-hydroxy phenoxy acetic acid or phenoxy acetic acid, or an activated derivative therefrom, preferably activated with N-hydroxysuccinimide or dicyclo hexylcarbodiimide, to form a compound of the general formula (I); or
b) is reacted with phenyl acetic acid or hydroxyphenyl acetic acid or an activated derivative therefrom, preferably activated with N-hydroxysuccinimide or dicyclo hexylcarbodiimide, to form a compound of the general formula (II).

21. A process for preparing the pharmaceutical composition of claim 15 which comprises providing at least one prodrug according to claim 1 or 2 in a suitable dosage form.

22. A process for preparing the combinations of claims 16 to 18 which comprises combining said antibody enzyme conjugate and said prodrug.

## Claims (Claims for the following Contracting State(s): ES)

1. A process of preparing a compound having the formula: 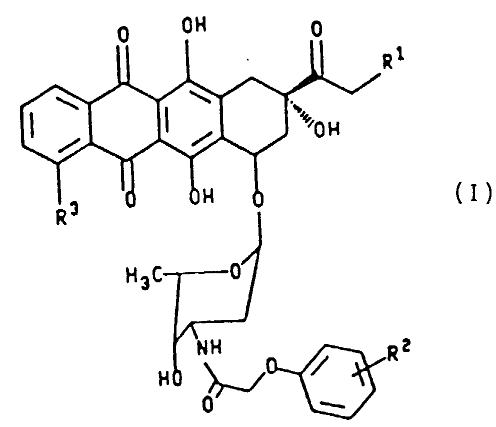 wherein:
R¹ is H, and R³ is OH or OCH₃; or
R¹ is OH and R³ is OCH₃; and
R² is H or OH; or
a compound having the formula 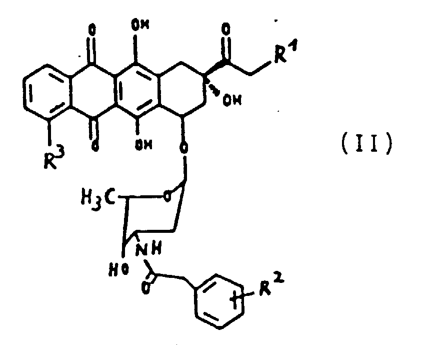 wherein:
R¹ is H, and R³ is OH or OCH₃; or
R¹ is OH and R³ is OCH₃; and
R² is H or OH.
characterized in that, a compound of the general formula 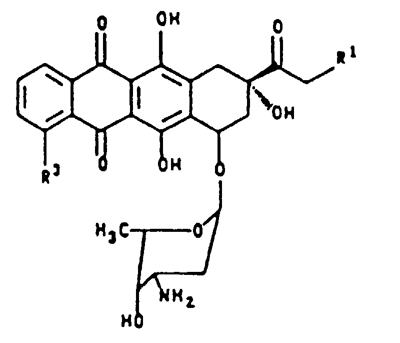
a) is reacted with p-hydroxy phenoxy acetic acid or phenoxy acetic acid, or an activated derivative therefrom, preferably activated with N-hydroxysuccinimide or dicyclo hexylcarbodiimide, to form a compound of the general formula (I): or
b) is reacted with phenyl acetic acid or hydroxyphenyl acetic acid or an activated derivative therefrom, preferably activated with N-hydroxysuccinimide or dicyclo hexylcarbodiimide, to form a compound of the general formula (II).

2. The process of claim 1, wherein N-(p-hydroxyphenoxyacetyl)adriamycin or N-(phenoxyacetyl)adriamycin is obtained.

3. The use of at least one prodrug prepared according to claim 1 or 2 that is weakly cytotoxic to tumor cells compared to its corresponding parent drug and of at least one antibody-enzyme conjugate comprising an antibody reactive with an antigen on the surface of tumor cells conjugated to an enzyme capable of converting said prodrug into the more cytotoxic parent drug, for preparing a pharmaceutical composition for the treatment of tumors.

4. The use of claim 3, wherein the antibody is selected from the group consisting of polyclonal, monoclonal or chimeric antibodies.

5. The use of claim 3, wherein the antibody is selected from the group consisting of monoclonal antibodies L6, 96,5 and 1F5.

6. The use of claim 3, wherein the enzyme is selected from penicillin amidases.

7. The use of claim 3, wherein the enzyme is penicillin V amidase of penicillin G amidase.

8. The use of claim 3, wherein the prodrug is selected from the group consisting of N-(p-hydroxyphenoxyacetyl)-adriamycin, N-(phenoxyacetyl)adriamycin, N-(p-hydroxyphenylacetyl)adriamycin or N-(phenylacetyl)adriamycin.

9. The use of claim 3, wherein the antibody-enzyme conjugate is L6-PVA or 1F5-PVA.

10. The use of claim 3, wherein the antibody-enzyme conjugate is L6-PVA and the prodrug is selected from the group consisting of N-(p-hydroxyphenoxyacetyl)adriamycin and N-(phenoxyacetyl)adriamycin.

11. The use of claim 3, wherein the tumor cells are of an origin selected from the group consisting of carcinomas, melanomas, lymphomas, bone and soft tissue sarcomas and other tumors.

12. The use of anyone of the claims 3 to 11 wherein more than one prodrug is used, each of which is weakly cytotoxic to tumor cells compared to its corresponding parent drug.

13. The use of claim 12, wherein the prodrugs are selected from the group consisting of N-phenoxyacetyl derivatives of adriamycin and N-phenylacetyl derivatives of adriamycin.

14. The use of claim 3, wherein more than one antibody-enzyme conjugate is used, wherein the antibody of each conjugate is reactive with the same or a different antigen located on the surface of said tumor cells and the enzyme of each conjugate is the same or different and is capable of converting at least one prodrug, that is weakly cytotoxic to tumor cells compared to its corresponding parent drug, into the more cytotoxic parent drug.

15. A process of preparing a pharmaceutically acceptable composition consisting of two separate components useful in the treatment of tumors which process comprises providing as one component a pharmaceutically effective amount of at least one prodrug prepared according to claim 1 or 2.

16. A process of preparing a combination of two separate components, wherein one component comprises at least one antibody-enzyme conjugate and the other component comprises at least one prodrug that is weakly cytotoxic to tumor cells compared to its corresponding parent drug, as defined in anyone of claims 1 to 14, which process comprises combining said antibody enzyme conjugate and said prodrug.

17. The process of claim 16, wherein the antibody-enzyme conjugate is L6-PVA.

18. The process of claim 16, wherein the prodrug is selected from the group consisting of N-phenoxyacetyl derivatives of adriamycin and N-phenylacetyl derivatives of adriamycin.

19. The use of claim 3, wherein the antibody-enzyme conjugate is a fusion protein comprising at least the antigen binding region of an antibody reactive with a tumor-associated antigen linked to at least a functionally active portion of an enzyme capable of converting at least one weakly cytotoxic prodrug into its more cytotoxic parent drug.

## Claims (Claims for the following Contracting State(s): GR)

1. A compound having the formula: 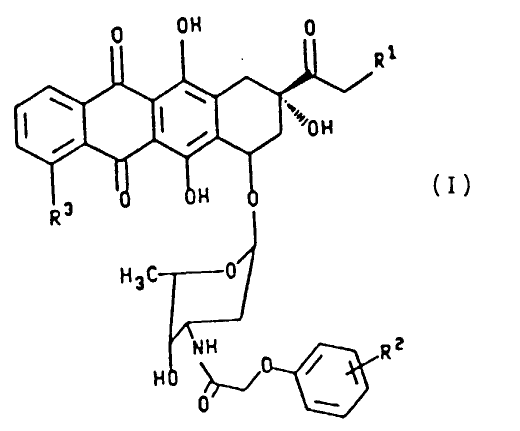 wherein:
R¹ is H, and R³ is OH or OCH₃; or
R¹ is OH and R³ is OCH₃; and
R² is H or OH, or
a compound having the formula 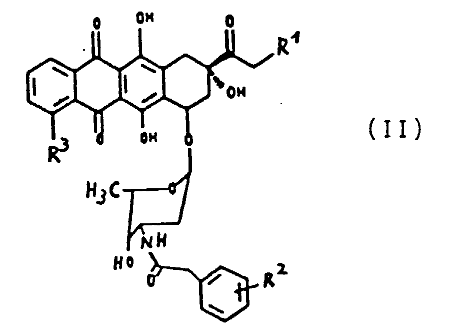 wherein:
R¹ is H, and R³ is OH or OCH₃; or
R¹ is OH and R³ is OCH₃; and
R² is H or OH.

2. N-(p-hydroxyphenoxyacetyl)adriamycin or N-(phenoxyacetyl) adriamycin.

3. The use of at least one prodrug of claim 1 or 2 that is weakly cytotoxic to tumor cells compared to its corresponding parent drug and of at least one antibody-enzyme conjugate comprising an antibody reactive with an antigen on the surface of tumor cells conjugated to an enzyme capable of converting said prodrug into the more cytotoxic parent drug, for preparing a pharmaceutical composition for the treatment of tumors.

4. The use of claim 3, wherein the antibody is selected from the group consisting of polyclonal, monoclonal or chimeric antibodies.

5. The use of claim 3, wherein the antibody is selected from the group consisting of monoclonal antibodies L6, 96,5 and 1F5.

6. The use of claim 3, wherein the enzyme is selected from penicillin amidases.

7. The use of claim 3, wherein the enzyme is penicillin V amidase of penicillin G amidase.

8. The use of claim 3, wherein the prodrug is selected from the group consisting of N-(p-hydroxyphenoxyacetyl)-adriamycin, N-(phenoxyacetyl) adriamycin, N- (p-hydroxyphenylacetyl) adriamycin or N-(phenylacetyl) adriamycin.

9. The use of claim 3, wherein the antibody-enzyme conjugate is L6-PVA or 1F5-PVA.

10. The use of claim 3, wherein the antibody-enzyme conjugate is L6-PVA and the prodrug is selected from the group consisting of N-(p-hydroxyphenoxyacetyl)adriamycin and N-(phenoxyacetyl) adriamycin.

11. The use of claim 3, wherein the tumor cells are of an origin selected from the group consisting of carcinomas, melanomas, lymphomas, bone and soft tissue sarcomas and other tumors.

12. The use of anyone of the claims 3 to 11 wherein more than one prodrug is used, each of which is weakly cytotoxic to tumor cells compared to its corresponding parent drug.

13. The use of claim 12, wherein the prodrugs are selected from the group consisting of N-phenoxyacetyl derivatives of adriamycin and N-phenylacetyl derivatives of adriamycin.

14. The use of claim 3, wherein more than one antibody-enzyme conjugate is used, wherein the antibody of each conjugate is reactive with the same or a different antigen located on the surface of said tumor cells and the enzyme of each conjugate is the same or different and is capable of converting at least one prodrug, that is weakly cytotoxic to tumor cells compared to its corresponding parent drug, into the more cytotoxic parent drug.

15. A process of preparing a pharmaceutically acceptable composition consisting of two separate components useful in the treatment of tumors which process comprises providing as one component a pharmaceutically effective amount of at least one prodrug according to claim 1 or 2.

16. A combination of two separate components, wherein one component comprises at least one antibody-enzyme conjugate and the other component comprises at least one prodrug that is weakly cytotoxic to tumor cells compared to its corresponding parent drug, as defined in anyone of claims 1 to 14.

17. The combination of claim 16, wherein the antibody-enzyme conjugate is L6-PVA.

18. The combination of claim 16, wherein the prodrug is selected from the group consisting of N-phenoxyacetyl derivatives of adriamycin and N-phenylacetyl derivatives of adriamycin.

19. The use of claim 3, wherein the antibody enzyme conjugate is a fusion protein comprising at least the antigen binding region of an antibody reactive with a tumor-associated antigen linked to at least a functionally active portion of an enzyme capable of converting at least one weakly cytotoxic prodrug into its more cytotoxic parent drug.

20. A process for the preparation of the compounds of claims 1 and 2, characterized in that, a compound of the general formula 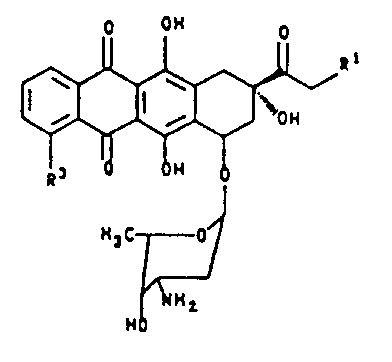
a) is reacted with p-hydroxy phenoxy acetic acid or phenoxy acetic acid, or an activated derivative therefrom, preferably activated with N-hydroxy-succinimide or dicyclo hexylcarbodiimide, to form a compound of the general formula (I); or
b) is reacted with phenyl acetic acid or hydroxyphenyl acetic acid or an activated derivative therefrom, preferably activated with N-hydroxysuccinimide or dicyclo hexylcarbodiimide, to form a compound of the general formula (II).

21. A process for preparing the combinations of claims 16 to 18 which comprises combining said antibody enzyme conjugate and said prodrug.

## Patentansprüche (Patentansprüche für folgende(n) Vertragsstaat(en): AT, BE, CH, DE, FR, GB, IT, LI, LU, NL, SE)

1. Verbindung der Formel: 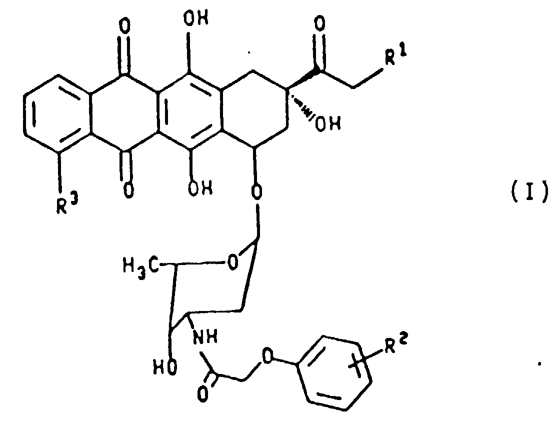 worin
R¹ für H und R³ für OH oder OCH₃ steht; oder
R¹ für OH und R³ für OCH₃ steht; und
R² für H oder OH steht, oder eine
Verbindung der Formel: 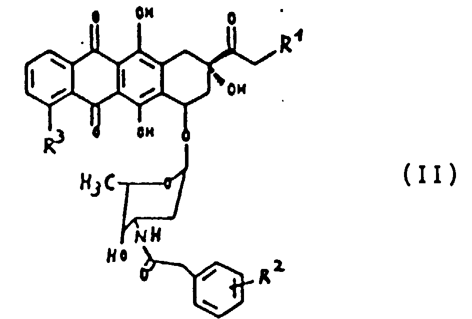 worin
R¹ für H und R³ für OH oder OCH₃ steht; oder
R¹ für OH und R³ für OCH₃ steht; und
R² für H oder OH steht.

2. N-(p-Hydroxyphenoxyacetyl)adriamycin oder N-(Phenoxyacetyl)adriamycin.

3. Verwendung wenigstens einer Prodrug nach Anspruch 1 oder 2, die im Vergleich zu ihrer Parent-Drug schwach cytotoxisch gegenüber Tumorzellen ist, und wenigstens eines Antikörper-Enzym-Konjugates, umfassend einen mit einem zur Umwandlung der Prodrug in die stärker cytotoxische Parent-Drug befähigten Enzym konjugierten Antikörper, der gegenüber einem Antigen auf der Oberfläche von Tumorzellen reaktiv ist, zur Herstellung eines pharmazeutischen Mittels zur Behandlung von Tumoren.

4. Verwendung nach Anspruch 3, wobei der Antikörper ausgewählt ist unter polyklonalen, monoklonalen oder chimären Antikörpern.

5. Verwendung nach Anspruch 3, wobei der Antikörper ausgewählt ist unter den monoklonalen Antikörpern L6, 96,5 und 1F5.

6. Verwendung nach Anspruch 3, wobei das Enzym ausgewählt ist unter Penicillin-Amidasen.

7. Verwendung nach Anspruch 3, wobei das Enzym die Penicillin V-Amidase oder Penicillin G-Amidase ist.

8. Verwendung nach Anspruch 3, wobei die Prodrug ausgewählt ist unter N-(p-Hydroxyphenoxyacetyl)adriamycin, N-(Phenoxyacetyl)adriamycin, N-(p-Hydroxyphenylacetyl)adriamycin oder N-(Phenylacetyl)adriamycin.

9. Verwendung nach Anspruch 3, wobei das Antikörper-Enzym-Konjugat L6-PVA oder 1F5-PVA ist.

10. Verwendung nach Anspruch 3, wobei das Antikörper-Enzym-Konjugat L6-PVA ist und die Prodrug ausgewählt ist unter N-(p-Hydroxyphenoxyacetyl)adriamcyin und N-(Phenoxyacetyl)adriamycin.

11. Verwendung nach Anspruch 3, wobei die Tumorzellen von Karzinomen, Melanomen, Lymphomen, Knochensarkomen und Weichteil-Sarkomen und anderen Tumoren stammen.

12. Verwendung nach einem der Ansprüche 3 bis 11, wobei mehr als eine Prodrug verwendet werden, welche, im Vergleich zu ihrer Parent-Drug, jeweils schwach cytotoxisch gegenüber Tumorzellen sind.

13. Verwendung nach Anspruch 12, wobei die Prodrugs ausgewählt sind unter N-Phenoxyacetyl-Derivaten von Adriamycin und N-Phenylacetyl-Derivaten von Adriamycin.

14. Verwendung nach Anspruch 3, wobei mehr als ein Antikörper-Enzym-Konjugat verwendet wird, wobei die Antikörper der einzelnen Konjugate jeweils gegenüber dem gleichen oder einem anderen auf der Oberfläche des Tumors lokalisierten Antigen reaktiv sind und wobei die Enzyme der Konjugate gleich oder verschieden sind und die Fähigkeit besitzen, wenigstens eine Prodrug, die im Vergleich zu ihrer entsprechenden Parent-Drug schwach cytotoxisch gegenüber Tumorzellen ist, in die stärker cytotoxische Parent-Drug umzuwandeln.

15. Pharmazeutisch verträgliches Mittel, das aus zwei getrennten, zur Behandlung von Tumoren brauchbaren Komponenten besteht und als eine Komponente eine pharmazeutisch wirksame Menge wenigstens einer Prodrug nach Anspruch 1 oder 2 umfaßt.

16. Kombination von zwei getrennten Komponenten, wobei eine Komponente wenigstens ein Antikörper-Enzym-Konjugat und die andere Komponente wenigstens eine im Vergleich zu ihrer entsprechenden Parent-Drug gegenüber Tumorzellen schwach cytotoxische Prodrug nach einem der Ansprüche 1 bis 14 umfaßt.

17. Kombination nach Anspruch 16, wobei das Antikörper-Enzym-Konjugat L6-PVA ist.

18. Kombination nach Anspruch 16, wobei die Prodrug ausgewählt ist unter N-Phenoxyacetyl-Derivaten von Adriamycin und N-Phenylacetyl-Derivaten von Adriamycin.

19. Verwendung nach Anspruch 3, wobei das Antikörper-Enzym-Konjugat ein Fusionsprotein ist, das wenigstens die Antigenbindungsregion eines Antikörpers umfaßt, der gegenüber einem Tumor-assoziierten Antigen reaktiv ist und mit wenigstens einem funktionell aktiven Bereich eines Enzyms verknüpft ist, das zur Umwandlung wenigstens einer schwach cytotoxischen Prodrug in ihre stärker cytotoxische Parent-Drug befähigt ist.

20. Verfahren zur Herstellung von Verbindungen nach Anspruch 1 und 2, dadurch gekennzeichnet, daß eine Verbindung der allgemeinen Formel 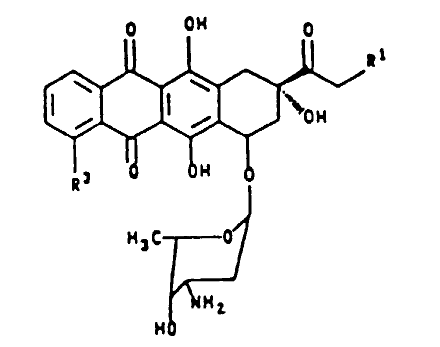
a) mit p-Hydroxyphenoxyessigsäure oder Phenoxyessigsäure oder einem aktivierten Derivat davon, das vorzugsweise mit N-Hydroxysuccinimid oder Dicyclohexylcarbodiimid aktiviert ist, zu einer Verbindung der allgemeinen Formel (I) umgesetzt wird; oder
b) mit Phenylessigsäure oder Hydroxyphenylessigsäure oder einem aktivierten Derivat davon, das vorzugsweise mit N-Hydroxysuccinimid oder Dicyclohexylcarbodiimid aktiviert ist, zu einer Verbindung der allgemeinen Formel (II) umgesetzt wird.

21. Verfahren zur Herstellung des pharmazeutischen Mittels nach Anspruch 15, wobei wenigstens eine Prodrug nach Anspruch 1 oder 2 in einer geeigneten Dosisform bereitgestellt wird.

22. Verfahren zur Herstellung der Kombinationen nach Anspruch 16 bis 18, umfassend das Kombinieren des Antikörper-Enzym-Konjugates und der Prodrug.

## Patentansprüche (Patentansprüche für folgende(n) Vertragsstaat(en): ES)

1. Verfahren zur Herstellung einer Verbindung der Formel: 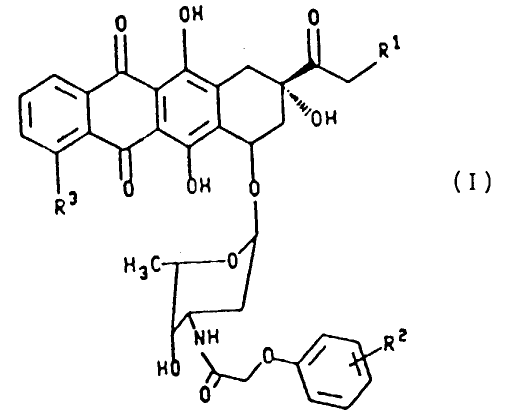 worin
R¹ für H und R³ für OH oder OCH₃ steht; oder
R¹ für OH und R³ für OCH₃ steht; und
R² für H oder OH steht, oder
einer Verbindung der Formel: 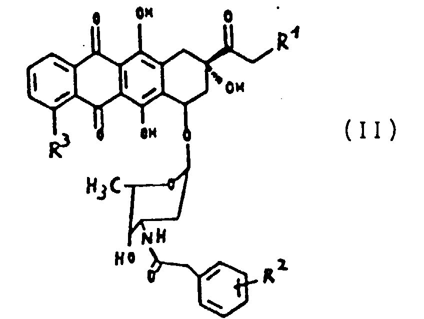 worin
R¹ für H und R³ für OH oder OCH₃ steht; oder
R¹ für OH und R³ für OCH₃ steht; und
R² für H oder OH steht,
dadurch gekennzeichnet, daß eine Verbindung der allgemeinen Formel 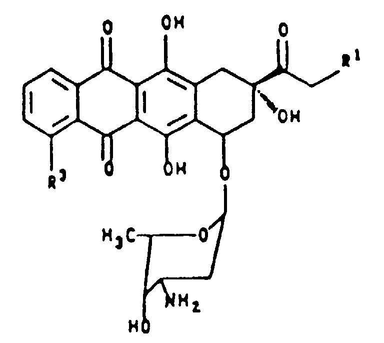
a) mit p-Hydroxyphenoxyessigsäure oder Phenoxyessigsäure oder einem aktivierten Derivat davon, das vorzugsweise mit N-Hydroxysuccinimid oder Dicyclohexylcarbodiimid aktiviert ist, zu einer Verbindung der allgemeinen Formel (I) umgesetzt wird; oder
b) mit Phenylessigsäure oder Hydroxyphenylessigsäure oder einem aktivierten Derivat davon, das vorzugsweise mit N-Hydroxysuccinimid oder Dicyclohexylcarbodiimid aktiviert ist, zu einer Verbindung der allgemeinen Formel (II) umgesetzt wird.

2. Verfahren nach Anspruch 1, wobei man N-(p-Hydroxyphenoxyacetyl)adriamycin oder N-(Phenoxyacetyl)adriamycin erhält.

3. Verwendung wenigstens einer Prodrug nach Anspruch 1 oder 2, die im Vergleich zu ihrer Parent-Drug schwach cytotoxisch gegenüber Tumorzellen ist, und wenigstens eines Antikörper-Enzym-Konjugates, umfassend einen mit einem zur Umwandlung der Prodrug in die stärker cytotoxische Parent-Drug befähigten Enzym konjugierten Antikörper, der gegenüber einem Antigen auf der Oberfläche von Tumorzellen reaktiv ist, zur Herstellung eines pharmazeutischen Mittels zur Behandlung von Tumoren.

4. Verwendung nach Anspruch 3, wobei der Antikörper ausgewählt ist unter polyklonalen, monoklonalen oder chimären Antikörpern.

5. Verwendung nach Anspruch 3, wobei der Antikörper ausgewählt ist unter den monoklonalen Antikörpern L6, 96,5 und 1F5.

6. Verwendung nach Anspruch 3, wobei das Enzym ausgewählt ist unter Penicillin-Amidasen.

7. Verwendung nach Anspruch 3, wobei das Enzym die Penicillin V-Amidase oder Penicillin G-Amidase ist.

8. Verwendung nach Anspruch 3, wobei die Prodrug ausgewählt ist unter N-(p-Hydroxyphenoxyacetyl)adriamycin, N-(Phenoxyacetyl)adriamycin, N-(p-Hydroxyphenylacetyl)adriamycin oder N-(Phenylacetyl)adriamycin.

9. Verwendung nach Anspruch 3, wobei das Antikörper-Enzym-Konjugat L6-PVA oder 1F5-PVA ist.

10. Verwendung nach Anspruch 3, wobei das Antikörper-Enzym-Konjugat L6-PVA ist und die Prodrug ausgewählt ist unter N-(p-Hydroxyphenoxyacetyl)adriamcyin und N-(Phenoxyacetyl)adriamycin.

11. Verwendung nach Anspruch 3, wobei die Tumorzellen von Karzinomen, Melanomen, Lymphomen, Knochensarkomen und Weichteil-Sarkomen und anderen Tumoren stammen.

12. Verwendung nach einem der Ansprüche 3 bis 11, wobei mehr als eine Prodrug verwendet werden, welche, im Vergleich zu ihrer Parent-Drug, jeweils schwach cytotoxisch gegenüber Tumorzellen sind.

13. Verwendung nach Anspruch 12, wobei die Prodrugs ausgewählt sind unter N-Phenoxyacetyl-Derivaten von Adriamycin und N-Phenylacetyl-Derivaten von Adriamycin.

14. Verwendung nach Anspruch 3, wobei mehr als ein Antikörper-Enzym-Konjugat verwendet wird, wobei die Antikörper der einzelnen Konjugate jeweils gegenüber dem gleichen oder einem anderen auf der Oberfläche des Tumors lokalisierten Antigen reaktiv sind und wobei die Enzyme der Konjugate gleich oder verschieden sind und die Fähigkeit besitzen, wenigstens eine Prodrug, die im Vergleich zu ihrer entsprechenden Parent-Drug schwach cytotoxisch gegenüber Tumorzellen ist, in die stärker cytotoxische Parent-Drug umzuwandeln.

15. Verfahren zur Herstellung eines pharmazeutisch verträglichen Mittels, das aus zwei getrennten, zur Behandlung von Tumoren brauchbaren Komponenten besteht, wobei das Verfahren die Bereitstellung einer pharmazeutisch wirksamen Menge wenigstens einer Prodrug nach Anspruch 1 oder 2 als eine Komponente umfaßt.

16. Verfahren zur Herstellung einer Kombination von zwei getrennten Komponenten, wobei eine Komponente wenigstens ein Antikörper-Enzym-Konjugat und die andere Komponente wenigstens eine im Vergleich zu ihrer entsprechenden Parent-Drug gegenüber Tumorzellen schwach cytotoxische Prodrug nach einem der Ansprüche 1 bis 14 umfaßt und wobei man bei diesem Verfahren das Antikörper-Enzym-Konjugat mit der Prodrug kombiniert.

17. Verfahren nach Anspruch 16, wobei das Antikörper-Enzym-Konjugat L6-PVA ist.

18. Verfahren nach Anspruch 16, wobei die Prodrug ausgewählt ist unter N-Phenoxyacetyl-Derivaten von Adriamycin und N-Phenylacetyl-Derivaten von Adriamycin.

19. Verwendung nach Anspruch 3, wobei das Antikörper-Enzym-Konjugat ein Fusionsprotein ist, das wenigstens die Antigenbindungsregion eines Antikörpers umfaßt, der gegenüber einem Tumor-assoziierten Antigen reaktiv ist und mit wenigstens einem funktionell aktiven Bereich eines Enzyms verknüpft ist, das zur Umwandlung wenigstens einer schwach cytotoxischen Prodrug in ihre stärker cytotoxische Parent-Drug befähigt ist.

## Patentansprüche (Patentansprüche für folgende(n) Vertragsstaat(en): GR)

1. Verbindung der Formel: 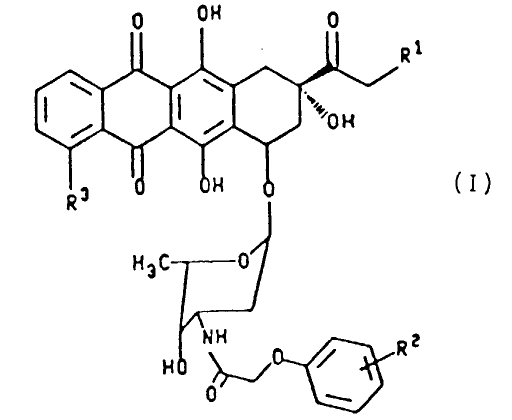 worin
R¹ für H und R³ für OH oder OCH₃ steht; oder
R¹ für OH und R³ für OCH₃ steht; und
R² für H oder OH steht, oder eine
Verbindung der Formel: 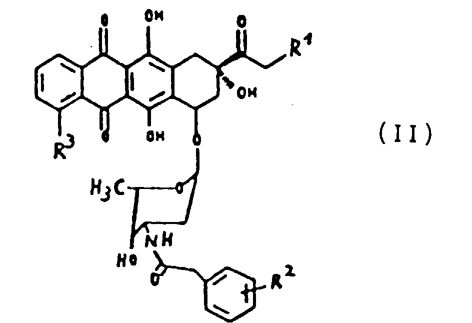 worin
R¹ für H und R³ für OH oder OCH₃ steht; oder
R¹ für OH und R³ für OCH₃ steht; und
R² für H oder OH steht.

2. N-(p-Hydroxyphenoxyacetyl)adriamycin oder N-(Phenoxyacetyl)adriamycin.

3. Verwendung wenigstens einer Prodrug nach Anspruch 1 oder 2, die im Vergleich zu ihrer Parent-Drug schwach cytotoxisch gegenüber Tumorzellen ist, und wenigstens eines Antikörper-Enzym-Konjugates, umfassend einen mit einem zur Umwandlung der Prodrug in die stärker cytotoxische Parent-Drug befähigten Enzym konjugierten Antikörper, der gegenüber einem Antigen auf der Oberfläche von Tumorzellen reaktiv ist, zur Herstellung eines pharmazeutischen Mittels zur Behandlung von Tumoren.

4. Verwendung nach Anspruch 3, wobei der Antikörper ausgewählt ist unter polyklonalen, monoklonalen oder chimären Antikörpern.

5. Verwendung nach Anspruch 3, wobei der Antikörper ausgewählt ist unter den monoklonalen Antikörpern L6, 96,5 und 1F5.

6. Verwendung nach Anspruch 3, wobei das Enzym ausgewählt ist unter Penicillin-Amidasen.

7. Verwendung nach Anspruch 3, wobei das Enzym die Penicillin V-Amidase oder Penicillin G-Amidase ist.

8. Verwendung nach Anspruch 3, wobei die Prodrug ausgewählt ist unter N-(p-Hydroxyphenoxyacetyl)adriamycin, N-(Phenoxyacetyl)adriamycin, N-(p-Hydroxyphenylacetyl)adriamycin oder N-(Phenylacetyl)adriamycin.

9. Verwendung nach Anspruch 3, wobei das Antikörper-Enzym-Konjugat L6-PVA oder 1F5-PVA ist.

10. Verwendung nach Anspruch 3, wobei das Antikörper-Enzym-Konjugat L6-PVA ist und die Prodrug ausgewählt ist unter N-(p-Hydroxyphenoxyacetyl)adriamcyin und N-(Phenoxyacetyl)adriamycin.

11. Verwendung nach Anspruch 3, wobei die Tumorzellen von Karzinomen, Melanomen, Lymphomen, Knochensarkomen und Weichteil-Sarkomen und anderen Tumoren stammen.

12. Verwendung nach einem der Ansprüche 3 bis 11, wobei mehr als eine Prodrug verwendet werden, welche, im Vergleich zu ihrer Parent-Drug, jeweils schwach cytotoxisch gegenüber Tumorzellen sind.

13. Verwendung nach Anspruch 12, wobei die Prodrugs ausgewählt sind unter N-Phenoxyacetyl-Derivaten von Adriamycin und N-Phenylacetyl-Derivaten von Adriamycin.

14. Verwendung nach Anspruch 3, wobei mehr als ein Antikörper-Enzym-Konjugat verwendet wird, wobei die Antikörper der einzelnen Konjugate jeweils gegenüber dem gleichen oder einem anderen auf der Oberfläche des Tumors lokalisierten Antigen reaktiv sind und wobei die Enzyme der Konjugate gleich oder verschieden sind und die Fähigkeit besitzen, wenigstens eine Prodrug, die im Vergleich zu ihrer entsprechenden Parent-Drug schwach cytotoxisch gegenüber Tumorzellen ist, in die stärker cytotoxische Parent-Drug umzuwandeln.

15. Verfahren zur Herstellung eines pharmazeutisch verträglichen Mittels, das aus zwei getrennten, zur Behandlung von Tumoren brauchbaren Komponenten besteht, wobei das Verfahren die Bereitstellung einer pharmazeutisch wirksamen Menge wenigstens einer Prodrug nach Anspruch 1 oder 2 als eine Komponente umfaßt.

16. Kombination von zwei getrennten Komponenten, wobei eine Komponente wenigstens ein Antikörper-Enzym-Konjugat und die andere Komponente wenigstens eine im Vergleich zu ihrer entsprechenden Parent-Drug gegenüber Tumorzellen schwach cytotoxische Prodrug nach einem der Ansprüche 1 bis 14 umfaßt.

17. Kombination nach Anspruch 16, wobei das Antikörper-Enzym-Konjugat L6-PVA ist.

18. Kombination nach Anspruch 16, wobei die Prodrug ausgewählt ist unter N-Phenoxyacetyl-Derivaten von Adriamycin und N-Phenylacetyl-Derivaten von Adriamycin.

19. Verwendung nach Anspruch 3, wobei das Antikörper-Enzym-Konjugat ein Fusionsprotein ist, das wenigstens die Antigenbindungsregion eines Antikörpers umfaßt, der gegenüber einem Tumor-assoziierten Antigen reaktiv ist und mit wenigstens einem funktionell aktiven Bereich eines Enzyms verknüpft ist, das zur Umwandlung wenigstens einer schwach cytotoxischen Prodrug in ihre stärker cytotoxische Parent-Drug befähigt ist.

20. Verfahren zur Herstellung von Verbindungen nach Anspruch 1 und 2, dadurch gekennzeichnet, daß eine Verbindung der allgemeinen Formel 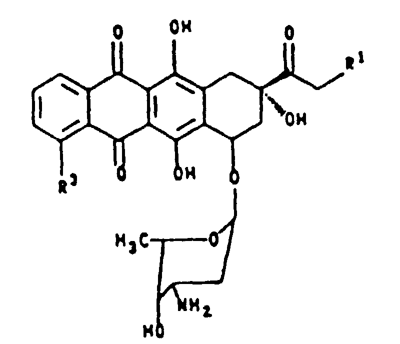
a) mit p-Hydroxyphenoxyessigsäure oder Phenoxyessigsäure oder einem aktivierten Derivat davon, das vorzugsweise mit N-Hydroxysuccinimid oder Dicyclohexylcarbodiimid aktiviert ist, zu einer Verbindung der allgemeinen Formel (I) umgesetzt wird; oder
b) mit Phenylessigsäure oder Hydroxyphenylessigsäure oder einem aktivierten Derivat davon, das vorzugsweise mit N-Hydroxysuccinimid oder Dicyclohexylcarbodiimid aktiviert ist, zu einer Verbindung der allgemeinen Formel (II) umgesetzt wird.

21. Verfahren zur Herstellung der Kombinationen nach Anspruch 16 bis 18, wobei man das Antikörper-Enzym-Konjugat mit der Prodrug kombiniert.

## Revendications (Revendications pour l'(les) Etat(s) contractant(s) suivant(s): AT, BE, CH, DE, FR, GB, IT, LI, LU, NL, SE)

1. Composé ayant pour formule : 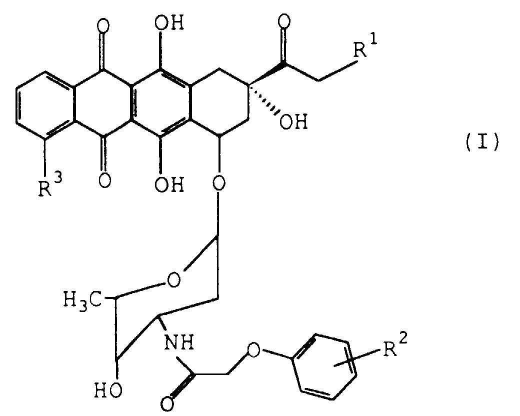 où :
R¹ est H, et R³ est OH ou OCH₃; ou bien
R¹ est OH et R³ est OCH₃; et
R² est H ou OH, ou bien
un composé ayant pour formule 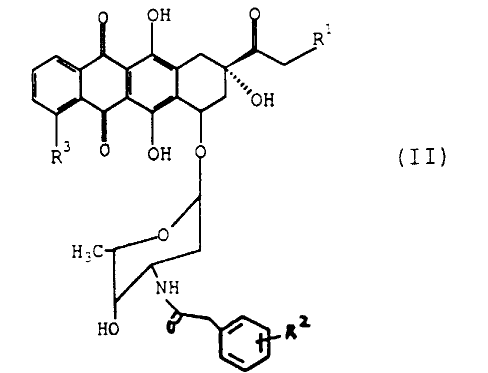 où
R¹ est H, et R³ est OH ou OCH₃; ou
R¹ est OH et R³ est OCH₃; et
R² est H ou OH.

2. N-(p-hydroxyphénoxyacétyl)adriamycine ou N-(phénoxyacétyl)adriamycine.

3. Utilisation d'au moins une pro-drogue de la revendication 1 ou 2 qui est faiblement cytotoxique vis-à-vis des cellules tumorales en comparaison avec son médicament parent correspondant et d'au moins un conjugué anticorps-enzyme comprenant un anticorps réactif avec un antigène à la surface des cellules tumorales conjugué avec une enzyme capable de convertir ladite pro-drogue en le médicament parent plus cytotoxique, pour la préparation d'une composition pharmaceutique pour le traitement des tumeurs.

4. Utilisation de la revendication 3, où l'anticorps est sélectionné dans le groupe consistant en anticorps polyclonaux, monoclonaux ou chimériques.

5. Utilisation de la revendication 3, où l'anticorps est sélectionné dans le groupe consistant en anticorps monoclonaux L6, 96,5 et 1F5.

6. Utilisation de la revendication 3, où l'enzyme est sélectionnée parmi les pénicillines amidases.

7. Utilisation de la revendication 3, où l'enzyme est la pénicilline V amidase ou la pénicilline G amidase.

8. Utilisation de la revendication 3, où la pro-drogue est sélectionnée dans le groupe consistant en N-(p-hydroxyphénoxyacétyl)-adriamycine, N-(phénoxyacétyl) adriamycine, N-(p-hydroxyphénylacétyl)adriamycine ou N-(phénylacétyl)adriamycine.

9. Utilisation de la revendication 3, où le conjugué anticorps enzyme est L6-PVA ou 1F5-PVA.

10. Utilisation de la revendication 3, où le conjugué anticorps enzyme est L6-PVA et la pro-drogue est sélectionnée dans le groupe consistant en N-(p-hydroxyphénoxyacétyl)adriamycine et N-(phénoxyacétyl) adriamycine.

11. Utilisation de la revendication 3, où les cellules tumorales sont d'une origine sélectionnée dans le groupe consistant en carcinomes, mélanomes, lymphomes, sarcomes des tissus osseux et mous et autres tumeurs.

12. Utilisation de l'une quelconque des revendications 3 à 11 où plus d'une pro-drogue est utilisée, chacune d'entre elles étant faiblement cytotoxique vis-à-vis des cellules tumorales en comparaison avec son médicament parent correspondant.

13. Utilisation de la revendication 12, où les prodrogues sont sélectionnées dans le groupe consistant en dérivés N-phénoxyacétylés de l'adriamycine et dérivés N-phénylacétylés de l'adriamycine.

14. Utilisation de la revendication 3, où plus d'un conjugué anticorps-enzyme est utilisé, où l'anticorps de chaque conjugué est réactif avec le même antigène ou un antigène différent placé à la surface desdites cellules tumorales et l'enzyme de chaque conjugué est la même ou différente et est capable de convertir au moins une pro-drogue qui est faiblement cytotoxique vis-à-vis des cellules tumorales, en comparaison avec son médicament parent correspondant, en le médicament parent plus cytotoxique.

15. Composition pharmaceutiquement acceptable consistant en deux composants séparés, utile dans le traitement des tumeurs qui comprend, en tant qu'un composant, une quantité pharmaceutiquement efficace d'au moins une pro-drogue selon la revendication 1 ou 2.

16. Combinaison de deux composants séparés où un composant comprend au moins un conjugué anticorps-enzyme et l'autre composant comprend au moins une pro-drogue qui est faiblement cytotoxique vis-à-vis des cellules tumorales en comparaison avec son médicament parent correspondant, tels que définis selon l'une quelconque des revendications 1 à 14.

17. Combinaison de la revendication 16, où le conjugué anticorps-enzyme est L6-PVA.

18. Combinaison de la revendication 16, où la pro-drogue est sélectionnée dans le groupe consistant en dérivés N-phénoxyacétylés de l'adriamycine et dérivés N-phénylacétylés de l'adriamycine.

19. Utilisation de la revendication 3, où le conjugué anticorps-enzyme est une protéine de fusion comprenant au moins la région de liaison de l'antigène d'un anticorps réactif avec un antigène associé à une tumeur enchaîné à au moins une portion fonctionnellement active d'une enzyme capable de convertir au moins une pro-drogue faiblement cytotoxique en son médicament parent plus cytotoxique.

20. Procédé pour la préparation des composés des revendications 1 et 2, caractérisé en ce qu'un composé de la formule générale 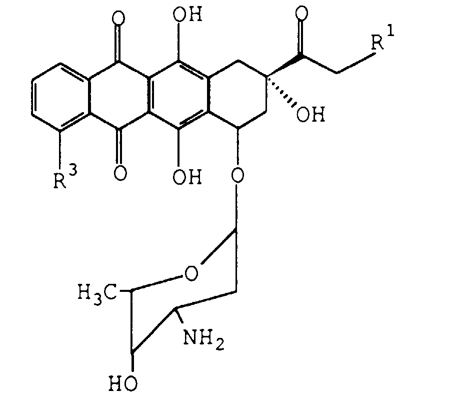
a) est mis à réagir avec de l'acide p-hydroxy phénoxy acétique ou de l'acide phénoxy acétique, ou bien un dérivé activé de ceux-ci, de préférence activé avec du N-hydroxy-succinimide ou du dicyclo hexylcarbodiimide, pour former un composé de la formule générale (I); ou
b) est mis à réagir avec de l'acide phényl acétique ou de l'acide hydroxy phényl acétique ou leur dérivé activé, de préférence activé avec du N-hydroxysuccinimide ou du dicyclo hexylcarbodiimide, pour former un composé de la formule générale (II).

21. Procédé de préparation de la composition pharmaceutique de la revendication 15, qui consiste à prévoir au moins une pro-drogue selon la revendication 1 ou 2 sous une forme de dosage approprié.

22. Procédé de préparation des combinaisons des revendications 16 à 18 qui consiste à combiner ledit conjugué anticorps-enzyme et ladite pro-drogue.

## Revendications (Revendications pour l'(les) Etat(s) contractant(s) suivant(s): ES)

1. Procédé de préparation d'un composé ayant pour formule : 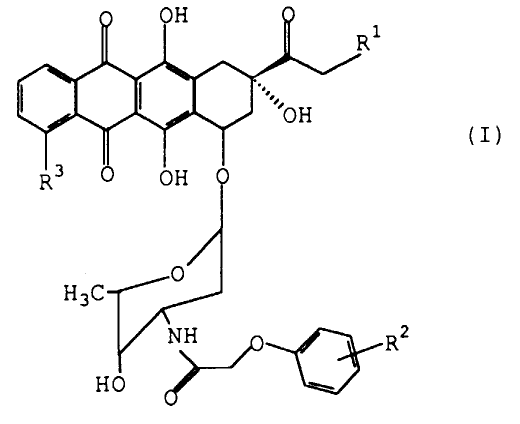 où :
R¹ est H, et R³ est OH ou OCH₃; ou
R¹ est OH et R³ est OCH₃; et
R² est H ou OH; ou
d'un composé ayant pour formule 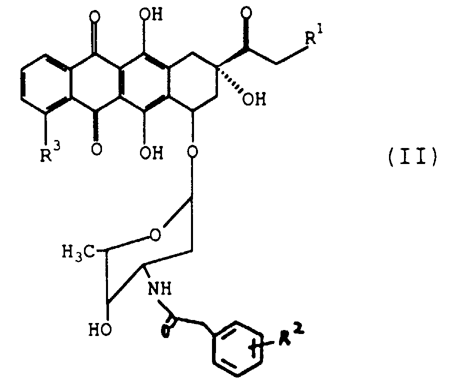 où :
R¹ est H, et R³ est OH ou OCH₃; ou
R¹ est OH et R³ est OCH₃; et
R² est H ou OH.
caractérisé en ce qu'un composé de la formule générale 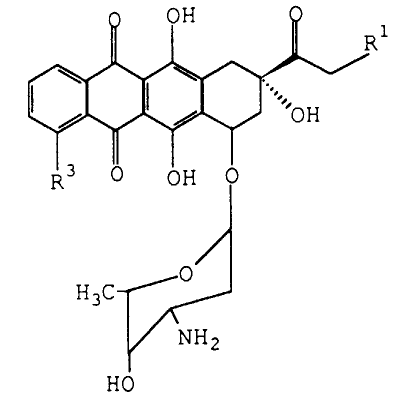
a) est mis à réagir avec de l'acide p-hydroxy phénoxy acétique ou de l'acide phénoxy acétique, ou bien un dérivé activé de ceux-ci, de préférence activé avec du N-hydroxy-succinimide ou du dicyclo hexylcarbodiimide, pour former un composé de la formule générale (I); ou
b) est mis à réagir avec de l'acide phényl acétique ou de l'acide hydroxy phényl acétique ou leur dérivé activé, de préférence activé avec du N-hydroxysuccinimide ou du dicyclo hexylcarbodiimide, pour former un composé de la formule générale (II).

2. Procédé de la revendication 1, où on obtient la N-(p-hydroxyphénoxyacétyl)adriamycine ou la N-(phénoxyacétyl)adriamycine.

3. Utilisation d'au moins une pro-drogue préparée selon la revendication 1 ou 2 qui est faiblement cytotoxique vis-à-vis des cellules tumorales en comparaison avec son médicament parent correspondant et d'au moins un conjugué anticorps-enzyme comprenant un anticorps réactif avec un antigène à la surface des cellules tumorales conjugué avec une enzyme capable de convertir ladite pro-drogue en le médicament parent plus cytotoxique, pour la préparation d'une composition pharmaceutique pour le traitement des tumeurs.

4. Utilisation de la revendication 3, où l'anticorps est sélectionné dans le groupe consistant en anticorps polyclonaux, monoclonaux ou chimériques.

5. Utilisation de la revendication 3, où l'anticorps est sélectionné dans le groupe consistant en anticorps monoclonaux L6, 96,5 et 1F5.

6. Utilisation de la revendication 3, où l'enzyme est sélectionnée parmi les pénicillines amidases.

7. Utilisation de la revendication 3, où l'enzyme est la pénicilline V amidase ou la pénicilline G amidase.

8. Utilisation de la revendication 3, où la pro-drogue est sélectionnée dans le groupe consistant en N-(p-hydroxyphénoxyacétyl)-adriamycine, N-(phénoxyacétyl)adriamycine, N-(p-hydroxyphénylacétyl)adriamycine ou N-(phénylacétyl)adriamycine.

9. Utilisation de la revendication 3, où le conjugué anticorps enzyme est L6-PVA ou 1F5-PVA.

10. Utilisation de la revendication 3, où le conjugué anticorps enzyme est L6-PVA et la pro-drogue est sélectionnée dans le groupe consistant en N-(p-hydroxyphénoxyacétyl)adriamycine et N-(phénoxyacétyl) adriamycine.

11. Utilisation de la revendication 3, où les cellules tumorales sont d'une origine sélectionnée dans le groupe consistant en carcinomes, mélanomes, lymphomes, sarcomes des tissus osseux et mous et autres tumeurs.

12. Utilisation de l'une quelconque des revendications 3 à 11 où plus d'une pro-drogue est utilisée, chacune d'entre elle est faiblement cytotoxique vis-à-vis des cellules tumorales en comparaison avec son médicament parent correspondant.

13. Utilisation de la revendication 12, où les prodrogues sont sélectionnées dans le groupe consistant en dérivés N-phénoxyacétylés de l'adriamycine et dérivés N-phénylacétylés de l'adriamycine.

14. Utilisation de la revendication 3, où plus d'un conjugué anticorps-enzyme est utilisé, où l'anticorps de chaque conjugué est réactif avec le même antigène ou un antigène différent placé à la surface desdites cellules tumorales et l'enzyme de chaque conjugué est la même ou différente et est capable de convertir au moins une pro-drogue qui est faiblement cytotoxique vis-à-vis des cellules tumorales en comparaison avec son médicament parent correspondant, en le médicament parent plus cytotoxique.

15. Procédé de préparation d'une composition pharmaceutiquement acceptable consistant en deux composants séparés, utile dans le traitement des tumeurs, lequel procédé consiste à prévoir en tant qu'un composé, une quantité pharmaceutiquement efficace d'au moins une pro-drogue préparée selon la revendication 1 ou 2.

16. Procédé de préparation d'une combinaison de deux composants séparés, où un composant comprend au moins un conjugué anticorps-enzyme et l'autre composant comprend au moins une pro-drogue qui est faiblement cytotoxique vis-à-vis des cellules tumorales, en comparaison avec son médicament parent correspondant, telle que définie selon l'une quelconque des revendications 1 à 14, lequel procédé consiste à combiner ledit conjugué anticorps-enzyme et ladite pro-drogue.

17. Procédé de la revendication 16, où le conjugué anticorps-enzyme est L6-PVA.

18. Procédé de la revendication 16, où la pro-drogue est sélectionnée dans le groupe consistant en dérivés N-phénoxyacétylés de l'adriamycine et dérivés N-phénylacétylés de l'adriamycine.

19. Utilisation de la revendication 3, où le conjugué anticorps-enzyme est une protéine de fusion comprenant au moins la région de liaison de l'antigène d'un anticorps réactif avec un antigène associé à une tumeur enchaîné à au moins une portion fonctionnellement active d'une enzyme capable de convertir au moins une pro-drogue faiblement cytotoxique en son médicament parent plus cytotoxique.

## Revendications (Revendications pour l'(les) Etat(s) contractant(s) suivant(s): GR)

1. Composé ayant pour formule : 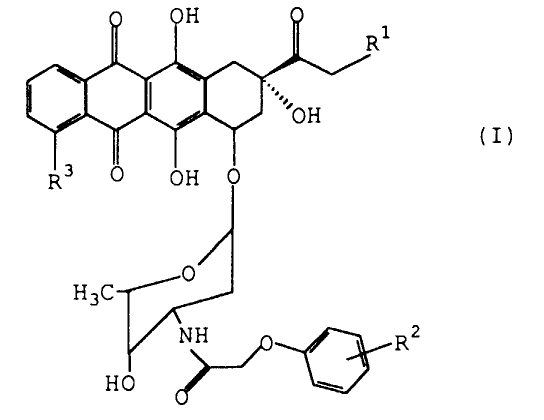 où :
R¹ est H, et R³ est OH ou OCH₃; ou bien
R¹ est OH et R³ est OCH₃; et
R² est H ou OH, ou bien
un composé ayant pour formule 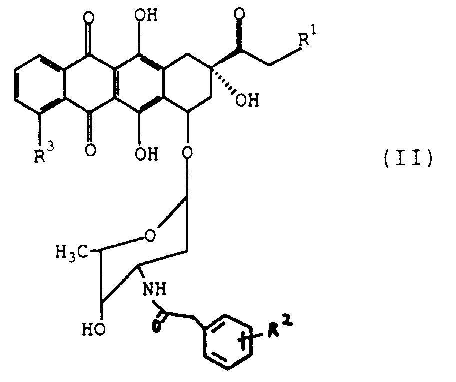 où
R¹ est H, et R³ est OH ou OCH₃ ; ou
R¹ est OH et R³ est OCH₃; et
R² est H ou OH.

2. N-(p-hydroxyphénoxyacétyl)adriamycine ou N-(phénoxyacétyl)adriamycine.

3. Utilisation d'au moins une pro-drogue de la revendication 1 ou 2 qui est faiblement cytotoxique vis-à-vis des cellules tumorales en comparaison avec son médicament parent correspondant et d'au moins un conjugué anticorps-enzyme comprenant un anticorps réactif avec un antigène à la surface des cellules tumorales, conjugué avec une enzyme capable de convertir ladite pro-drogue en le médicament parent plus cytotoxique, pour la préparation d'une composition pharmaceutique pour le traitement des tumeurs.

4. Utilisation de la revendication 3, où l'anticorps est sélectionné dans le groupe consistant en anticorps polyclonaux, monoclonaux ou chimériques.

5. Utilisation de la revendication 3, où l'anticorps est sélectionné dans le groupe consistant en anticorps monoclonaux L6, 96,5 et 1F5.

6. Utilisation de la revendication 3, où l'enzyme est sélectionnée parmi les pénicillines amidases.

7. Utilisation de la revendication 3, où l'enzyme est la pénicilline V amidase ou la pénicilline G amidase.

8. Utilisation de la revendication 3, où la pro-drogue est sélectionnée dans le groupe consistant en N-(p-hydroxyphénoxyacétyl)-adriamycine, N-(phénoxyacétyl)adriamycine, N-(p-hydroxyphénylacétyl)adriamycine ou N-(phénylacétyl)adriamycine.

9. Utilisation de la revendication 3, où le conjugué anticorps-enzyme est L6-PVA ou 1F5-PVA.

10. Utilisation de la revendication 3, où le conjugué anticorps enzyme est L6-PVA et la pro-drogue est sélectionnée dans le groupe consistant en N-(p-hydroxyphénoxyacétyl)adriamycine et N-(phénoxyacétyl) adriamycine.

11. Utilisation de la revendication 3, où les cellules tumorales sont d'une origine sélectionnée dans le groupe consistant en carcinomes, mélanomes, lymphomes, sarcomes des tissus osseux et mous et autres tumeurs.

12. Utilisation de l'une quelconque des revendications 3 à 11 où plus d'une pro-drogue est utilisée, chacune d'entre elles est faiblement cytotoxique vis-à-vis des cellules tumorales en comparaison avec son médicament parent correspondant.

13. Utilisation de la revendication 12, où les prodrogues sont sélectionnées dans le groupe consistant en dérivés N-phénoxyacétylés de l'adriamycine et dérivés N-phénylacétylés de l'adriamycine.

14. Utilisation de la revendication 3, où plus d'un conjugué anticorps-enzyme est utilisé, où l'anticorps de chaque conjugué est réactif avec le même antigène ou un antigène différent placé à la surface desdites cellules tumorales et l'enzyme de chaque conjugué est la même ou différente et est capable de convertir au moins une pro-drogue qui est faiblement cytotoxique vis-à-vis des cellules tumorales, en comparaison avec son médicament parent correspondant, en le médicament parent plus cytotoxique.

15. Procédé de préparation d'une composition pharmaceutiquement acceptable consistant en deux composants séparés, utile dans le traitement des tumeurs, lequel procédé consiste à prévoir, en tant qu'un composé, une quantité pharmaceutiquement efficace d'au moins une pro-drogue préparée selon la revendication 1 ou 2.

16. Combinaison de deux composants séparés où un composant comprend au moins un conjugué anticorps-enzyme et l'autre composant comprend au moins une pro-drogue qui est faiblement cytotoxique vis-à-vis des cellules tumorales, en comparaison avec son médicament parent correspondant, tels que définis selon l'une quelconque des revendications 1 à 14.

17. Combinaison de la revendication 16, où le conjugué anticorps-enzyme est L6-PVA.

18. Combinaison de la revendication 16, où la pro-drogue est sélectionnée dans le groupe consistant en dérivés N-phénoxyacétylés de l'adriamycine et dérivés N-phénylacétylés de l'adriamycine.

19. Utilisation de la revendication 3, où le conjugué anticorps-enzyme est une protéine de fusion comprenant au moins la région de liaison de l'antigène d'un anticorps réactif avec un antigène associé à une tumeur enchaîné à au moins une portion fonctionnellement active d'une enzyme capable de convertir au moins une pro-drogue faiblement cytotoxique en son médicament parent plus cytotoxique.

20. Procédé pour la préparation des composés des revendications 1 et 2, caractérisé en ce qu'un composé de la formule générale 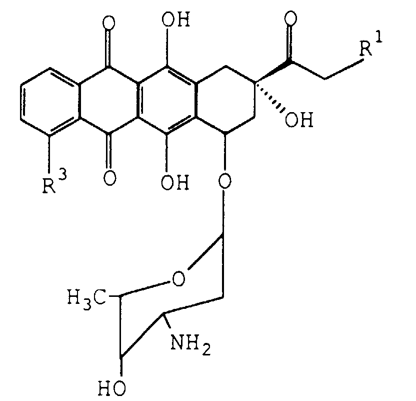
a) est mis à réagir avec de l'acide p-hydroxy phénoxy acétique ou de l'acide phénoxy acétique, ou bien un dérivé activé de ceux-ci, de préférence activé avec du N-hydroxy-succinimide ou du dicyclo hexylcarbodiimide, pour former un composé de la formule générale (I); ou
b) est mis à réagir avec de l'acide phényl acétique ou de l'acide hydroxy phényl acétique ou leur dérivé activé, de préférence activé avec du N-hydroxysuccinimide ou du dicyclo hexylcarbodiimide, pour former un composé de la formule générale (II).

21. Procédé de préparation des combinaisons des revendications 16 à 18 qui consiste à combiner ledit conjugué anticorps-enzyme et ladite pro-drogue.
